# EUROPEAN PATENT SPECIFICATION

(11) **EP 1 025 114 B1**
(45) Date of publication and mention of the grant of the patent: **25.02.2004**
(21) Application number: 98946087.8
(22) Date of filing: 16.09.1998
(51) Int. Cl.: C07H 17/08, A61K 31/70

(54) **3'-N-MODIFIED 6-O-SUBSTITUTED ERYTHROMYCIN KETOLIDE DERIVATIVES HAVING ANTIBACTERIAL ACTIVITY**
3'-N-MODIFIZIERTE 6-0 SUBSTITUIERTE ERYTHROMYCIN KETOLIDE DERIVATE MIT ANTIBAKTERIELLER WIRKUNG
DERIVES D'ERYTHROMYCINE CETOLIDES, MODIFIES EN N-3' ET SUBSTITUES EN O-6, POSSEDANT UNE ACTIVITE ANTIBACTERIENNE

(30) Priority: 30.09.1997 US 940871; 12.08.1998 US 133121
(43) Date of publication of application: 09.08.2000
(73) Proprietor: Abbott Laboratories, Abbott Park, Illinois 60064-3500 (US)
(72) Inventor: OR, Yat, Sun, Libertyville, IL 60048 (US); MA, Zhenkun, Gurnee, IL 60031 (US); CHU, Daniel, T., Santa Clara, CA 95051 (US)
(74) Representative: Modiano, Guido, Dr.-Ing.
(86) International application number: PCT/US1998/019311
(87) International publication number: WO 1999/016779

(56) References cited:
- WO-A-97/17356
- US-A- 5 444 051
- SUWA T ET AL: "UPTAKE OF O-ALKYL ERYTHROMYCIN DERIVATIVES IN THE LUNG TISSUE AND CELLS OF RATS" JOURNAL OF PHARMACEUTICAL SCIENCES, vol. 79, no. 9, September 1989, page 783/784 XP002035991
- MORIMOO S. ET AL.: "Chemical modifications of erythromycins. II. Synthesis and antibacterial activity of o-alkyl derivatives of erythromycin A" THE JOURNAL OF ANTIBIOTICS, vol. 43, 1990, pages 286-294, XP002093093 cited in the application

## Description

### Technical Field

This invention relates to novel semi-synthetic macrolides having antibacterial activity, to pharmaceutical compositions comprising these compounds, and to a medical method of treatment. More particularly, the invention relates to 3'-N-modified 6-O-substituted erythromycin ketolide derivatives and methods for preparing them, compositions containing these compounds, and a method of treating bacterial infections with such compositions.

### Background of the Invention

Erythromycins A through D, represented by formula (E). 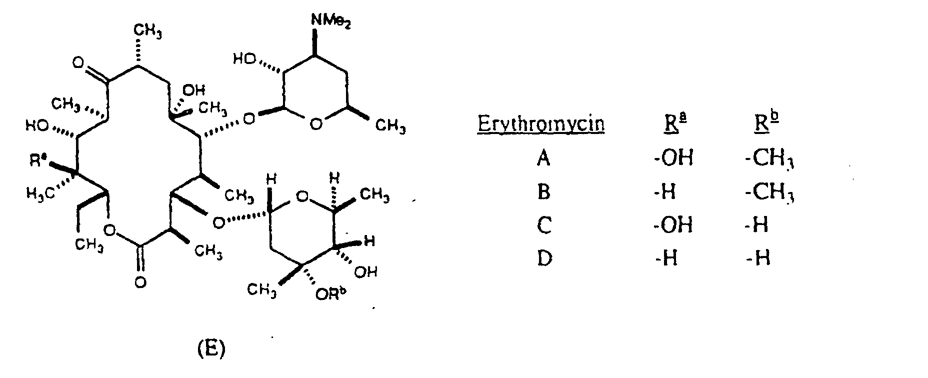 are well-known and potent antibacterial agents, used widely to treat and prevent bacterial infection. As with other antibacterial agents, however, bacterial strains having resistance or insufficient susceptibility to erythromycin have been identified. Also, erythromycin A has only weak activity against Gram-negative bacteria. Therefore, there is a continuing need to identify new erythromycin derivative compounds which possess improved antibacterial activity, which have less potential for developing resistance, which possess the desired Gram-negative activity, or which possess unexpected selectivity against target microorganisms. Consequently, numerous investigators have prepared chemical derivatives of erythromycin in an attempt to obtain analogs having modified or improved profiles of antibiotic activity.

Morimoto *et al.* describes the preparation of 6-O-methyl erythromycin A in J. Antibiotics 37:187 (1984). Morimoto *et al.* further discloses 6-O-alkyl erythromycin A derivatives in *J*. *Antibiotics.* 43: 286 (1990) and in European Patent Application 272, 110, published June 22,1988. European Patent Application 215.355, published March 28, 1987. discloses 6-O-loweralkyl erythromycins as stimulants of gastrointestinal contractile motion.

United States Patent 5.444,051 discloses 6-O-substituted-3-oxoerythromycin A derivatives in which the substituents are selected from alkyl, -CONH₂, -CONHC(O)alkyl and -CONHSO₂alkyl. PCT application WO 97/10251, published March 20, 1997, discloses 6-O-methyl 3-descladinose erythromycin derivatives, and PCT application WO 97/17356, published May 15, 1997. discloses 3-deoxy-3-descladinose erythromycin derivatives.. PCT application WO 92/09614, published June 11, 1992, discloses tricyclic 6-O-methyl erythromycin A derivatives. Certain intermediates to the present invention are disclosed in U.S. Patent 5,866,549.

European Patent Application 596802. published May 11, 1994, discloses bicyclic 6-O-methyl-3-oxo erythromycin A derivatives.

### Summary of the Invention.

The present invention provides a novel class of 3'-N-modified 6-O-substituted erythromycin ketolide derivatives which possess antibacterial activity.

In one aspect of the present invention are compounds, or pharmaceutically acceptable salts and esters thereof, having a formula selected from the group consisting of 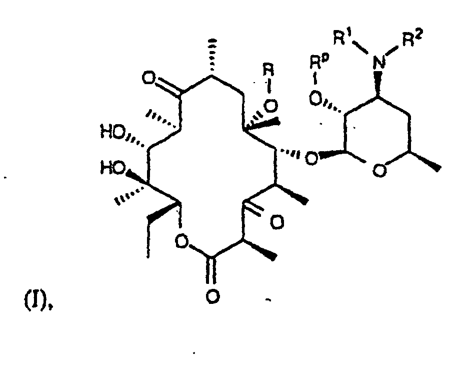 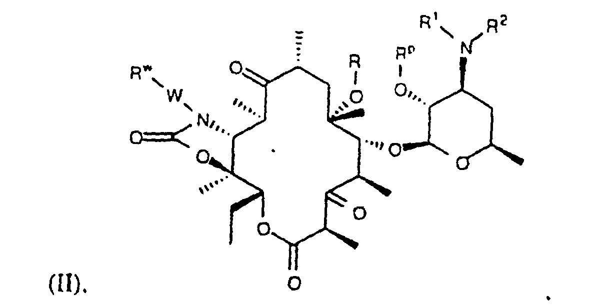 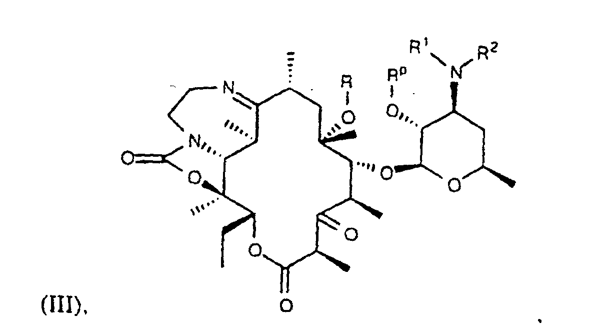 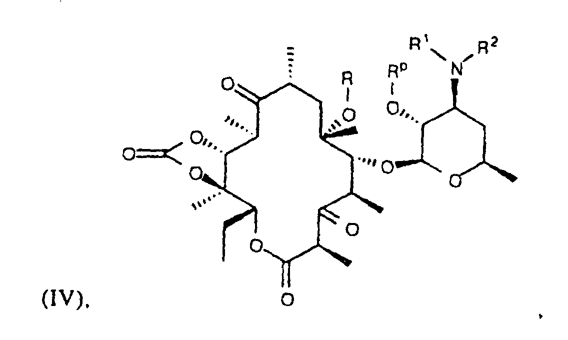 and 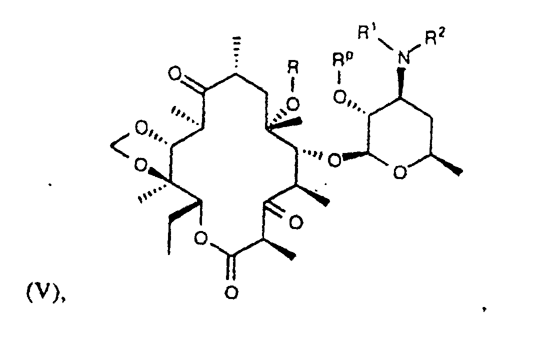 or a pharmaceutically acceptable salt, ester or prodrug thereof, wherein
R¹ and R², with the proviso that R¹ and R² are not both methyl, are independently selected from the group consisting of
(1) hydrogen,
(2) C₁-C₆-alkyl optionally substituted with a substituent-selected from the group consisting of
   (a) halogen,
   (b) C₃-C₆-cycloalkyl,
   (c) aryl,
   (d) substituted aryl,
   (e) heteroaryl.
   (f) substituted heteroaryl,
   (g) -CHO,
   (h) -C(O)-C₁-C₆-alkyl, and
   (i) -C(O)-NR'R", wherein R' and R" are independently selected from the group consisting of hydrogen, C₁-C₃-alkyl, C₁-C₃-alkyl substituted with aryl, substituted aryl, heteroaryl, and substituted heteroaryl,
(3) C₂-C₆-alkyl optionally substituted with a substituent selected from the group consisting of
   (a) C₁-C₆-alkoxy,
   (b) -NR'R", wherein R' and R" are as previously defined,
   (c) -NH-C(O)-C₁-C₆-alkyl,
   (d) -NH-C(O)-O-C₁-C₆-alkyl,
   (e) -O-C(O)-O-C₁-C₆-alkyl,
   (f) -O-C(O)-C₁-C₆-alkyl,
   (g) -CH(=N-O-C₁-C₆-alkyl),
   (h) -C(=N-O-C₁-C₆-alkyl)-C₁-C₆-alkyl,
   (i) -CH(=N-NH-C₁-C₆-alkyl), and
   (j) -C(=N-NH-C₁-C₆-alky)-C₁-C₆-alkyl,
(4) C₃-C₆-alkenyl optionally substituted with a substituent selected from the group consisting of
   (a) halogen,
   (b) C₃-C₆-cycloalkyl,
   (c) aryl,
   (d) substituted aryl,
   (e) heteroaryl,
   (f) substituted heteroaryl,
   (g) -NH-C(O)-C₁-C₆-alkyl,
   (h) -NH-C(O)-O-C₁-C₆-alkyl,
   (i) -O-C(O)-O-C₁-C₆-alkyl,
   (j) -O-C(O)-C₁-C₆-alkyl,
   (k) -CHO.
   (l) -C(O)-C₁-C₆-alkyl.
   (m) -C(O)-NR'R", wherein R' and R" are as previously defined,
   (n) -CH(=N-O-C₁-C₆-alkyl),
   (o) -C(=N-O-C₁-C₆-alkyl)-C₁-C₆-alkyl,
   (p) -CH(=N-NH-C₁-C₆-alkyl),
   (q) -C(=N-NH-C₁-C₆-alkyl)-C₁-C₆-alkyl, and
   (r) -C(O)-O-C₁-C₆-alkyl,
(5) C₃-C₆-alkynyl optionally substituted with a substituent selected from the group consisting of
   (a) halogen,
   (b) C₃-C₆-cycloalkyl,
   (c) aryl,
   (d) substituted aryl,
   (e) heteroaryl, and
   (f) substituted heteroaryl,
(6) C₃-C₆-cycfoalkyl,
(7) -CHO,
(8) -C(O)-C₁-C₆,-alkyl,
(9) -C(O)-NR'R", wherein R' and R" are as previously defined, and
(10) -C(O)-O-C₁-C₆-alkyl,
or R¹ and R² taken together may be -(CH₂)ₚ₋, wherein p is 3-to-7, which taken together with the nitrogen atom to which they are attached, thus form a heterocyclic ring containing one nitrogen atom and from 3 to 7 carbon atoms;
R is selected from the group consisting of
(1) methyl substituted with a substituent selected from the group consisting of
   (a) -CN,
   (b) -F,
   (c) -CO₂R³ wherein R³ is C₁-C₃-alkyl, aryl-substituted C₁-C₃-alkyl, or heteroaryl-substituted C₁-C₃-alkyl,
   (d) -S(O)ₙ-R³ wherein n is 0, 1, or 2, and R³ is as previously defined,
   (e) -NH-C(O)-R³ where R³ is as previously defined,
   (f) -NH-C(O)-NR⁴R⁵ wherein R⁴ and R⁵ are independently selected from the group consisting of
      (i) hydrogen,
      (ii) C₁-C₃-alkyl
      (iii) C₁-C₃-alkyl substituted with aryl,
      (iv) C₁-C₃-alkyl substituted with substituted aryl,
      (v) C₁-C₃-alkyl substituted with heteroaryl, and
      (vi) C₁-C₃-alkyl substituted with and substituted heteroaryl,
   (g) aryl,
   (h) substituted aryl,
   (i) heteroaryl,
      and
   (j) substituted heteroaryl,
(2) C₂-C₁₀-alkyl,
(3) C₂-C₁₀-alkyl substituted with one or more substituents selected from the group consisting of
   (a) halogen,
   (b) hydroxy,
   (c) C₁-C₃-alkoxy,
   (d) C₁-C₃-alkoxy-C₁-C₃-alkoxy,
   (e) oxo,
   (f) -N₃,
   (g) -CHO,
   (h) -O-SO₂-(substituted C₁-C₆-alkyl),
   (i) -NR⁶R⁷ wherein R⁶ and R⁷ are selected from the group consisting of
      (i) hydrogen,
      (ii) C₁-C₁₂-alkyl,
      (iii) substituted C₁-C₁₂-alkyl,
      (iv) C₁-C₁₂-alkenyl,
      (v) substituted C₁-C₁₂-alkenyl,
      (vi) C₁-C₁₂-alkynyl,
      (vii) substituted C₁-C₁₂-alkynyl,
      (viii) aryl,
      (ix) C₃-C₈-cycloalkyl,
      (x) substituted C₃-C₈-cycloalkyl,
      (xi) substituted aryl,
      (xii) heterocycloatkyl,
      (xiii) substituted heterocycloalkyl,
      (xiv) C₁-C₁₂-alkyl substituted with aryl,
      (xv) C₁-C₁₂-alkyl substituted with substituted aryl,
      (xvi) C₁-C₁₂-alkyl substituted with heterocycloalkyl,
      (xvii) C₁-C₁₂-alkyl substituted with substituted heterocycloalkyl,
      (xviii) C₁-C₁₂-alkyl substituted with C₃-C₈-cycloalkyl,
      (xix) C₁-C₁₂-alkyl substituted with substituted C₃-C₈-cycloalkyl,
      (xx) heteroaryl,
      (xxi) substituted heteroaryl,
      (xxii) C₁-C₁₂-alkyl substituted with heteroaryl,
         and
      (xxiii) C₁-C₁₂-alkyl substituted with substituted heteroaryl,
         or
      R⁶ and R⁷ are taken together with the atom to which they are attached form a 3-10 membered heterocycloalkyl ring which may be substituted with one or more substituents independently selected from the group consisting of
      (i) halogen,
      (ii) hydroxy,
      (iii) C₁-C₃-alkoxy,
      (iv) C₁-C₃-alkoxy-C₁-C₃-alkoxy,
      (v) oxo,
      (vi) C₁-C₃-alkyl,
      (vii) halo-C₁-C₃-alkyl,
         and
      (vii) C₁-C₃-alkoxy-C₁-C₃-alkyl,
   (j) -CO₂R³ wherein R³ is as previously defined,
   (k) -C(O)-NR⁴R⁵ wherein R⁴ and R⁵ are as previously defined,
   (l) =N-O-R3 wherein R³ is as previously defined,
   (m) -O≡N,
   (n) -O-S(O)ₙ-R³ wherein n and R³ are as previously defined,
   (o) aryl,
   (p) substituted aryl,
   (q) heteroaryl,
   (r) substituted heteroaryl,
   (s) C₃-C₈-cycloalkyl,
   (t) substituted C₃-C₈-cycloalkyl,
   (u) C₁-C₁₂-alkyl substituted with heteroaryl,
   (v) heterocycloalkyl,
   (w) substituted heterocycloalkyl,
   (x) -NH-C(O)-R³ where R³ is as previously defined,
   (y) -NH-C(O)-NR⁴R⁵ wherein R⁴ and R⁵ are as previously defined,
   (z) =N-NR⁶R⁷ wherein R⁶ and R⁷ are as previously defined,
   (aa) =N-R³ wherein R³ is as previously defined,
   (bb) =N-NH-C(O)-R⁴ wherein R⁴ is as previously defined.
      and
   (cc) =N-NH-C(O)-NR⁴R⁵ wherein R⁴ and R⁵ are as previously defined,
(4) C₃-alkenyl substituted with a moiety selected from the group consisting of
   (a) halogen,
   (b) -CHO,
   (c) -CO₂R³ where R³ is as previously defined,
   (d) -C(O)-R⁴ where R⁴ is as previously defined,
   (e) -C(O)-NR⁴R⁵ wherein R⁴ and R⁵ are as previously defined,
   (f) -C≡N,
   (g) aryl,
   (h) substituted aryl,
   (i) heteroaryl,
   (j) substituted heteroaryl,
   (k) C₃-C₇-cycloalkyl,
      and
   (I) C₁-C₁₂-alkyl substituted with heteroaryl,
(5) C₄-C₁₀-alkenyl,
(6) C₄-C₁₀-alkenyl substituted with one or more substituents selected from the group consisting of
   (a) halogen,
   (b) C₁-C₃-alkoxy,
   (c) oxo,
   (d) -CHO,
   (e) -CO₂R³ where R³ is as previously defined,
   (f) -C(O)-NR⁴R⁵ wherein R⁴ and R⁵ are as previously defined,
   (g) -NR⁶R⁷ wherein R⁶ and R⁷ are as previously defined,
   (h) =N-O-R³ wherein R³ is as previously defined,
   (i) -C≡N,
   (j) -O-S(O)ₙ-R³ wherein n is 0, 1, or 2 and R³ is as previously defined,
   (k) aryl,
   (l) substituted aryl,
   (m) heteroaryl,
   (n) substituted heteroaryl,
   (o) C₃-C₇-cycloalkyl,
   (p) C₁-C₁₂-alkyl substituted with heteroaryl,
   (q) -NH-C(O)-R³ where R³ is as previously defined,
   (r) -NH-C(O)-NR⁴R⁵ wherein R⁴ and R⁵ are as previously defined,
   (s) =N-NR⁶R⁷ wherein R⁶ and R⁷ are as previously defined,
   (t) =N-R³ wherein R³ is as previously defined,
   (u) =N-NH-C(O)-R³ where R³ is as previously defined,
      and
   (v) =N-NH-C(O)-NR⁴R⁵ wherein R⁴ and R⁵ are as previously defined,
(7) C₃-C₁₀-alkynyl,
   and
(8) C₃-C₁₀-alkynyl substituted with one or more substituents selected from the group consisting of
   (a) trialkylsilyl,
   (b) aryl,
   (c) substituted aryl,
   (d) heteroaryl,
      and
   (e) substituted heteroaryl,
   with the proviso that when R is allyl and R¹ is methyl, R² is not H;
   R^{p} is hydrogen or a hydroxy protecting group;
   R^{w} is selected from the group consisting of
   (1) hydrogen.
   (2) C₁-C₆-alkyl, optionally substituted with one or more substituents selected from the group consisting of
      (a) aryl,
      (b) substituted aryl,
      (c) heteroaryl,
      (d) substituted heteroaryl,
   (3) a group selected from option (2) as previously defined further substituted with -CH₂-M-R⁸, wherein M is selected from the group consisting of
      (i) -O-,
      (ii) -NH-,
      (ii) -N(CH₃)-,
      (iv) -S(O)ₙ-, wherein n is as described previously.
      (v) -NH-C(O)-, and
      (vi) -C(O)-NH-,
         and
      R⁸ is selected from the group consisting of
      (i) -(CH₂)ₙ-aryl, wherein n is as described previously,
      (ii) -(CH₂)ₙ-substituted aryl, wherein n is as described previously,
      (iii) -(CH₂)ₙ-heteroaryl, wherein n is as described previously,
      (iv) -(CH₂)ₙ-substituted heteroaryl, wherein n is as described previously,
         and
      (v) -(CH₂)ₙ-heterocycloakyl, wherein n is as described previously;
         and
   W is absent or is selected from the group consisting of -O-. -NH- and -N(CH₃)-.

The present invention also provides pharmaceutical compositions which comprise a therapeutically effective amount of a compound as defined previously in combination with a pharmaceutically acceptable carrier.

The invention further relates to a method of treating bacterial infections in a host mammal in need of such treatment comprising administering to a mammal in need of such treatment a therapeutically effective amount of a compound as defined previously.

In a further aspect of the present invention, processes are provided for the preparation of 3'-N-modified 6-O-substituted erythromycin ketolide derivatives of Formula (I), (II), (III), (IV) and (V) as described previously.

### Detailed Description of The Invention

### Definitions

As used throughout this specification and the appended claims, the following terms have the meanings specified.

The term "alkanoyl" as used herein refers to an C₁-C₆-alkyl group, as defined herein, attached to the parent molecular moiety through an carbonyl group. Examples of alkanoyl groups include acetyl, propanoyl, butanoyl.

The terms "C₁-C₃-alkyl", "C₁-C₆-alkyl", and "C₁-C₁₂-alkyl" as used herein refer to saturated, straight- or branched-chain hydrocarbon radicals derived from a hydrocarbon moiety containing between one and three, one and six, and one and twelve carbon atoms, respectively, by removal of a single hydrogen atom. Examples of C₁-C₃-alkyl radicals include methyl, ethyl, propyl and isopropyl, examples of C₁-C₆-alkyl radicals include, but are not limited to, methyl, ethyl, propyl, isopropyl, *n*-butyl, *tert*-butyl, neopentyl and n-hexyl. Examples of C₁-C₁₂-alkyl radicals include, but are not limited to, all the foregoing examples as well as n-heptyl, n-octyl, n-nonyl, n-decyl, n-undecyl and n-docecyl.

The term "C₁-C₆-alkoxy" as used herein refers to an C₁-C₆-alkyl group, as previously defined, attached to the parent molecular moiety through an oxygen atom. Examples of C₁-C₆-alkoxy, but are not limited to, methoxy, ethoxy, propoxy, *iso*-propoxy, *n*-butoxy, *tert*-butoxy. *neo*-pentoxy and *n*-hexoxy.

The term "C₁-C₁₂-alkenyl" denotes a monovalent group derived from a hydrocarbon moiety containing from two to twelve carbon atoms and having at least one carbon-carbon double bond by the removal of a single hydrogen atom. Alkenyl groups include, for example, ethenyl, propenyl, butenyl, 1-methyl-2-buten-1-yl.

The term "C₁-C₁₂-alkynyl" as used herein refers to a monovalent group derived from a hydrocarbon containing from two to twelve carbon atoms and having at least one carbon-carbon triple bond by the removal of a single hydrogen atom. Representative alkynyl groups include ethynyl, 2-propynyl (propargyl), 1-propynyl.

The term "alkylene" denotes a divalent group derived from a straight or branched chain saturated hydrocarbon by the removal of two hydrogen atoms, for example methylene, 1,2-ethylene, 1,1-ethylene, 1,3-propylene, 2,2-dimethylpropylene.

The term "C₁-C₃-alkylamino" as used herein refers to one or two C₁-C₃-alkyl groups, as previously defined, attached to the parent molecular moiety through a nitrogen atom. Examples of C₁-C₃-alkylamino include, but are not limited to methylamino, dimethylamino, ethylamino, diethylamino, and propylamino.

The term "oxo" denotes a group wherein two hydrogen atoms on a single carbon atom in an alkyl group as defined previously are replaced with a single oxygen atom (i.e. a carbonyl group).

The term "aprotic solvent" as used herein refers to a solvent that is relatively inert to proton activity, i.e., not acting as a proton-donor. Examples include, but are not limited to. hydrocarbons, such as hexane and toluene, for example, halogenated hydrocarbons, such as. for example, methylene chloride, ethylene chloride, chloroform, heteroaryl compounds, such as, for example, tetrahydrofuran and N-methylpyrrolidinone, and ethers such as diethyl ether, bis-methoxymethyl ether. Such compounds are well known to those skilled in the art, and it will be obvious to those skilled in the art that individual solvents or mixtures thereof may be preferred for specific compounds and reaction conditions, depending upon such factors as the solubility of reagents, reactivity of reagents and preferred temperature ranges, for example. Further discussions of aprotic solvents may be found in organic chemistry textbooks or in specialized monographs, for example: Organic Solvents Physical Properties and Methods of Purification, 4th ed., edited by John A. Riddick *et al.,* Vol. II, in the Techniques of Chemistry Series, John Wiley & Sons, NY, 1986.

The term "aryl" as used herein refers to a mono- or bicyclic carbocyclic ring system having one or two aromatic rings including, but not limited to, phenyl, naphthyl, tetrahydronaphthyl, indanyl, indenyl and the like. Aryl groups (including bicyclic aryl groups) can be unsubstituted or substituted with one, two or three substituents independently selected from loweralkyl, substituted loweralkyl, cycloalkyl, alkenyl, alkoxy, alkanoyl, haloalkyl, alkoxy, thioalkoxy, amino, alkylamino. dialkylamino, acylamino, cyano, hydroxy. hydroxyalkyl, halo, mercapto, nitro, carboxaldehyde, carboxy, alkoxycarbonyl, and carboxamide. In addition, substituted aryl groups include tetrafluorophenyl and pentafluorophenyl.

The term "C₃-C₁₂-cycloalkyl" denotes a monovalent group derived from a monocyclic or bicyclic saturated carbocyclic ring compound by the removal of a single hydrogen atom. Examples include cyclopropyl, cyclobutyl, cyclopentyl, cyclohexyl, bicyclo[2.2.1 1]heptyl, and bicyclo[2.2.2]octyl.

The terms "halo" and "halogen" as used herein refer to an atom selected from fluorine, chlorine, bromine and iodine.

The term "alkylamino" refers to a group having the structure -NHR' wherein R' is alkyl, as previously defined. Examples of alkylamino include methylamino, ethylamino, *iso*-propylamino.

The term "dialkylamino" refers to a group having the structure -NR'R" wherein R' and R" are independently selected from alkyl, as previously defined. Additionally, R' and R" taken together may optionally be -(CH₂)ₖ- wherein k is an integer of from 2 to 6. Examples of dialkylamino include, dimethylamino, diethylaminocarbonyl, methylethylamino, piperidino.

The term "haloalkyl" denotes an alkyl group, as defined previously, having one, two, or three halogen atoms attached thereto and is exemplified by such groups as chloromethyl, bromoethyl, trifluoromethyl.

The term "alkoxycarbonyl" represents an ester group: i.e. an alkoxy group, attached to the parent molecular moiety through a carbonyl group such as methoxycarbonyl, ethoxycarbonyl.

The term "thioalkoxy" refers to an alkyl group as previously defined attached to the parent molecular moiety through a sulfur atom.

The term "carboxaldehyde" as used herein refers to a group of formula -CHO.

The term "carboxy" as used herein refers to a group of formula -CO₂H.

The term "carboxamide" as used herein refers to a group of formula -CONHR'R" wherein R' and R" are independently selected from hydrogen or alkyl, or R' and R" taken together may optionally be -(CH₂)ₖ- where k is an integer of from 2 to 6.

The term "heteroaryl", as used herein, refers to a cyclic aromatic radical having from five to ten ring atoms of which one ring atom is selected from S, O and N; zero, one or two ring atoms are additional heteroatoms independently selected from S. O and N; and the remaining ring atoms are carbon, the radical being joined to the rest of the molecule via any of the ring atoms, such as, for example, pyridyl, pyrazinyl, pyrimidinyl, pyrrolyl, pyrazolyl, imidazolyl, thiazolyl, oxazolyl, isoxazolyl, thiadiazolyl, oxadiazolyl, thiophenyl, furanyl, quinolinyl, isoquinolinyl.

The term "heterocycloalkyl" as used herein, refers to a non-aromatic partially unsaturated or fully Saturated 3- to 10-membered ring system, which includes single rings of 3 to 8 atoms in size and bi- or tri-cyclic ring systems which may include aromatic six-membered aryl or heteroaryl rings fused to a non-aromatic ring. These heterocycloalkyl rings include those having from one to three heteroatoms independently selected from oxygen, sulfur and nitrogen, in which the nitrogen and sulfur heteroatoms may optionally be oxidized and the nitrogen heteroatom may optionally be quaternized.

Representative heterocycles include, but are not limited to, pyrrolidinyl, pyrazolinyl, pyrazolidinyl, imidazolinyl, imidazolidinyl, piperidinyl, piperazinyl, oxazolidinyl, isoxazolidinyl, morpholinyl, thiazolidinyl, isothiazolidinyl, and tetrahydrofuryl.

Specific heterocycloalkyl rings considered useful in preparing compounds of the invention include: 3-methyl-4-(3-methylphenyl)piperazine, 3-methylpiperidine, 4-(bis-(4-fluorophenyl)methyl)piperazine, 4-(diphenylmethyl)piperazine, 4-(ethoxycarbonyl)piperazine, 4-(ethoxycarbonylmethyl)piperazine, 4-(phenylmethyl)piperazine, 4-(1-phenylethyl)piperazine, 4-(1,1-dimethylethoxycarbonyl)piperazine, 4-(2-(bis-(2-propenyl)amino)ethyl)piperazine, 4-(2-(diethylamino)ethyl)piperazine, 4-(2-chlorophenyl)piperazine, 4-(2-cyanaphenyl)piperazine. 4-(2-ethoxyphenyl)piperazine, 4-(2-ethylphenyl)piperazine, 4-(2-fluorophenyl)piperazine, 4-(2-hydroxyethyl)piperazine, 4-(2-methoxyethyl)piperazine, 4-(2-methoxyphenyl)piperazine, 4-(2-methylphenyl)piperazine, 4-(2-methylthiophenyl)piperazine, 4-(2-nitrophenyl)piperazine, 4-(2-nitrophenyl)piperazine, 4-(2-phenylethyl)piperazine, 4-(2-pyridyl)piperazine, 4-(2-pyrimidinyl)piperazine, 4-(2,3-dimethylphenyl)piperazine, 4-(2,4-difluorophenyl)piperazine, 4-(2,4-dimethoxyphenyl)piperazine, 4-(2,4-dimethylphenyl)piperazine, 4-(2,5-dimethylphenyl)piperazine, 4-(2,6-dimethylphenyl)piperazine, 4-(3-chlorophenyl)piperazine, 4-(3-methylphenyl)piperazine, 4-(3-trifluoromethylphenyl)piperazine, 4-(3,4-dichlorophenyl)piperazine, 4-(3,4-dimethoxyphenyl)piperazine, 4-(3,4-dimethylphenyl)piperazine, 4-(3,4-methylenedioxyphenyl)piperazine, 4-(3,4,5-trimethoxyphenyl)piperazine, 4-(3,5-dichlorophenyl)piperazine, 4-(3,5-dimethoxyphenyl)piperazine, 4-(4-(phenylmethoxy)phenyl)piperazine, 4-(4-(1,1-dimethylethyl)phenylmethyl)piperazine, 4-(4-chloro-3-trifluoromethylphenyl)piperazine, 4-(4-chlorophenyl)-3-methylpiperazine, 4-(4-chlorophenyl)piperazine, 4-(4-chlorophenyl)piperazine, 4-(4-chlorophenylmethyl)piperazine, 4-(4-fluorophenyl)piperazine, 4-(4-methoxyphenyl)piperazine, 4-(4-methylphenyl)piperazine, 4-(4-nitrophenyl)piperazine, 4-(4-trifluoromethylphenyl)piperazine, 4-cyclohexylpiperazine, 4-ethylpiperazine, 4-hydroxy-4-(4-chlorophenyl)methylpiperidine, 4-hydroxy-4-phenylpiperidine, 4-hydroxypyrrolidine, 4-methylpiperazine, 4-phenylpiperazine, 4-piperidinylpiperazine, 4-((2-furanyl)carbonyl)piperazine, 4-((1,3-dioxolan-5-yl)methyl)piperazine, 6-fluoro-1,2,3,4-tetrahydro-2-methylquinoline, 1,4-diazacycloheptane, 2,3-dihydroindolyl, 3,3-dimethylpiperidine, 4,4-ethylenedioxypiperidine, 1,2,3,4-tetrahydroisoquinoline, 1,2,3,4-tetrahydroquinoline, azacyclooctane, decahydroquinoline, piperazine, piperidine, pyrrolidine, thiomorpholine, and triazole.

The term "heteroarylalkyl" as used herein, refers to a heteroaryl group as defined previously attached to the parent molecular moiety through an alkylene group wherein the alkylene group is of one to four carbon atoms.

"Hydroxy-protecting group", as used herein, refers to an easily removable group which is known in the art to protect a hydroxyl group against undesirable reaction during synthetic procedures and to be selectively removable. The use of hydroxy-protecting groups is well known in the art for protecting groups against undesirable reactions during a synthetic procedure and many such protecting groups are known, cf., for example, T.H. Greene and P.G.M. Wuts, Protective Groups in Organic Synthesis 2nd edition, John Wiley & Sons, New York (1991). Examples of hydroxy-protecting groups include, but are not limited to, methylthiomethyl, *tert*-dimethylsilyl, *tert*-butyldiphenylsilyl, ethers such as methoxymethyl, and esters including acetyl benzoyl.

The term "ketone protecting group", as used herein, refers to an easily removable group which is known in the art to protect a ketone group against undesirable reactions during synthetic procedures and to be selectively removable. The use of ketone-protecting group is well known in the art for protecting groups against undesirable reactions during a synthetic procedure and many such protecting groups are known, cf.. for example, T.H. Greene and P.G.M. Wuts, Protective Groups in Organic Synthesis, 2nd edition. John Wiley & Sons, New York (1991). Examples of ketone-protecting groups include, but are not limited to, ketals, oximes, O-substituted oximes for example O-benzyl oxime, O-phenylthiomethyl oxime, 1-isopropoxycyclohexyl oxime.

A the term "protected-hydroxy" refers to a hydroxy group protected with a hydroxy protecting group, as defined previously, including benzoyl, acetyl, trimethylsilyl, triethylsilyl, methoxymethyl groups, for example.

The term "protogenic organic solvent" as used herein refers to a solvent that tends to provide protons, such as an alcohol, for example, methanol, ethanol, propanol, isopropanol, butanol, t-butanol, and the like. Such solvents are well known to those skilled in the art, and it will be obvious to those skilled in the art that individual solvents or mixtures thereof may be preferred for specific compounds and reaction conditions, depending upon such factors as the solubility of reagents, reactivity of reagents and preferred temperature ranges, for example, Further discussions of protogenic solvents may be found in organic chemistry textbooks or in specialized monographs, for example: Organic Solvents Physical Properties and Methods of Purification, 4th ed.. edited by John A. Riddick *et al.*, Vol. II, in the Techniques of Chemistry Series, John Wiley & Sons, NY, 1986.

The term "substituted aryl" as used herein refers to an aryl group as defined herein substituted by independent replacement of one, two or three of the hydrogen atoms thereon with -Cl, -Br, -F, -I, -OH, -CN, C₁-C₃-alkyl, C₁-C₆-alkoxy, C₁-C₆-alkoxy substituted with aryl, haloalkyl, thioalkoxy, amino, alkylamino, dialkylamino, mercapto, nitro, carboxaldehyde, carboxy, cycloalkyl, alkenyl, alkoxy, alkanoyl, hydroxyalkyl, alkoxycarbonyl and carboxamide. In addition, any one substitutent may be an aryl, heteroaryl, or beterocycloalkyl group. Also, substituted aryl groups include tetrafluorophenyl and pentafluorophenyl.

The term "substituted heteroaryl" as used herein refers to a heteroaryl group as defined herein substituted by independent replacement of one, two or three of the hydrogen atoms thereon with -Cl, -Br, -F, -I, -OH, -CN, C₁-C₃-alkyl, C₁-C₆-alkoxy, C₁-C₆-alkoxy substituted with aryl, haloalkyl, thioalkoxy, amino, alkylamino, dialkylamino, mercapto, nitro, carboxaldehyde, carboxy, alkoxycarbonyl, and carboxamide. In addition, any one substitutent may be an aryl, heteroaryl, or heterocycloalkyl group.

The term "substituted heterocycloalkyl" as used herein, refers to a heterocycloalkyl group, as defined previously, substituted by independent replacement of one, two or three of the hydrogen atoms thereon with -Cl, -Br, -F, -I, -OH, -CN, -C₁-C₃-alkyl, C₁-C₆-alkoxy, C₁-C₆-alkoxy substituted with aryl, haloalkyl, thioalkoxy, amino, alkylamino, dialkylamino, mercapto, nitro, carboxaldehyde, carboxy, alkoxycarbonyl and carboxamide. In addition, any one substitutent may be an aryl, heteroaryl, or heterocycloalkyl group.

Numerous asymmetric centers may exist in the compounds of the present invention. Except where otherwise noted, the present invention contemplates the various stereoisomers and mixtures thereof. Accordingly, whenever a bond is represented by a wavy line, it is intended that a mixture of stereo-orientations or an individual isomer of assigned or unassigned orientation may be present.

As used herein, the term "pharmaceutically acceptable salt" refers to those salts which are, within the scope of sound medical judgment, suitable for use in contact with the tissues of humans and lower animals without undue toxicity, irritation, allergic response and the like, and are commensurate with a reasonable benefit/risk ratio. Pharmaceutically acceptable salts are well known in the art. For example, S. M. Berge, *et al.* describe pharmaceutically acceptable salts in detail in J. Pharmaceutical sciences, 66: 1-19 (1977), incorporated herein by reference. The salts can be prepared *in situ* during the final isolation and purification of the compounds of the invention, or separately by reacting the free base function with a suitable organic acid. Examples of pharmaceutical ly acceptable, nontoxic acid addition salts are salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid or malonic acid or by using other methods used in the art such as ion exchange. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate. lauryl sulfate, malate, maleate, malonate, methanesutfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, *p*-toluenesulfonate, undecanoate, valerate salts. Representative alkali or alkaline earth metal salts include sodium, lithium, potassium, calcium, magnesium. Further pharmaceutically acceptable salts include, when appropriate, nontoxic ammonium, quaternary ammonium, and amine cations formed using counterions such as halide, hydroxide, carboxylate, sulfate, phosphate, nitrate, loweralkyl sulfonate and aryl sulfonate.

As used herein, the term "pharmaceutically acceptable ester" refers to esters which hydrolyze *in vivo* and include those that break down readily in the human body to leave the parent compound or a salt thereof. Suitable ester groups include, for example, those derived from pharmaceutically acceptable aliphatic carboxylic acids, particularly alkanoic, alkenoic, cycloalkanoic and alkanedioic acids, in which each alkyl or alkenyl moiety advantageously has not more than 6 carbon atoms. Examples of particular esters includes formates, acetates, propionates, butyrates, acrylates and ethylsuccinates.

The term "pharmaceutically acceptable prodrugs" as used herein refers to those prodrugs of the compounds of the present invention which are, within the scope of sound medical judgment, suitable for use in contact with the tissues of humans and lower animals with undue toxicity, irritation, allergic response, commensurate with a reasonable benefit/risk ratio, and effective for their intended use, as well as the zwitterionic forms, where possible, of the compounds of the invention. The term "prodrug" refers to compounds that are rapidly transformed *in vivo* to yield the parent compound of the previously formula, for example by hydrolysis in blood. A thorough discussion is provided in T. Higuchi and V. Stella, Pro-drugs as Novel Delivery Systems, Vol. 14 of the A.C.S. Symposium Series, and in Edward B. Roche, ed., Bioreversible Carriers in Drug Design, American Pharmaceutical Association and Pergamon Press, 1987, both of which are incorporated herein by reference.

### Preferred Embodiments

In a first embodiment of the invention is a compound having the formula (I) as described previously. Compounds of formula (I) also have utility as intermediates in the preparation of compounds of formula (II)-(V) of the invention.

In a second embodiment of the invention is a compound having the formula (II) as described previously.

In a third embodiment of the invention is a compound having the formula (III) as described previously.

In a fourth embodiment of the invention is a compound having the formula (IV) as described previously.

In a fifth embodiment of the invention is a compound having the formula (V) as described previously.

Representative compounds of the invention are those selected from the group consisting of
Compound of Formula (I), R is -CH₂CH=CH-(3-quinolyl), R^{p} is H, R¹ is methyl, R² is hydrogen;
Compound of formula (II), R is -CH₂CH=CH-(3-quinolyl), R^{p} is acetyl, R¹ is H, R² is CH₃, W is absent, R^{w} is H;
Compound of Formula (II); R is -CH₂CH=CH-(3-quinolyl), R^{p} is H, W is absent, R^{w} is H, R¹ is H, R² is CH₃;
Compound of Formula (II): R is -CH₂CH=CH-(3-quinolyl), R^{p} is H, W is absent, R^{w} is H, R¹ is acetyl, R² is CH₃;
Compound of Formula (II); R is -CH₂CH=CH-(3-quinolyl), R^{p} is H, W is absent, R^{w} is H, R¹ is CH₂C(O)-O-CH₂CH₃, R² is CH₃;
Compound of Formula (II); R is -CH₂CH=CH-(3-(quinolyl), R^{p} is H, W is absent, R^{w} is H, R¹ is CH₂CH=CH₂, R² is CH₃;
Compound of Formula (II); R is -CH₂CH=CH-(3-quinolyl), R^{p} is H, W is absent, R"' is H, R¹ is CH₂CH₂F, R² is CH₃;
Compound of Formula (II): R is -CH₂CH=CH-(3-quinolyl), R^{p} is H, W is absent, R"' is H, R¹ is CH₂-phenyl, R² is CH₃;
Compound of Formula (II); R is -CH₂CH=CH-(3-quinolyl), R^{p} is H, W is absent, R^{w} is H, R¹ is CH₂-CN. R² is CH₃;
Compound of Formula (II): R is -CH₂CH=CH-(3-quinolyl), R^{p} is H, W is absent, R^{w} is H, R¹ is CH₂-C≡CH, R² is CH₃;
Compound of Formula (II); R is -CH₂CH=CH-(3-quinolyl), R^{p} is H, W is absent, R^{w} is H, R¹ is CH₂CH₂CH₃, R² is CH₃;
Compound of Formula (II); R is -CH₂CH=CH-(3-quinolyl), R^{p} is H, W is absent, R^{w} is H, R¹ is CH₂-cyclopropyl, R² is CH₃;
Compound of Formula (II); R is -CH₂CH=CH-(3-quinolyl), R^{p} is H, W is absent, R^{w} is H, R¹ is cyclopropyl, R² is CH₃;
Compound of Formula (II); R is -CH₂CH=CH-(3-quinolyl), R^{p} is H, W is absent, R^{w} is H, R¹ is CH₂-(3-pyridyl), R² is CH₃;
Compound of Formula (II); R is -CH₂CH=CH-(3-quinolyl), R^{p} is H, W is absent, R^{w} is H, R¹ is CH₂-(cyclo-C₃H₅), R² is CH₃;
Compound of Formula (II); R is -CH₂CH=CH-(3-quinolyl), R^{p} is H, W is absent, R^{w} is H, R¹ is CH₂CH₂CH₃, R² is CH₃;
Compound of Formula (II): R is -CH₂CH=CH-(3-quinolyl), R^{p} is H, W is absent, R^{w} is H, R¹ is CH₂CH=CHC₆H₅, R² is CH₃;
Compound of Formula (II); R is -CH₂CH=CH-(3-quinolyl), R^{p} is H, W is absent, R^{w} is H, R¹ is CH₂C(=CH₂)C(O)OCH₃, R² is CH₃;
Compound of Formula (II); R is -CH₂CH=CH-(3-quinolyl), R^{p} is H, W is absent, R^{w} is H, R¹ is CH₂C(=CH₂)CH₃, R² is CH₃;
Compound of Formula (II); R is -CH₂CH=CH-(3-quinolyl), R^{p} is H, W is absent, R^{w} is H, R¹ is cyclo-C₃H₅, R² is CH₃;
Compound of Formula (II): R is -CH₂CH=CH-(3-quinolyl), R^{p} is H, W is absent, R^{w} is H, R¹ is CH₂-(3-pyridyl), R² is CH₃;
Compound of Formula (II); R is -CH₂CH=CH-(3-quinolyl), R^{p} is H, W is absent, R^{w} is H, R¹ is CH₂-(3-hydroxyphenyl), R² is CH₃;
Compound of Formula (II): R is -CH₂CH=CH-(3-quinolyl), R^{p} is H, W is absent, R^{w} is H, R¹ is CH₂-(2-hydroxy-3-*tert*-butyl-5-methylphenyl), R² is CH₃;
Compound of Formula (II); R is -CH₂CH=CH-(3-quinolyl), R^{p} is H, W is absent, R^{w} is H, R¹ is CH₂-(2-hydroxy-3,4-dimethylphenyl), R² is CH₃;
Compound of Formula (II): R is -CH₂CH=CH-(3-quinolyl), R^{p} is H, W is absent, R^{w} is H, R¹ is CH₂-(2-hydroxy-3-methoxy-5-(2-propenyl)phenyl), R² is CH₃;
Compound of Formula (II): R is -CH₂CH=CH-(3-quinolyl), R^{p} is H, W is absent, R^{w} is H, R¹ is CH₂-(2-hydroxy-3-methoxy-5-methylphenyl), R² is CH₃;
Compound of Formula (II): R is -CH₂CH=CH-(3-quinolyl), R^{p} is H, W is absent, R^{w} is H, R¹ is CH₂-(2-hydroxy-5-cyclopentylphenyl), R² is CH₃;
Compound of Formula (II); R is -CH₂CH=CH-(3-quinolyl), R^{p} is H, W is absent, R^{w} is H, R¹ is CH₂-(2-hydroxy-5-carboxamidophenyl), R² is CH₃;
Compound of Formula (II); R is -CH₂CH=CH-(3-quinolyl), R^{p} is H, W is absent, R^{w} is H, R¹ is is CH₂-(2-hydroy-3-methoxy-5-(2-methoxycarbonylethyl)phenyl), R² is CH₃;
Compound of Formula (II); R is -CH₂CH=CH-(3-quinolyl), R^{p} is H, W is absent, R^{w} is H, R¹ is CH₂-(2-hydroxy-3-methyl-5-fluorophenyl), R² is CH₃;
Compound of Formula (II); R is -CH₂CH=CH-(3-quinolyl), R^{p} is H, W is absent, R^{w} is H, R¹ is CH₂-(2-hydroxy-3-methoxy-5-acetylphenyl), R² is CH₃;
Compound of Formula (II); R is -CH₂CH=CH-(3-quinolyl), R^{p} is H, W is absent, R^{w} is H, R¹ is CH₂-(2-hydroxy-3-bromophenyl), R² is CH₃;
Compound of Formula (II); R is -CH₂CH=CH-(3-quinolyl), R^{p} is H, W is absent, R^{w} is H, R¹ is CH₂-(2-hydroxy-3-methoxy-3-alkoxycarbonylphenyl), R² is CH₃;
Compound of Formula (II); R is -CH₂CH=CH-(3-quinolyl), R^{p} is H, W is absent, R^{w} is H, R¹ is CH₂-(2-hydroxy-3-ethylphenyl), R² is CH₃;
Compound of Formula (II); R is -CH₂CH=CH-(3-quinolyl), R^{p} is H, W is absent, R^{w} is H, R¹ is CH₂-(2-hydroxy-5-isobutylphenyl), R² is CH₃;
Compound of Formula (II); R is -CH₂CH=CH-(3-quinolyl), R^{p} is H, W is absent, R^{w} is H, R¹ is CH₂-(2-hydroxy-3-methyl-5-diethytamino-6-methylphenyl), R² is CH₃;
Compound of Formula (II); R is -CH₂CH=CH-(3-quinolyl), R^{p} is H, W is absent, R^{w} is H, R¹ is CH₂-(2-hydroxy-4-methyl-5-bromo-6-methylphenyl), R² is CH₃; and
Compound of Formula (II): R is -CH₂CH=CH-(3-quinolyl), R^{p} is H, W is absent, R^{w} is H, R¹ is CH₂-(2-hydroxy-3-hydroxymethylphenyl), R² is CH₃.

### Antibacterial Activity

Representative compounds of the present invention were assayed *in vitro* for antibacterial activity as follows: Twelve petri dishes containing successive aqueous dilutions of the test compound mixed with 10 mL of sterilized Brain Heart infusion (BHI) agar (Difco 0418-01-5) were prepared. Each plate was inoculated with 1:100 (or 1:10 for slow-growing strains, such as *Micrococcus* and *Streptococcus)* dilutions of up to 32 different microorganisms, using a Steers replicator block. The inoculated plates were incubated at 35-37 °C for 20 to 24 hours. In addition, a control plate, using BHI agar containing no test compound, was prepared and incubated at the beginning and end of each test.

An additional plate containing a compound having known susceptibility patterns for the organisms being tested and belonging to the same antibiotic class as the test compound was also prepared and incubated as a further control, as well as to provide test-to-test comparability. Erythromycin A was used for this purpose.

After incubation, each plate was visually inspected. The minimum inhibitory concentration (MIC) was defined as the lowest concentration of drug yielding no growth, a slight haze, or sparsely isolated colonies on the inoculum spot as compared to the growth control. The results of this assay, shown below in Table 2 demonstrate the antibacterial activity of the compounds of the invention.

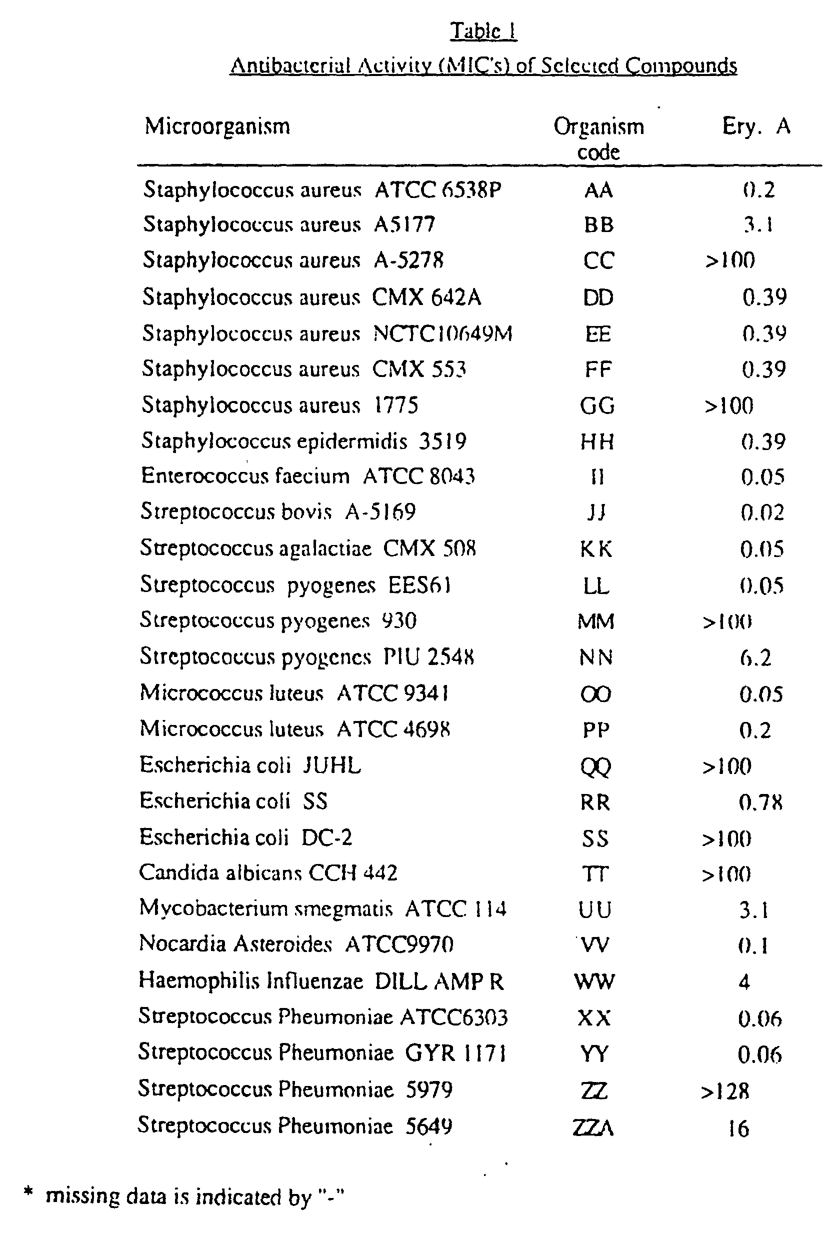

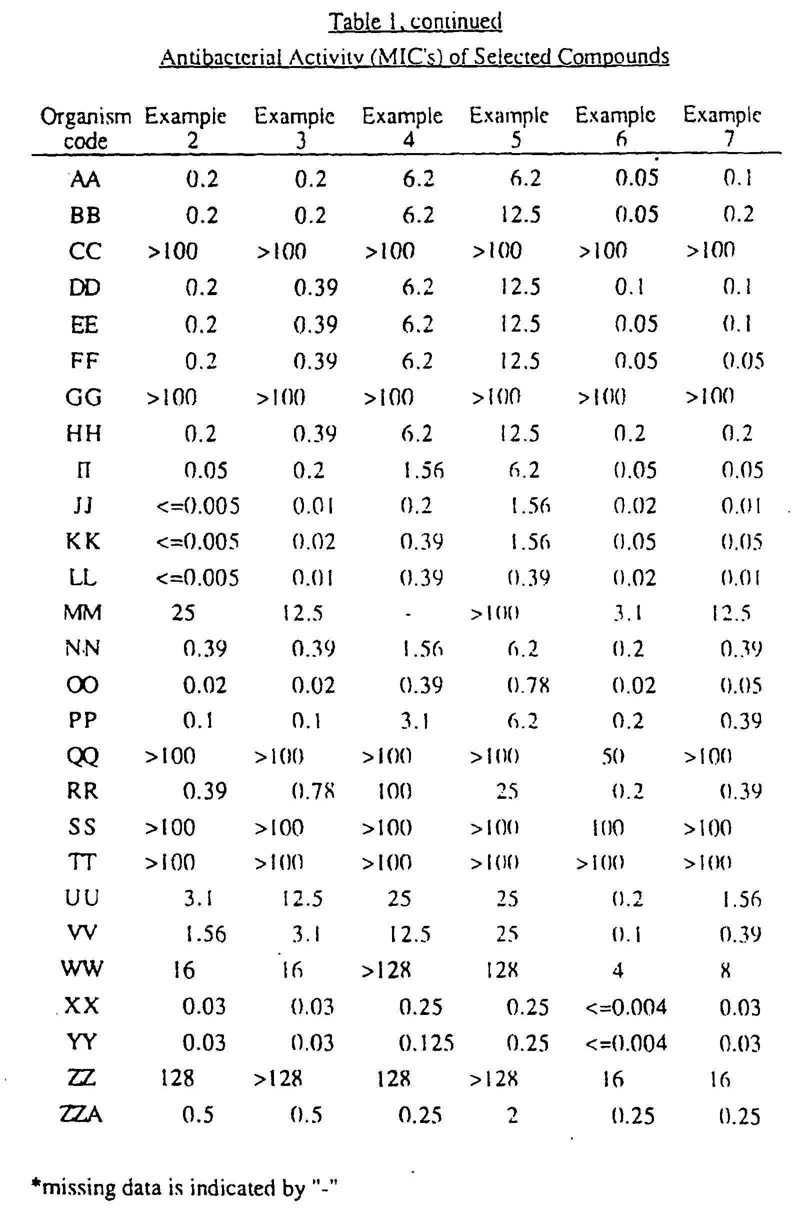

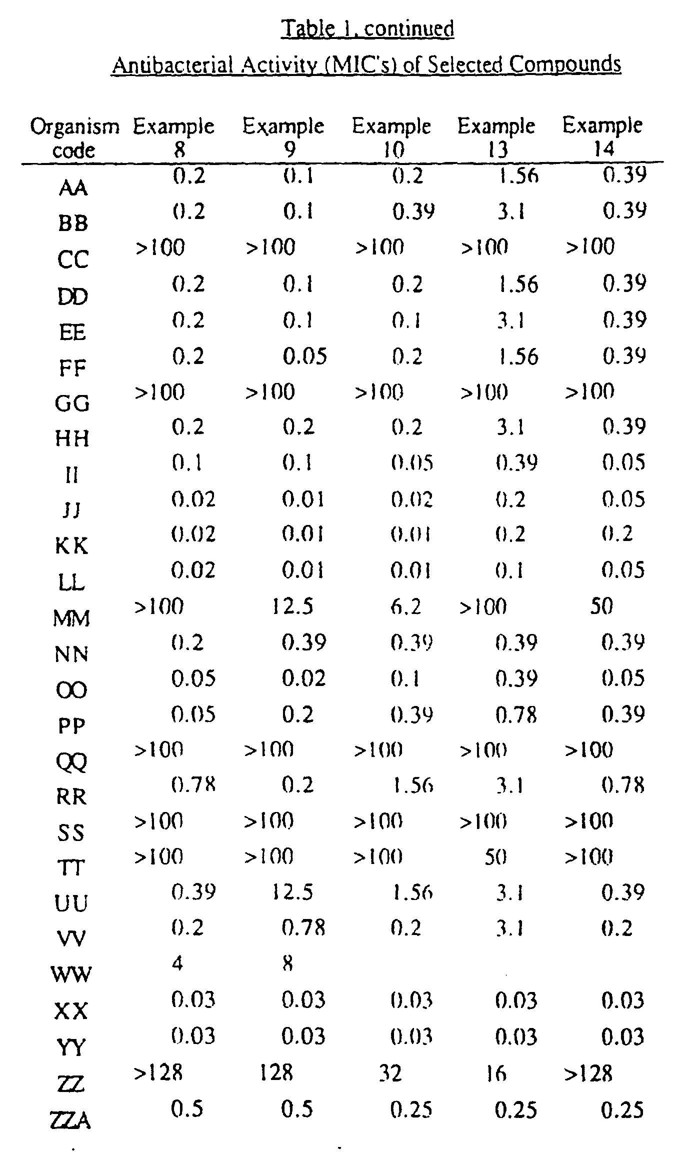

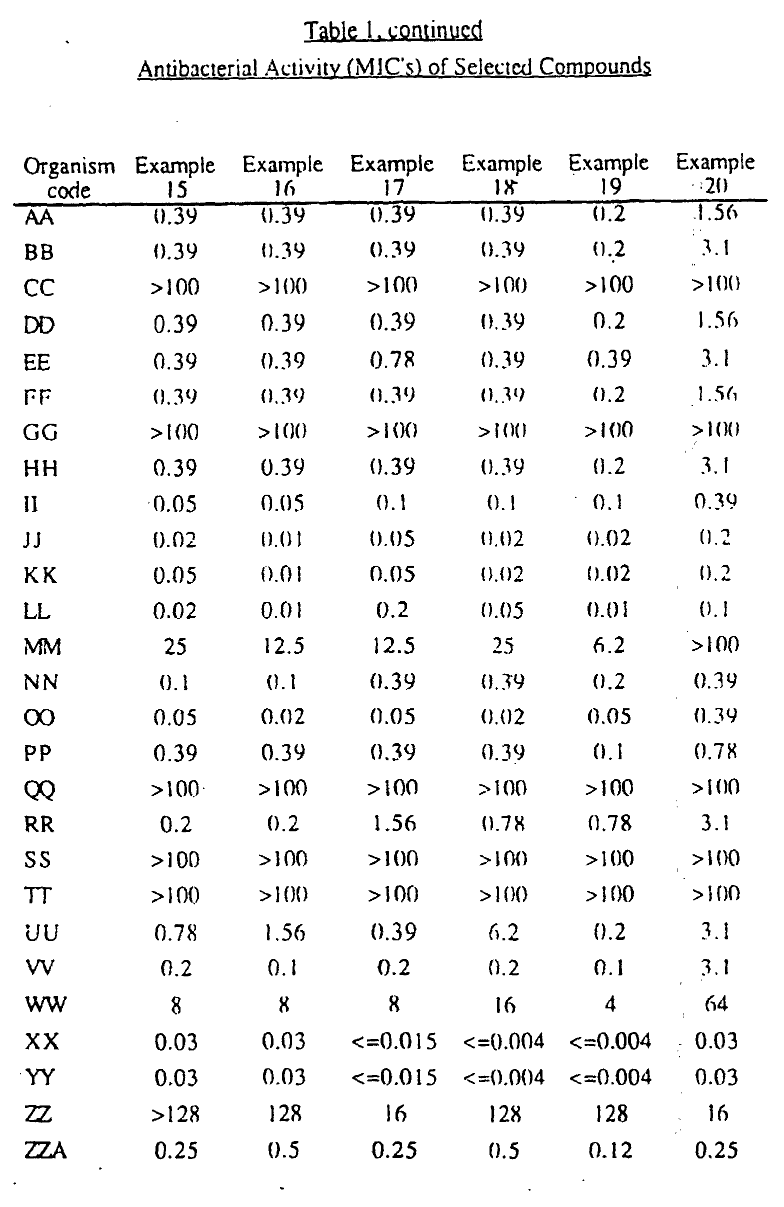

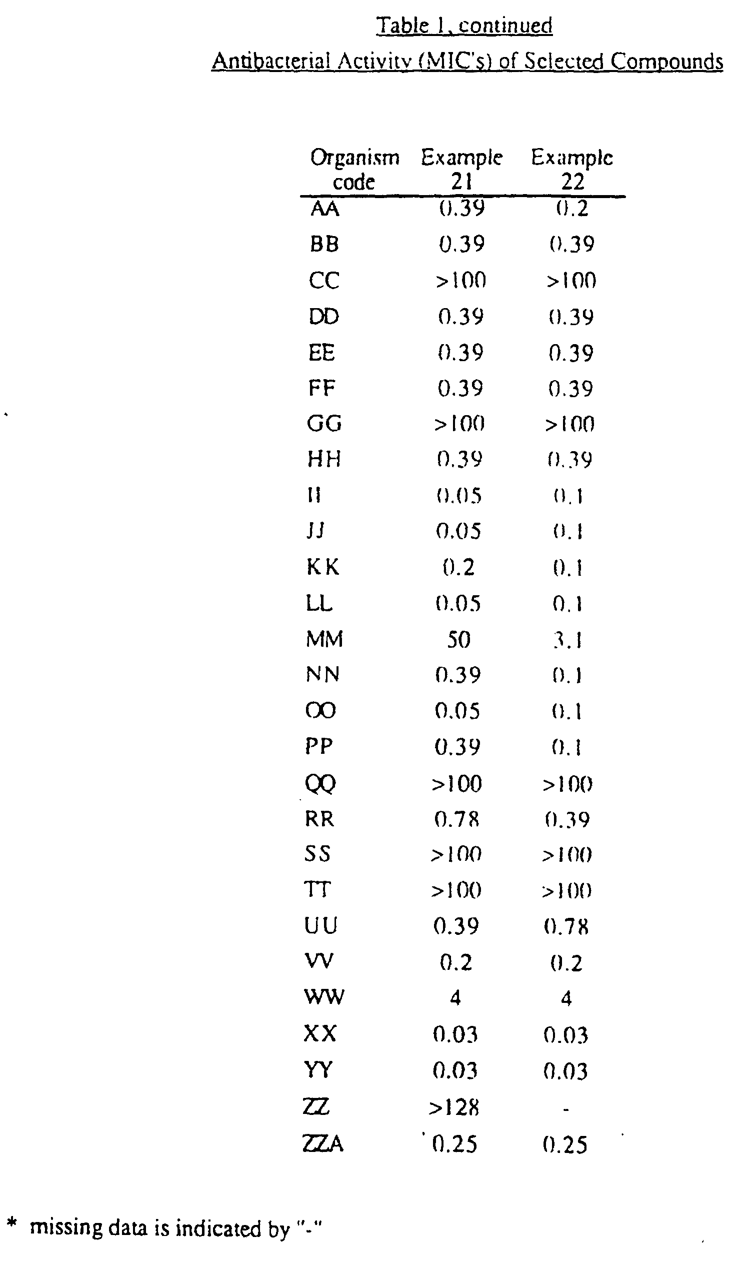

### Pharmaceutical Compositions

The pharmaceutical compositions of the present invention comprise a therapeutically effective amount of a compound of the present invention formulated together with one or more pharmaceutically acceptable carriers. As used herein, the term "pharmaceutically acceptable carrier" means a non-toxic, inert solid, semi-solid or liquid filler, diluent, encapsulating material or formulation auxiliary of any type. Some examples of materials which can serve as pharmaceutically acceptable carriers are sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository waxes; oils such as peanut oil, cottonseed oil; safflower oil; sesame oil; olive oil; corn oil and soybean oil; glycols; such a propylene glycol; esters such as ethyl oleate and ethyl laurate; agar; buffering agents such as magnesium hydroxide and aluminum hydroxide; alginic acid; pyrogen-free water; isotonic saline; Ringer's solution; ethyl alcohol, and phosphate buffer solutions, as well as other non-toxic compatible lubricants such as sodium lauryl sulfate and magnesium stearate, as well as coloring agents, releasing agents, coating agents, sweetening, flavoring and perfuming agents, preservatives and antioxidants can also be present in the composition, according to the judgment of the formulator. The pharmaceutical compositions of this invention can be administered to humans and other animals orally, rectally, parenterally, intracisternally, intravaginally, intraperitoneally, topically (as by powders, ointments, or drops), bucally, or as an oral or nasal spray.

Liquid dosage forms for oral administration include pharmaceutically acceptable emulsions, microemulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, the oral compositions can also include adjuvants such as wetting agents, emulsifying and suspending agents, sweetening, flavoring, and perfuming agents.

Injectable preparations, for example, sterile injectable aqueous or oleaginous suspensions may be formulated according to the known art using suitable dispersing or wetting agents and suspending agents. The sterile injectable preparation may also be a sterile injectable solution, suspension or emulsion in a nontoxic parenterally acceptable diluent or solvent for example, as a solution in 1,3-butanediol. Among the acceptable vehicles and solvents that may be employed are water. Ringer's solution, U.S.P. and isotonic sodium chloride solution. In addition, sterile, fixed oils are conventionally employed as a solvent or suspending medium. For this purpose any bland fixed oil can be employed including synthetic mono- or diglycerides. In addition, fatty acids such as oleic acid are used in the preparation of injectables.

The injectable formulations can be sterilized, for example, by filtration through a bacterial-retaining filter, or by incorporating sterilizing agents in the form of sterile solid compositions which can be dissolved or dispersed in sterile water or other sterile injectable medium prior to use.

In order to prolong the effect of a drug, it is often desirable to slow the absorption of the drug from subcutaneous or intramuscular injection. This may be accomplished by the use of a liquid suspension of crystalline or amorphous material with poor water solubility. The rate of absorption of the drug then depends upon its rate of dissolution which, in turn, may depend upon crystal size and crystalline form. Alternatively, delayed absorption of a parenterally administered drug form is accomplished by dissolving or suspending the drug in an oil vehicle. Injectable depot forms are made by forming microencapsule matrices of the drug in biodegradable polymers such as polylactide-polyglycolide. Depending upon the ratio of drug to polymer and the nature of the particular polymer employed, the rate of drug release can be controlled. Examples of other biodegradable polymers include poly(orthoesters) and poly(anhydrides) Depot injectable formulations are also prepared by entrapping the drug in liposomes or microemulsions which are compatible with body tissues.

Compositions for rectal or vaginal administration are preferably suppositories which can be prepared by mixing the compounds of this invention with suitable non-irritating excipients or carriers such as cocoa butter, polyethylene glycol or a suppository wax which are solid at ambient temperature but liquid at body temperature and therefore melt in the rectum or vaginal cavity and release the active compound.

Solid dosage forms for oral administration include capsules, tablets, pills, powders, and granules. In such solid dosage forms, the active compound is mixed with at least one inert, pharmaceutically acceptable excipient or carrier such as sodium citrate or dicalcium phosphate and/or a) fillers or extenders such as starches, lactose, sucrose, glucose, mannitol, and silicic acid, b) binders such as, for example, carboxymethylcellulose, alginates, gelatin, polyvinylpyrrolidinone, sucrose, and acacia, c) humectants such as glycerol, d) disintegrating agents such as agar-agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. In the case of capsules, tablets and pills, the dosage form may also comprise buffering agents.

Solid compositions of a similar type may also be employed as fillers in soft and hard-filled gelatin capsules using such excipients as lactose or milk sugar as well as high molecular weight polyethylene glycols.

The solid dosage forms of tablets, dragees, capsules, pills, and granules can be prepared with coatings and shells such as enteric coatings and other coatings well known in the pharmaceutical formulating art. They may optionally contain opacifying agents and can also be of a composition that they release the active ingredient(s) only, or preferentially, in a certain part of the intestinal tract, optionally, in a delayed manner. Examples of embedding compositions which can be used include polymeric substances and waxes.

Solid compositions of a similar type may also be employed as fillers in soft and hard-filled gelatin capsules using such excipients as lactose or milk sugar as well as high molecular weight polyethylene glycols.

The active compounds can also be in micro-encapsulated form with one or more excipients as noted previously. The solid dosage forms of tabtets, dragees, capsules, pills, and granules can be prepared with coatings and shells such as enteric coatings, release controlling coatings and other coatings well known in the pharmaceutical formulating art. In such solid dosage forms the active compound may be admixed with at least one inert diluent such as sucrose, lactose or starch. Such dosage forms may also comprise, as is normal practice, additional substances other than inert diluents, e.g., tableting lubricants and other tableting aids such a magnesium stearate and microcrystalline cellulose. In the case of capsules, tablets and pills, the dosage forms may also comprise buffering agents. They may optionally contain opacifying agents and can also be of a composition that they release the active ingredient(s) only, or preferentially, in a certain part of the intestinal tract, optionally, in a delayed manner. Examples of embedding compositions which can be used include polymeric substances and waxes.

Dosage forms for topical or transdermal administration of a compound of this invention include ointments, pastes, creams, lotions, gels, powders, solutions, sprays, inhalants or patches. The active component is admixed under sterile conditions with a pharmaceutically acceptable carrier and any needed preservatives or buffers as may he required. Ophthalmic formulation, car drops, eye ointments, powders and solutions are also contemplated as being within the scope of this invention.

The ointments, pastes, creams and gels may contain, in addition to an active compound of this invention, excipients such as animal and vegetable fats, oils, waxes, paraffins, starch, tragacanth, cellulose derivatives, polyethylene glycols. silicones, bentonites, silicic acid, talc and zinc oxide, or mixtures thereof.

Powders and sprays can contain, in addition to the compounds of this invention, excipients such as lactose, talc, silicic acid, aluminum hydroxide, calcium silicates and polyamide powder, or mixtures of these substances. Sprays can additionally contain customary propellants such as chlorofluorohydrocarbons.

Transdermal patches have the added advantage of providing controlled delivery of a compound to the body. Such dosage forms can be made by dissolving or dispensing the compound in the proper medium. Absorption enhancers can also be used to increase the flux of the compound across the skin. The rate can be controlled by either providing a rate controlling membrane or by dispersing the compound in a polymer matrix or gel.

According to the methods of treatment of the present invention, bacterial infections are treated or prevented in a patient such as a human or lower mammal by administering to the patient a therapeutically effective amount of a compound of the Invention, in such amounts and for such time as is necessary to achieve the desired result. By a "therapeutically effective amount" of a compound of the invention is meant a sufficient amount of the compound to treat bacterial infections, at a reasonable benefit/risk ratio applicable to any medical treatment. It will be understood, however, that the total daily usage of the compounds and compositions of the present invention will be decided by the attending physician within the scope of sound medical judgment. The specific therapeutically effective dose level for any particular patient will depend upon a variety of factors including the disorder being treated and the severity of the disorder, the activity of the specific compound employed; the specific composition employed; the age, body weight, general health, sex and diet of the patient; the time of administration, route of administration, and rate of excretion of the specific compound employed; the duration of the treatment; drugs used in combination or coincidental with the specific compound employed; and like factors well known in the medical arts.

The total daily dose of the compounds of this invention administered to a human or other mammal in single or in divided doses can be in amounts, for example, from 0.01 to 50 mg/kg body weight or more usually from 0.1 to 25 mg/kg body weight. Single dose compositions may contain such amounts or submultiples thereof to make up the daily dose. In general, treatment regimens according to the present invention comprise administration to a patient in need of such treatment from about 10 mg to about 2000 mg of the compound(s) of this invention per day in single or multiple doses.

In another aspect, the present invention is a process for preparing a compound selected from the group consisting of 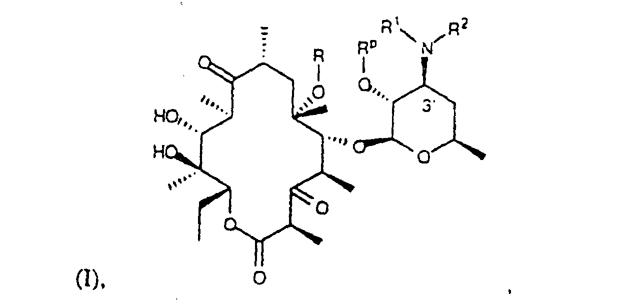 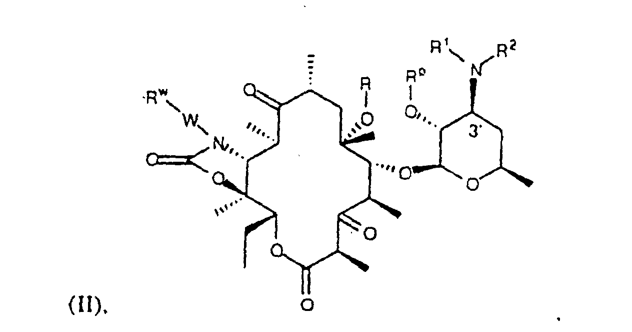 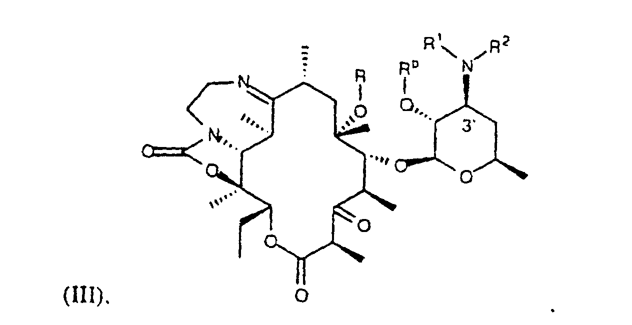 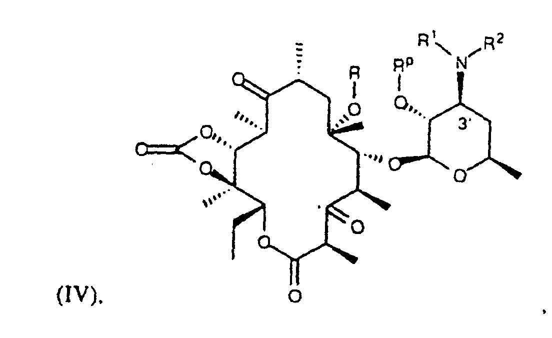 and 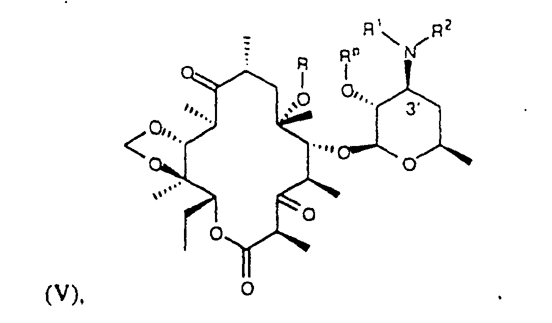 wherein
R¹ and R², with the proviso that R¹ and R² are not both methyl, are independently selected from the group consisting of
(1) hydrogen,
(2) C₁-C₆-alkyl optionally substituted with a substituent selected from the group consisting of
   (a) halogen,
   (b) C₃-C₆-cycloakyl,
   (c) aryl,
   (d) substituted aryl,
   (e) heteroaryl,
   (f) substituted heteroaryl,
   (g) -CHO,
   (h) -C(O)-C₁-C₆-alkyl, and
   (i) -C(O)-NR'R", wherein R' and R" are independently selected from the group consisting of hydrogen, C₁-C₃-alkyl, C₁-C₃-alkyl substituted with aryl, substituted aryl, heteroaryl, and substituted heteroaryl,
(3) C₂-C₆-alkyl optionally substituted with a substituent selected from the group consisting of
   (a) C₁-C₆-alkoxy,
   (b) -NR'R", wherein R' and R" are as previously defined,
   (c) -NH-C(O)-C₁-C₆-alkyl,
   (d) -NH-C(O)-O-C₁-C₆-alkyl,
   (e) -O-C(O)-O-C₁-C₆-alkyl,
   (f) -O-C(O)-C₁-C₆-alkyl,
   (g) -CH(=N-O-C₁-C₆-alkyl),
   (h) -C(=N-O-C₁-C₆-alkyl)-C₁-C₆-alkyl,
   (i) -CH(=N-NH-C₁-C₆-alkyl), and
   (j) -C(=N-NH-C₁-C₆-alkyl)-C₁-C₆-alkyl,
(4) C₃-C₆-alkenyl optionally substituted with a substituent selected from the group consisting of
   (a) halogen,
   (b) C₃-C₆-cycloalkyl,
   (c) aryl,
   (d) substituted aryl,
   (e) heteroaryl,
   (f) substituted heteroaryl,
   (g) -NH-C(O)-C₁-C-₆-alkyl,
   (h) -NH-C(O)-O-C₁-C₆-alkyl,
   (i) -O-C(O)-O-C₁-C₆-alkyl,
   (j) -O-C(O)-C₁-C₆-alkyl,
   (k) -CHO,
   (l) -C(O)-C₁-C₆-alkyl,
   (m) -C(O)-NR'R", wherein R' and R" are as previously defined,
   (n) -CH(=N-O-C₁-C₆-alkyl),
   (o) -C(=N-O-C₁-C₆-alkyl)-C₁-C₆-alkyl,
   (p) -CH(=N-NH-C₁-C₆-alkyl),
   (q) -C(=N-NH-C₁-C₆-alkyl)-C₁-C₆-alkyl, and
   (r) -C(O)-O-C₁-C₆-alkyl,
(5) C₃-C₆-alkynyl optionally substituted with a substituent selected from the group consisting of
   (a) halogen,
   (b) C₃-C₆-cycloalkyl,
   (c) aryl,
   (d) substituted aryl,
   (e) heteroaryl, and
   (f) substituted heteroaryl,
(6) C₃-C₆-cycloalkyl,
(7) -CHO,
(8) -C(O)-C₁-C₆-alkyl,
(9) -C(O)-NR'R", wherein R' and R" are as previously defined, and
(10) -C(O)-O-C₁-C₆-alkyl,
or R¹ and R² taken together may be -(CH₂)ₚ-, wherein p is 3-to-7, which taken together with the nitrogen atom to which they are attached, thus form a heterocyclic ring containing one nitrogen atom and from 3 to 7 carbon atoms;
R is selected from the group consisting of
(1) methyl substituted with a substituent selected from the group consisting of
   (a) -CN,
   (b) -F,
   (c) -CO₂R³ wherein R³ is C₁-C₃-alkyl, aryl-substituted C₁-C₃-alkyl, or heteroaryl-substituted C₁-C₃-alkyl,
   (d) -S(O)ₙ-R³ wherein n is 0, 1, or 2, and R³ is as previously defined,
   (e) -NH-C(O)-R³ where R³ is as previously defined,
   (f) -NH-C(O)-NR⁴R⁵ wherein R⁴ and R⁵ are independently selected from the group consisting of
      (i) hydrogen,
      (ii) C₁-C₃-alkyl
      (iii) C₁-C₃-alkyl substituted with aryl,
      (iv) C₁-C₃-alkyl substituted with substituted aryl,
      (v) C₁-C₃-alkyl substituted with heteroaryl, and
      (vi) C₁-C₃-alkyl substituted with and substituted heteroaryl,
   (g) aryl,
   (h) substituted aryl,
   (i) heteroaryl,
      and
   (j) substituted heteroaryl,
(2) C₂-C₁₀-alkyl,
(3) C₂-C₁₀-alkyl substituted with one or more substituents selected from the group consisting of
   (a) halogen,
   (b) hydroxy,
   (c) C₁-C₃-alkoxy,
   (d) C₁-C₃-alkoxy-C₁-C₃-alkoxy,
   (e) oxo,
   (f) -N₃,
   (g) -CHO,
   (h) -O-SO₂-(substituted C₁-C₆-alkyl),
   (i) -NR⁶R⁷ wherein R⁶ and R⁷ are selected from the group consisting of
      (i) hydrogen,
      (ii) C₁-C₁₂-alkyl,
      (iii) substituted C₁-C₁₂-alkyl,
      (iv) C₁-C₁₂-alkenyl,
      (v) substituted C₁-C₁₂-alkenyl,
      (vi) C₁-C₁₂-alkynyl,
      (vii) substituted C₁-C₁₂-alkynyl,
      (viii) aryl,
      (ix) C₃-C₈-cycloalkyl,
      (x) substituted C₃-C₈-cycloalkyl,
      (xi) substituted aryl,
      (xii) heterocycloalkyl,
      (xiii) substituted heterocycloalkyl,
      (xiv) C₁-C₁₂-alkyl substituted with aryl,
      (xv) C₁-C₁₂-alkyl substituted with substituted aryl,
      (xvi) C₁-C₁₂-alkyl substituted with heterocycloalkyl,
      (xvii) C₁-C₁₂-alkyl substituted with substituted heterocycloalkyl,
      (xviii) C₁-C₁₂-alkyl substituted with C₃-C₈-cycloalkyl,
      (xix) C₁-C₁₂-alkyl substituted with substituted C₃-C₈-cycloalkyl,
      (xx) heteroaryl,
      (xxi) substituted heteroaryl,
      (xxii) C₁-C₁₂-alkyl substituted with heteroaryl,
         and
      (xxiii) C₁-C₁₂-alkyl substituted with substituted heteroaryl,
         or
      R⁶ and R⁷ are taken together with the atom to which they are attached form a 3-10 membered heterocycloalkyl ring which may be substituted with one or more substituents independently selected from the group consisting of
      (i) halogen,
      (ii) hydroxy,
      (iii) C₁-C₃-alkoxy,
      (iv) C₁-C₃-alkoxy-C₁-C₃-alkoxy,
      (v) oxo,
      (vi) C₁-C₃-alkyl,
      (vii) halo-C₁-C₃-alkyl,
         and
      (vii) C₁-C₃-alkoxy-C₁-C₃-alkyl,
      (j) -CO₂R³ wherein R³ is as previously defined,
      (k) -C(O)-NR⁴R⁵ wherein R⁴ and R⁵ are as previously defined,
      (l) =N-O-R³ wherein R³ is as previously defined,
      (m) -C≡N,
      (n) -O-S(O)ₙ-R³ wherein n and R³ are as previously defined,
      (o) aryl,
      (p) substituted aryl,
      (q) heteroaryl,
      (r) substituted heteroaryl,
      (s) C₃-C₈-cycloalkyl,
      (t) substituted C₃-C₈-cycloalkyl,
      (u) C₁-C₁₂-alkyl substituted with heteroaryl,
      (v) heterocycloalkyl,
      (w) substituted heterocycloalkyl,
      (x) -NH-C(O)-R³ where R³ is as previously defined,
      (y) -NH-C(O)-NR⁴R⁵ wherein R⁴ and R⁵ are as previously defined,
      (z) =N-NR⁶R⁷ wherein R⁶ and R⁷ are as previously defined,
      (aa) =N-R³ wherein R³ is as previously defined,
      (bb) =N-NH-C(O)-R⁴ wherein R⁴ is as previously defined,
         and
      (cc) =N-NH-C(O)-NR⁴R⁵ wherein R⁴ and R⁵ are as previously defined,
(4) C₃-alkenyl substituted with a moiety selected from the group consisting of
   (a) halogen,
   (b) -CHO,
   (c) -CO₂R³ where R³ is as previously defined,
   (d) -C(O)-R⁴ where R⁴ is as previously defined,
   (e) -C(O)-NR⁴R⁵ wherein R⁴ and R⁵ are as previously defined,
   (f) -C≡N,
   (g) aryl,
   (h) substituted aryl,
   (i) heteroaryl,
   (j) substituted heteroaryl,
   (k) C₃-C₇-cycloalkyl,
      and
   (l) C₁-C₁₂-alkyl substituted with heteroaryl,
(5) C₄-C₁₀-alkenyl,
(6) C₄-C₁₀-alkenyl substituted with one or more substituents selected from the group consisting of
   (a) halogen,
   (b) C₁-C₃-alkoxy,
   (c) oxo,
   (d) -CHO,
   (e) -CO₂R³ where R³ is as previously defined,
   (f) -C(O)-NR⁴R⁵ wherein R⁴ and R⁵ are as previously defined,
   (g) -NR⁶R⁷ wherein R⁶ and R⁷ are as previously defined,
   (h) =N-O-R³ wherein R³ is as previously defined,
   (i) -C≡N,
   (j) -O-S(O)ₙ-R³ wherein n is 0, 1, or 2 and R³ is as previously defined,
   (k) aryl,
   (I) substituted aryl,
   (m) heteroaryl,
   (n) substituted heteroaryl,
   (o) C₃-C₇-cycloalkyl,
   (p) C₁-C₁₂-alkyl substituted with heteroaryl,
   (q) -NH-C(O)-R³ where R³ is as previously defined,
   (r) -NH-C(O)-NR⁴R⁵ wherein R⁴ and R⁵ are as previously defined,
   (s) =N-NR⁶R⁷ wherein R⁶ and R⁷ are as previously defined,
   (t) =N-R³ wherein R³ is as previously defined,
   (u) =N-NH-C(O)-R³ where R³ is as previously defined,
      and
   (v) =N-NH-C(O)-NR⁴R⁵ wherein R⁴ and R⁵ are as previously defined,
(7) C₃-C₁₀-alkynyl,
   and
(8) C₃-C₁₀-alkynyl substituted with one or more substituents selected from the group consisting of
   (a) trialkylsilyl,
   (b) aryl,
   (c) substituted aryl,
   (d) heteroaryl,
      and
   (e) substituted heteroaryl,
with the proviso that when R is allyl and R¹ is methyl, R² is not H;
R^{p} is hydrogen or a hydroxy protecting group;
R^{w} is selected from the group consisting of
(1) hydrogen,
(2) C₁-C₆-alkyl, optionally substituted with one or more substituents selected from the group consisting of
   (a) aryl,
   (b) substituted aryl,
   (c) heteroaryl,
   (d) substituted heteroaryl,
(3) a group selected from option (2) as previously defined further substituted with - CH₂-M-R⁸, wherein M is selected from the group consisting of
   (i) -O-,
   (ii) - NH-,
   (ii) -N(CH₃)-,
   (iv) -S(O)ₙ-, wherein n is as described previously,
   (v) -NH-C(O)-, and
   (vi) -C(O)-NH-,
      and
   R⁸ is selected from the group consisting of
   (i) -(CH₂)ₙ-aryl, wherein n is as described previously,
   (ii) -(CH₂)ₙ-substituted aryl, wherein n is as described previously,
   (iii) -(CH₂)ₙ-heteroaryl, wherein n is as described previously,
   (iv) -(CH₂)ₙ-substituted heteroaryl, wherein n is as described previously,
      and
   (v) -(CH₂)ₙ-heterocycloalkyl, wherein n is as described previously;
      and
W is absent or is selected from the group consisting of -O-, -NH- and -N(CH₃)-, the method comprising:
(a) sequentially desmethylating 3'-nitrogen of a compound selected from the group consisting of 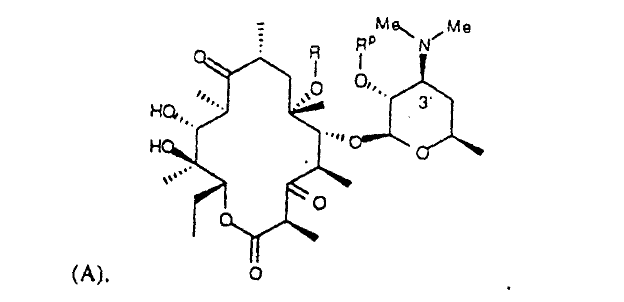 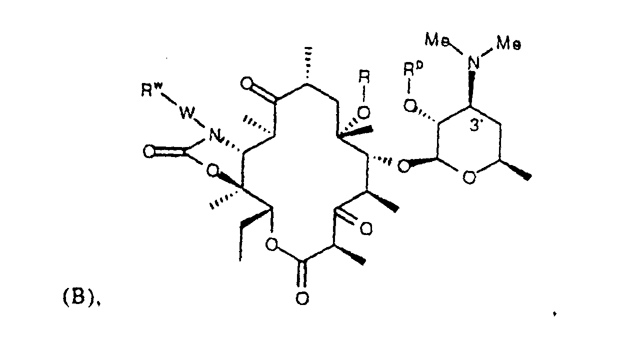 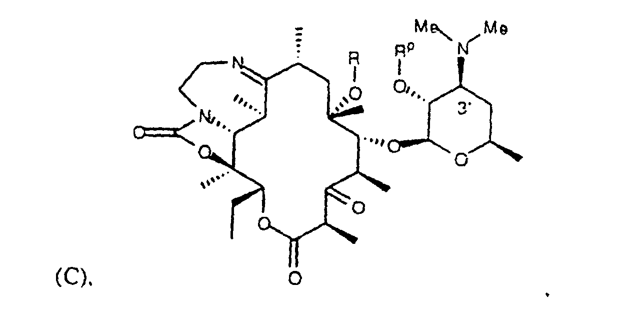 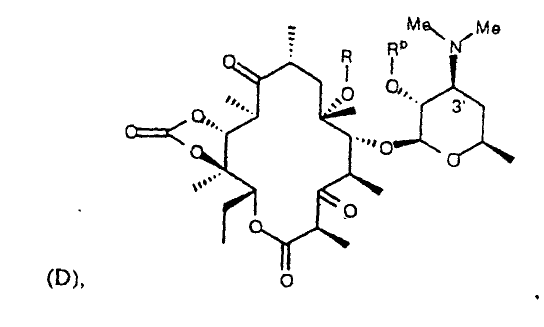 and 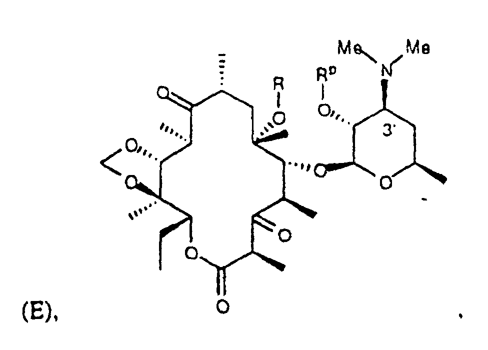 wherein R, R^{p}, W and R^{w} are as defined previously; and
(b) sequentially reacting the compound from step (a) with a R¹-and a R²-precursor compound.

### Abbreviations

Abbreviations which have been used in the descriptions of the scheme and the examples that follow are: AIBN for azobisisobutyronitrile; Bu₃SnH for tributyltin hydride: CDI for carbonyldiimidazole: DBU for 1,8-diazabicyclo[5,4,0]undec-7-ene; DEAD for diethylazodicarboxylate; DMF for dimethylformamide; DMSO for dimethylsulfoxide: DPPA for diphenylphosphoryl azide: Et₃N for triethylamine: EtOAc for ethyl acetate: Et₂O for diethyl ether; EtOH for ethanol; HOAc for acetic acid: MeOH for methanol; NaN(TMS)₂ for sodium bis(trimethylsilyl)amide; NMMO for N-methylmorpholine N-oxide; TEA for triethylamine; THF for tetrahydrofuran: and TPP for triphenylphosphine.

### Synthetic Methods

The compounds and processes of the present invention will be better understood in connection with the following synthetic schemes 1-9 which illustrate the methods by which the compounds of the invention may be prepared. The compounds of the present invention are prepared by the representative methods described below. The groups R¹, R², R, R^{p} and R^{w} are as defined previously.

The preparation of the compounds of the invention of formula (I) - (V) from erythromycin A is outlined in Schemes 1- 9. The preparation of protected erythromycin A is described in the following United States patents, US 4,990,602; US 4,331,803,US 4,680,368, and US 4,670,549 which are incorporated by reference. Also incorporated by reference is European Patent Application EP 260,938.

As shown in Scheme 1, the C-9-carbonyl group of compound 1 is protected with an oxime to give the compound 2, wherein V is =N-O-R³ or =N-O-C(R⁸)(R⁹)-O-R³ where R³ is defined previously and R⁸ and R⁹ are each independently selected from the group consisting of (a) hydrogen, (b) unsubstituted C₁-C₁₂-alkyl, (c) C₁-C₁₂-alkyl substituted with aryl, and (d) C₁-C₁₂-alkyl substituted with substituted aryl, or R⁹ and R¹⁰ taken together with the carbon to which they are attached form a C₃-C₁₂-cycloalkyl ring. An especially preferred carbonyl protecting group V is O-(1-isopropoxycyclohexyl) oxime.

The 2'- and 4"-hydroxy groups of 2 are protected by reaction with a suitable hydroxy protecting reagent, such as those described by T.W. Greene and P.G.M. Wuts in Protective Groups in Organic Synthesis, 2nd ed., John Wiley & Son. Inc., 1991, which is incorporated by reference. Hydroxy protecting groups include, for example, acetic anhydride, benzoic anhydride, benzyl chloroformate, hexamethyldisilazane, or a trialkylsilyl chloride in an aprotic solvent. Examples of aprotic solvents are dichloromethane, chloroform, DMF, tetrahydrofuran (THF), N-methyl pyrrolidinone, dimethylsulfoxide, diethylsulfoxide, N,N-dimethylformamide, N,N-dimethylacetamide, hexamethylphosphoric triamide, a mixture thereof or a mixture of one of these solvents with ether, tetrahydrofuran, 1,2-dimethaxyethane, acetonitrile, ethyl acetate, acetone and the like. Aprotic solvents do not adversely affect the reaction, and are preferably dichloromethane, chloroform, DMF, tetrahydrofuran (THF). N-methyl pyrrolidinone or a mixture thereof. Protection of 2'- and 4"-hydroxy groups of 2 may be accomplished sequentially or simultaneously to provide compound 3 where R^{p} is a hydroxy protecting group. A preferred protecting group R^{p} is trimethylsilyl.

The 6-hydroxy group of compound 3 is then alkylated by reaction with an alkylating agent in the presence of base to give compound 4. Alkylating agents include alkyl chlorides, bromides, iodides or alkyl sulfonates. Specific examples of alkylating agents include allyl bromide, propargyl bromide, benzyl bromide, 2-fluoroethyl bromide, 4-nitrobenzyl bromide, 4-chlorobenzyl bromide, 4-methoxybenzyl bromide, α-bromo-p-tolunitrile, cinnamyl bromide, methyl 4-bromocrotonate, crotyl bromide, 1-bromo-2-pentene, 3-bromo-1-propenyl phenyl sulfone, 3-bromo-1-trimethylsilyl-1-propyne, 3-bromo-2-octyne, 1-bromo-2-butyne, 2-picolyl chloride, 3-picolyl chloride, 4-picolyl chloride, 4-bromomethyl quinoline, bromoacetonitrile, epichlorohydrin, bromofluoromethane, bromonitromethane, methyl-bromoacetate, methoxymethyl chloride, bromoacetamide, 2-bromoacetophenone, 1-bromo-2-butanone, bromo chloromethane, bromomethyl phenyl sulfone, 1,3-dibromo-1-propene, and the like. Examples of alkyl sulfonates are: allyl-O-tosylate, 3-phenylpropyl-O-trifluoromethane sulfonate, n-butyl -O-methanesulfonate and the like. Examples of the solvents used are aprotic solvents such as dimethylsulfoxide, diethylsulfoxide, N,N-dimethylformamide, N,N-dimethylacetamide, N-methyl-2-pyrrolidone, hexamethylphosphoric triamide, a mixture thereof or a mixture of one of these solvents with ether, tetrahydrofuran, 1,2-dimethoxyethane, acetonitrile, ethyl acetate, acetone and the like. Examples of the base which can be used include potassium hydroxide, cesium hydroxide, tetraalkylammonium hydroxide, sodium hydride, potassium hydride, potassium isopropoxide, potassium *tert*-butoxide, potassium isobutoxide.

Additional procedures for further elaboration of the 6-position moiety of the compounds of the invention are described in Schemes 2-4 below.

The deprotection of the 2'- and 4"-hydroxyl groups is then carried out according to methods described in literature, for example, by T.W. Greene and P.G.M. Wuts in Protective Groups in Organic Synthesis, 2nd ed., John Wiley & Son, Inc., 1991, which is incorporated herein by reference. The conditions used for the deprotection of the 2'- and 4"-hydroxyl groups usually results in the conversion of X to =N-OH. (For example, using acetic acid in acetonitrile and water results in the deprotection of the 2'- and 4"-hydroxyl groups and the conversion of X from =N-O-R³ or =N-O-C(R⁸)(R⁹)-O-R³ where R³, R⁸ and R⁹ are as defined previously to =N-OH.) If this is not the case, the conversion is carried out in a separate step.

The deoximation reaction can be carried out according to the methods described in the literature, for example by Greene *(op. cit.)* and others. Examples of the deoximating agent are inorganic sulfur oxide compounds such as sodium hydrogen sulfite, sodium pyrosulfate, sodium thiosulfate, sodium sulfate, sodium sulfite, sodium hydrosulfite, sodium metabisulfite, sodium dithionate, potassium thiosulfate, potassium metabisulfite and the like, Examples of the solvents used are protic solvents such as water, methanol, ethanol, propanol, isopropanol, trimethylsilanol or a mixture of one or more of the mentioned solvents and the like. The deoximation reaction is more conveniently carried out in the presence of an organic acid such as formic acid, acetic acid and trifluoroacetic acid. The amount of acid used is from about 1 to about 10 equivalents of the amount of compound 5 used. In a preferred embodiment, the deoximation is carried out using an organic acid such is formic acid in ethanol and water to give the desired 6-O-substituted erythromycin compound 6.

Schemes 2-4 describe representative procedures for further elaboration of the 6-O-substituted moiety of the compounds of the invention. It will be appreciated by one skilled in the art that the decision as to when to perform these reactions may be dependent upon the presence of other reactive moieties within the molecule. Therefore, suitable protection and deprotection steps may be required, as are well known and applied within the art. It will sometimes be desirable to perform these modifications upon macrolide molecules such as the erythromycin derivative 6. In other instances it will be desirable to perform the operation upon a later intermediate in the preparation of compounds of the invention. Specifically, the modifications may be performed upon certain compounds of the invention, including selected compounds of formulas (I)-(V) wherein R is allyl, in order to prepare additional compounds of formulas (I)-(V).

Scheme 2 illustrates reactions suitable for modification of 6-O-allyl substituted macrolide compounds. For example, compound 7 wherein M' represents a selected macrolide derivative can be further derivatized. The double bond of the allyl compound can be (a) catalytically reduced to give the 6-O-propyl compound 8: (b) treated with osmium tetraoxide to give the 2,3-dihydroxypropyl compound 9 which in turn may be functionalized, such as by esterification with an acylating agent such as an acyl halide or acyl anhydride, at each oxygen atom to give 10; (c) oxidized with m-chloroperoxybenzoic acid in an aprotic solvent to give the epoxy methyl compound 11 which can be opened with nucleophilic compounds, for example, amines or N-containing heteroaryl compounds, to give compounds with N-containing side chains 12; (d) oxidized under Wacker conditions as described by Henry in "Palladium Catalyzed Oxidation of Hydrocarbons", Reidel Publishing Co., Dordrecht, Holland (1980), to give the 6-O-CH₂-C(O)-CH₃ compound 13: and (e) ozonized to give the aldehyde 14 which can in turn converted to oximes 15 and 16 by reaction with H₂NOR³ or H₂NOH respectively, or reductively aminated, such as with a suitable amine in the presence of a borohydride reducing agent or by formation of the imine and subsequent catalytic reduction, to give the amine 17. Reaction of the oxime 16 with diisopropyl carbodiimide in an aprotic solvent in the presence of CuCl gives the nitrile 18. Reaction of 7 with an aryl halide under Heck conditions in the presence of (Pd(II) or Pd(O), phosphine, and amine or inorganic base (see *Organic Reactions,* **1982*,*** 27, 345-390) gives 19. Reduction of the double bond in 19, for example using H₂ and palladium on carbon gives 20.

Representative examples of still further elaboration of the 6-position are shown in Scheme 3. The desired 6-O-substituted compound may be prepared by chemical modification of an initially prepared 6-O-propargyl compound.

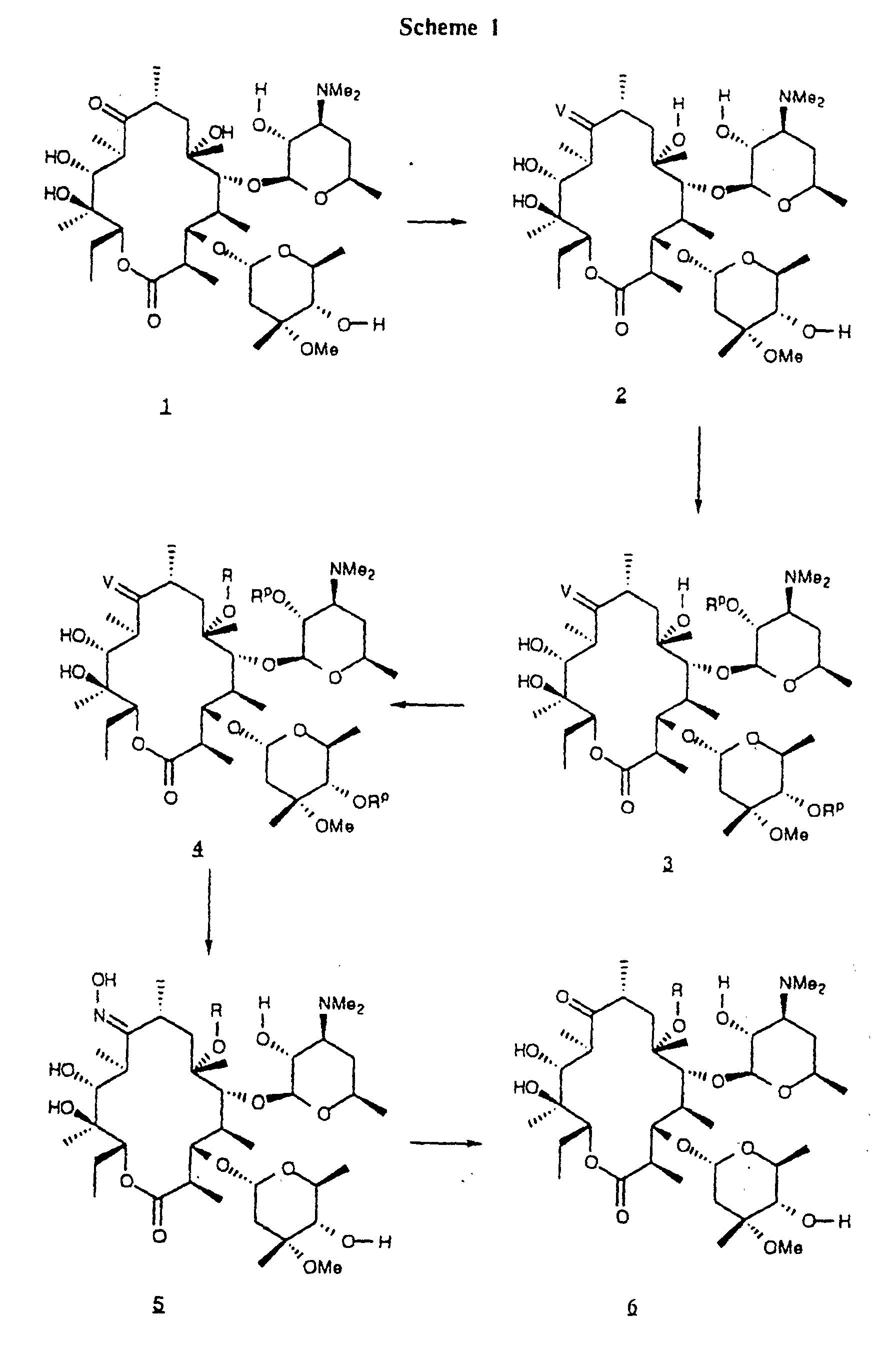

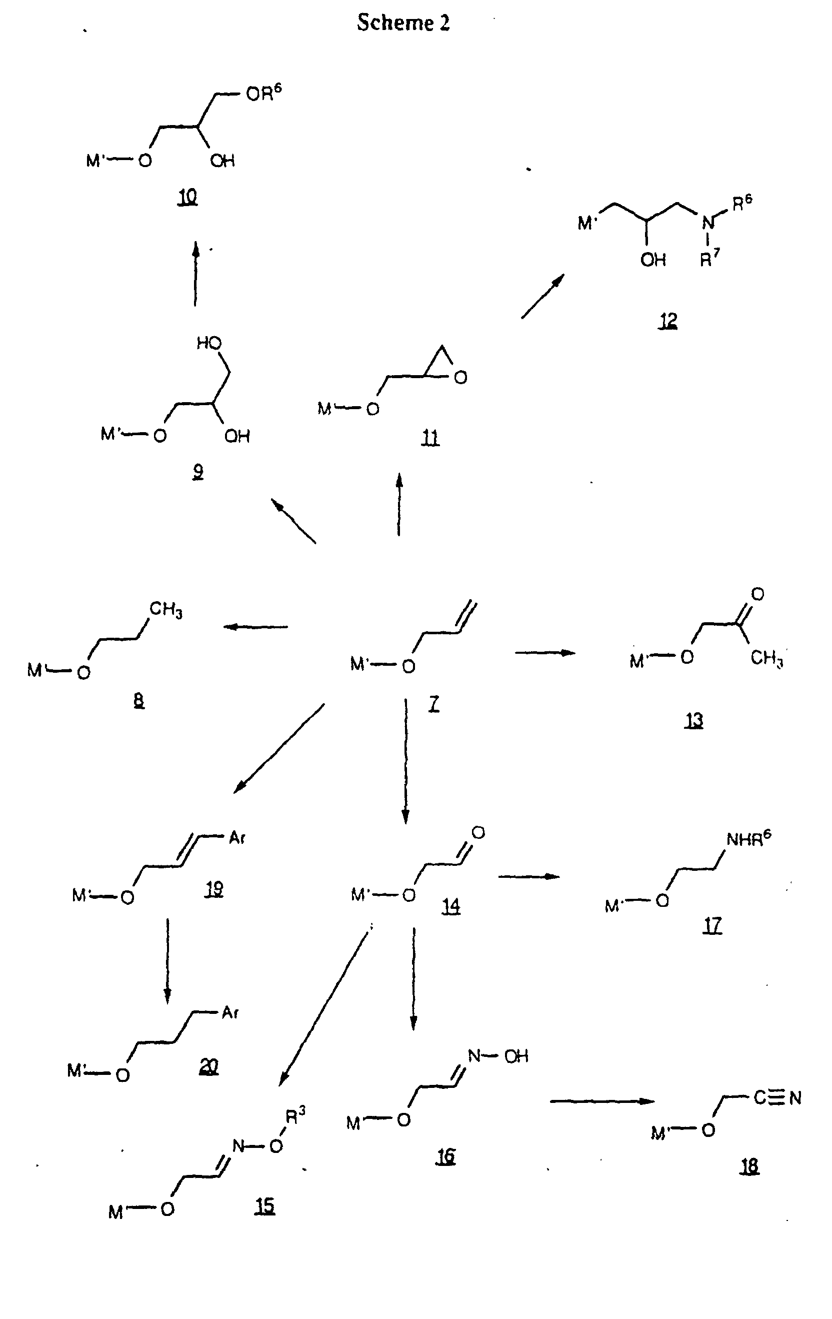

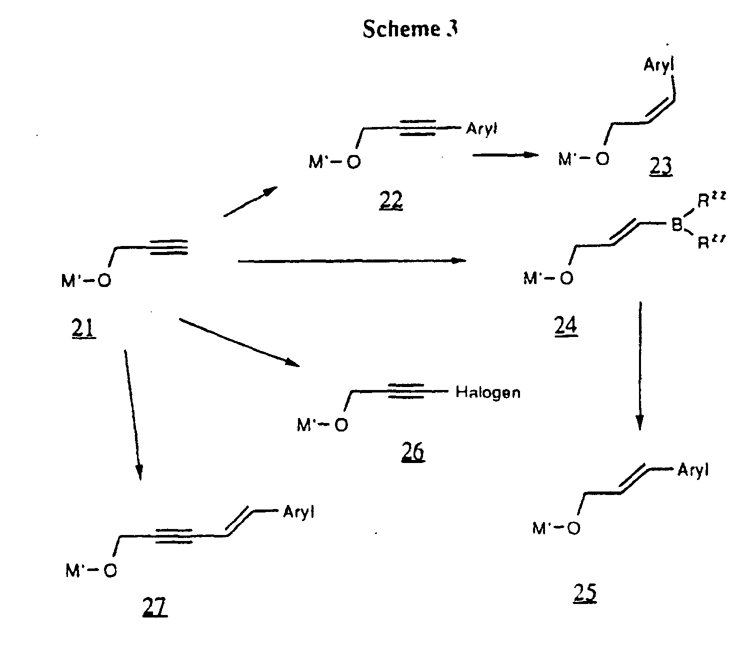

For example, compound 21, which illustrates a compound of the invention where R is propargyl and M' represents the macrolide ring system, can be further derivatized. The triple bond of compound 21 can be treated with an aryl halide, a substituted aryl halide, a heteroaryl halide or substituted heteroaryl halide in the presence of Pd(triphenylphosphine)₂Cl₂ and Cul in the presence of an organic amine, such as triethylamine, to give the compound 22. Compound 22 can be further selectively reduced to the corresponding *cis*-olefin compound 23 by catalytic hydrogenation in ethanol at atmospheric pressure in the presence of 5% Pd/BaSO₄ and quinoline (Rao *et al., J. Org. Chem.,* (**1986**), 51: 4158-4159). Compound 21 may also be treated with a boronic acid derivative HB(OR^{zz}), wherein R^{zz} is H or C₁-C₁₀-alkyl, in an aprotic solvent at 0 °C to ambient temperature to give compounds 24, which are then treated with Pd(triphenylphosphine)₄ and an aryl halide, a substituted aryl halide, an heteroaryl halide or substituted heteroaryl halide under Suzuki reaction conditions to give compounds 25. Compound 21 may also be treated with N-halosuccinimide in acetic acid to give compounds 26. Also, compound 21 may be treated with a substituted alkenyl halide, such as Ar-CH=CH-halogen, wherein Ar is aryl, substituted aryl, heteroaryl or substituted heteroaryl, in the presence of Pd(triphenylphosphine)₂Cl₂ and Cul in the presence of an organic amine, such as triethylamine, to give the appropriately substituted compounds 27.

Scheme 4 describes the preparation of intermediates to compounds of formula (I) of the invention from the 6-substituted erythromycin derivative 6 prepared in Scheme 1. The cladinose moiety of macrolide 6 is removed either by mild aqueous acid hydrolysis or by enzymatic hydrolysis to give 28. Representative acids suitable for this procedure include dilute hydrochloric acid, sulfuric acid, perchloric acid, chloroacetic acid, dichloroacetic acid or trifluoroacetic acid. Suitable solvents for the reaction include methanol, ethanol, isopropanol, butanol, and the like. Reaction times are typically 0.5 to 24 hours, and the reaction temperature is preferably -10 °C to 35 °C.

The 2'-hydroxy group of 28 is protected to give the compound 29 by means of a suitable hydroxy protecting reagent such as acetic anhydride. benzoyl anhydride, benzyl chloroformate or trialkylsilyl chloride in an aprotic solvent, as defined previously, preferably dichloromethane, chloroform, DMF, tetrahydrofuran (THF), N-methyl pyrrolidinone or a mixture thereof. A particularly preferred protecting group R^{p} is benzoate. It is possible to reverse the order of the steps for removing the cladinose and protecting the hydroxy groups without affecting the yield of the process.

The 3-hydroxy group of 29 is oxidized to the ketone 30 using a modified Swern oxidation procedure. Suitable oxidizing agents are N-chlorosuccinimide-dimethyl sulfide or carbodiimide-dimethylsulfoxide. In a typical example, 29 is added into a pre-formed N-chlorosuccinimide and dimethyl sulfide complex in a chlorinated solvent such as methylene chloride at -10 °C to 25 °C. After being stirred for about 0.5 to 4 hours, a tertiary amine, such as triethylamine or Hunig's base, for example, is added to produce the ketone 30 wherein R^{p} is a hydroxy protecting group. The conversion of intermediate compound 30 to a compound of the invention is shown below in Scheme 9.

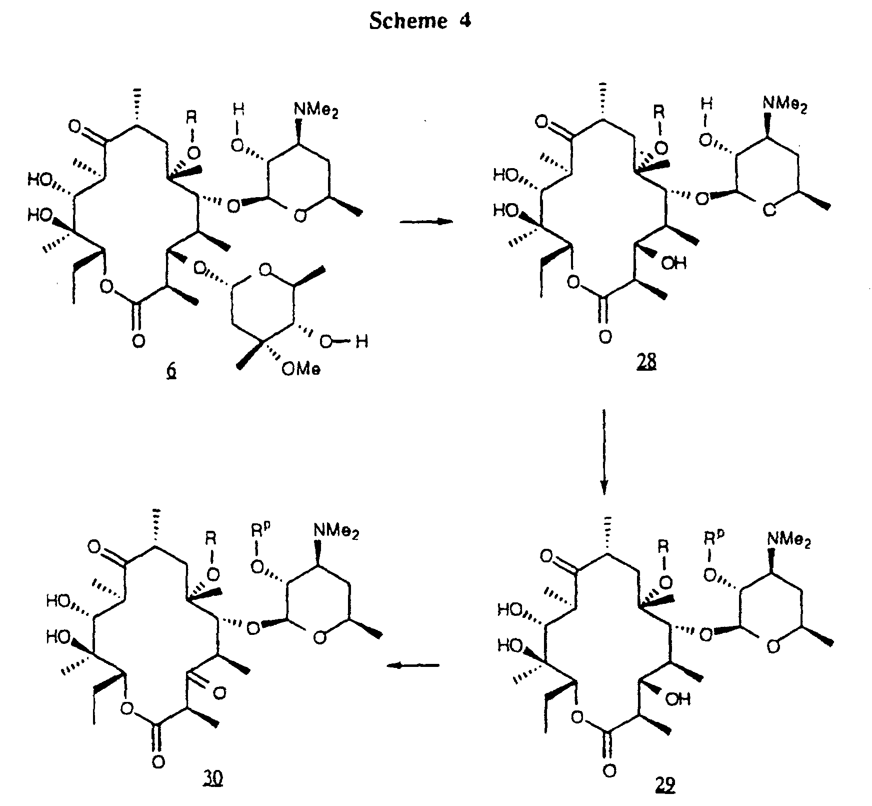

Scheme 5 illustrates the preparation of the compounds of formula (II). Accordingly, compound 6 is first protected with a suitable hydroxy protecting group to give compound 31, by the procedures referenced previously. Compound 31 is then treated with an excess of sodium hexamethyldisilazide or a hydride base in the presence of carbonyldiimidazole in an aprotic solvent for 8 to 24 hours at about -30 °C to room temperature to give compound 32. The hydride base may be, for example, sodium hydride, potassium hydride, or lithium hydride, and the aprotic solvent may be one as defined previously. The reaction is preferably maintained under an inert atmosphere, such as nitrogen or argon, for example. The reaction may require cooling or heating from about -20 °C to about 70 °C, depending on the conditions used, and preferably from about 0 °C to about room temperature. The reaction requires about 0.5 hours to about 10 days, and preferably about 1-5 days, to complete. Portions of this reaction sequence follow the procedure described by Baker *et al., J. Org. Chem.,* **1988**, *53*, 2340, which is incorporated herein by reference.

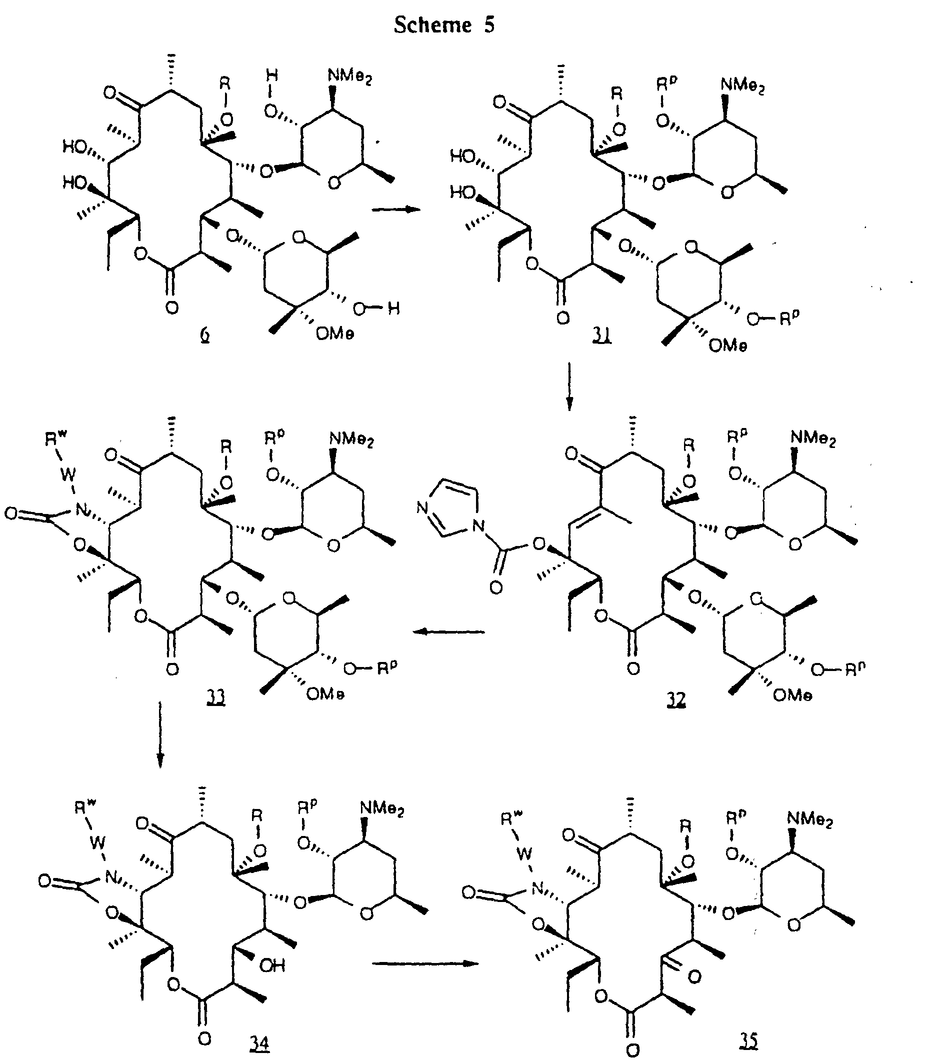

Compound 32 can then be used to form a wider series of intermediate compound to formula (II). For example, treatment of compound 32 with aqueous ammonia results in formation of the cyclic carbamate 33 wherein W is absent and R^{w} is H. Likewise, reaction of compound 6B with a substituted amine of the formula H₂N-R^{w} results in formation of the cyclic carbamate 33.

Treatment of compound 32 with a substituted amine compound of the formula H₂N-W-R^{w}, wherein W is absent and R^{w} is as previously defined except not H gives 33 in which W is -NH- and R^{w} is as previously defined except not H.

Also, treatment of compound 32 with a hydroxylamine compound of the formula H₂N-W-R^{w}, wherein W -O- and R^{w} is as previously defined, results in formation of 33 wherein W is-O- and R^{w} is as previously defined.

Treatment of compound 32 with unsubstituted hydrazine results in formation of the cyclic carbamate 33 wherein W is -NH- and R^{w} is H.

Treatment of compound 32 with a substituted hydrazine compound of the formula H₂N-NH-R^{w}, wherein R^{w} is as previously defined except not H, results in formation of 33 wherein W is -NH- and R^{w} is as previously defined except not H.

Alternate or additional procedures may be used to prepare intermediates of formula (II). For example, treatment of a compound 32 wherein W is absent and R^{w} is H with an alkylating agent having the formula R^{w}-halogen. wherein R^{w} is as previously defined except not H, gives a compound 33 wherein W is absent and R^{w} is not hydrogen.

Similarly, treatment of a compound 32 wherein W is -NH- and R^{w} is H with an alkylating agent having the formula R^{w}-halogen, wherein R^{w} is as previously defined except not H, gives a compound 33 wherein W is -NH- and R^{w} is not hydrogen.

Treatment of compound 32 wherein W is absent and R^{w} is H with an acylating agent selected from the group consisting of the acyl halide R^{w}-C(O)-halogen and the acid anhydride (R^{w}-C(O))₂-O, wherein R^{w} is as previously defined except not H, gives a compound 33 wherein W is -NH-CO- and R^{w} is as previously defined.

Treatment of a compound 32 wherein W is -NH- and R^{w} is H with an aldehyde R^{w}-CHO, wherein R^{w} is as previously defined, gives a compound 33 wherein W is -N=CH- and R^{w} is as previously defined.

Removal of the cladinose moiety from a compound 33 by acid hydrolysis as described previously gives a compound 34. The 3-hydroxy group of 34 is oxidized to the ketone 35 using a modified Swern oxidation procedure as described previously. The conversion of intermediate compound 35 to a compound (II) of the invention is shown below in Scheme 9.

Scheme 6 describes the preparation of intermediate compounds for formula (III). Compound 32 is treated with ethylenediamine 36 in a suitable solvent such as aqueous acetonitrile, DMF or aqueous DMF, to give the bicyclic carbamate intermediate (not shown) which is then cyclized by treatment with dilute acid, such as acetic acid or HCl, in a suitable organic solvent such as ethanol or propanol, to give compound 37.

The cladinose moiety is then removed from compound 37, to give compound 38. The 3-hydroxy group of 38 is oxidized to the ketone 39 using a modified Swern oxidation procedure as described previously, The conversion of intermediate compound 39 to a compound (III) of the invention is shown below in Scheme 9.

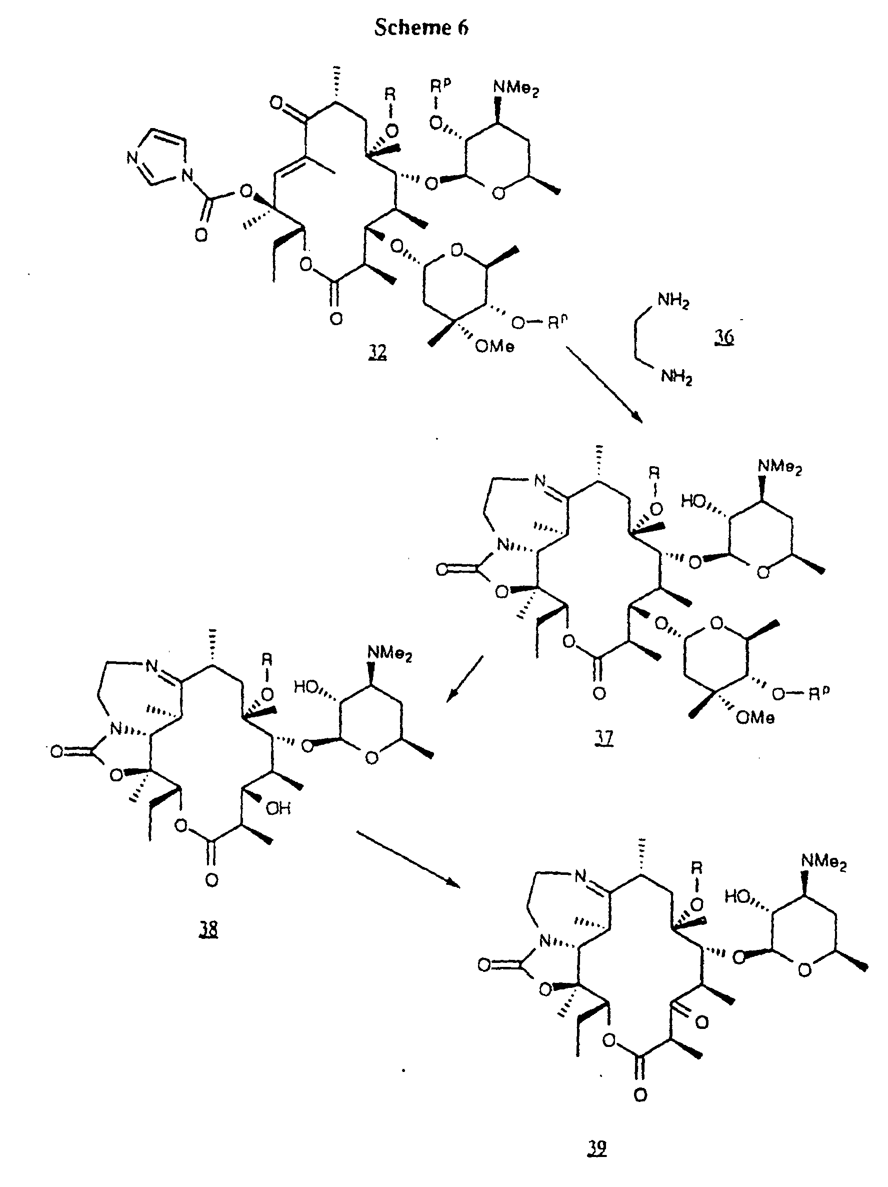

Scheme 7 illustrates the preparation of the cyclic carbonate compounds of formula (IV). In particular, the 2'-protected compound 30, prepared as shown in Scheme 4, is converted to the cyclic carbonate 40 by controlled reaction at low temperatures (about -30 °C) for a short period (about 30 minutes) with carbonyldiimidazole and sodium hexamethyldisilazide. Alternately, compound 40 is prepared from 30 by careful reaction with sodium hydride or lithium hydride and phosgene, diphosgene or triphosgene under anhydrous conditions with careful control of the amount of base present in order to prevent base catalyzed decarboxylation. The conversion of intermediate compound 40 to a compound (IV) of the invention is shown below in Scheme 9.

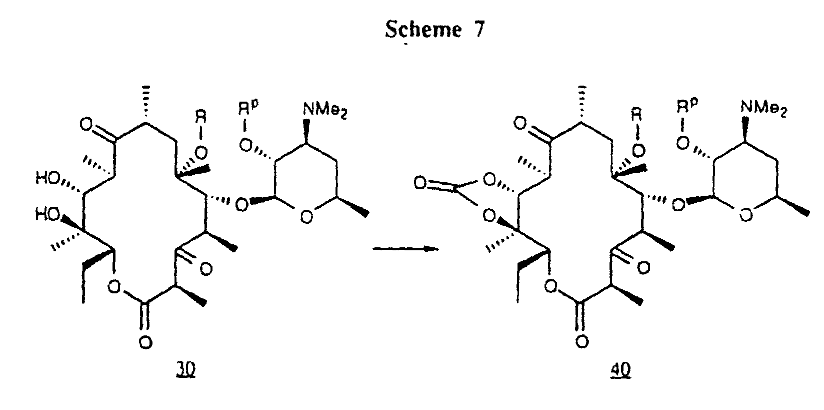

Scheme 8 illustrates the preparation of the cyclic methylene compounds of formula (V). Compound 31 may be treated with formaldehyde in the presence of an acid, or with chloroiodomethane in the presence of base (according to the procedure of Hunt *et al., J. Antibiotics.* **(1988).** 41: 1644) to give the protected 11,12-methylenedioxy compound 41 which is an intermediate to compounds of formula (V). Compound 41 is hydrolyzed to give compound 42. The 3-hydroxy group of 42 is oxidized to the ketone 43 using a modified Swern oxidation procedure as described previously. The conversion of intermediate compound 43 to a compound (IV) of the invention is shown below in Scheme 9.

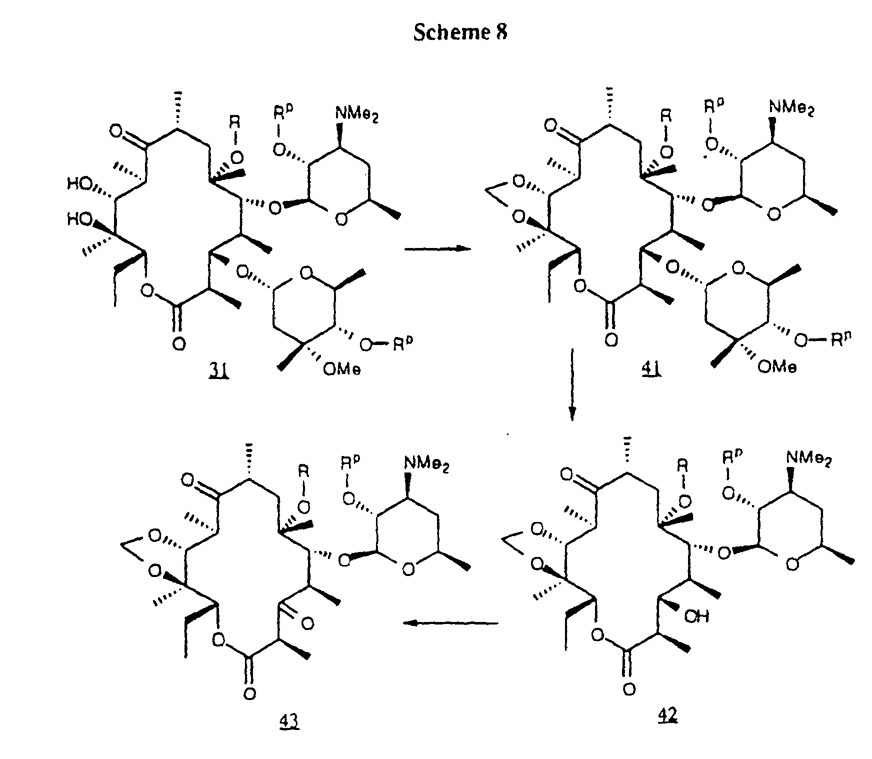

Scheme 9 describes procedures whereby compounds 30, 35, 39, 40, or 43 can be converted to the desired compound of formulas (I)-(V) of the invention. Compounds 30, 35, 39, 40, or 43 are treated with N-iodosuccinimide to give compound 44 wherein one of R¹ and R² is H and the other is methyl. For convenience R² is shown as the methyl group.

Compound 44 can be reacted in the presence of base with a suitable R¹- precursor compound such as R¹-X, wherein R¹ is as defined previously and X is a suitable leaving group, such as a halide or a sulfonate, such as methyl sulfonate, tosylate or trifluoromethylsulfonate, for example, to give compound 45. Alternately, compound 44 can be reductively alkylated with an aldehyde of formula R*-CHO. which when reduced becomes R^{*}-CH₂- which is the R¹ moiety described previously, in the presence of a reducing agent such as NaBH₃CN or H₂ and Pd/C. Typically, suitable R¹-precursor compounds are C₁-C₆-alkyl halides or sulfonates optionally substituted with a group such as halogen, C₃-C₆-cycloalkyl, aryl, substituted-aryl, heteroaryl, and substituted-heteroaryl.

Other suitable precursor compounds are C₂-C₆-alkyl halides or sulfonates optionally substituted with a substituent group such as C₁-C₆-alkoxy, an amine group.-NR'R", wherein R' and R" are independently selected from hydrogen, C₁-C₃-alkyl, C₁-C₃-alkyl substituted with aryl, substituted aryl, heteroaryl, and substituted heteroaryl, -NH-C(O)-C₁-C₆-alkyl, -NH-C(O)-O-C₁-C₆-alkyl, -O-C(O)-O-C₁-C₆-alkyl, -O-C(O)-C₁-C₆-alkyl, -CHO, -C(O)-C₁-C₆-alkyl, -C(O)-NR'R", wherein R' and R" are as previously defined, CH(=N-O-C₁-C₆-alkyl), C(=N-O-C₁-C₆-alkyl)-C₁-C₆-alkyl, C(=N-NH-C₁-C₆-alkyl)-H, and C(=N-NH-C₁-C₆-alkyl)-C₁-C₆-alkyl.

Other additional precursor compounds are C₃-C₆-alkenyl halides optionally substituted with a substituent group such as halogen, C₃-C₆-cycloalkyl, aryl, substituted aryl, heteroaryl, and substituted heteroaryl, C₁-C₆-alkoxy, an amine group -NR'R", wherein R' and R" are independently selected from hydrogen, C₁-C₃-alkyl, C₁-C₃-alkyl substituted with aryl, substituted aryl, heteroaryl, and substituted heteroaryl, -NH-C(O)-C₁-C₆-alkyl, -NH-C(O)-O-C₁-C₆-alkyl, -O-C(O)-O-C₁-C₆-alkyl, -O-C(O)-C₁-C₆-alkyl, -C(O)-H C(O)-C₁-C₆-alkyl, C(O)-NR'R", wherein R' and R" are as previously defined, -CH(=N-O-C₁-C₆-alkyl), -C(=N-O-C₁-C₆-alkyl)-C₁-C₆-alkyl, -CH(=N-NH-C₁-C₆-alkyl), and C(=N-NH-C₁-C₆-alkyl)-C₁-C₆-alkyl. It will be obvious to those skilled in the art that certain of the substituents may not be directly substituted upon an unsaturated carbon atom.

Other additional precursor compounds are C₃-C₆-alkynyl halides optionally substituted with a substituent group such as halogen, C₃-C₆-cycloalkyl, aryl, substituted-aryl, heteroaryl, and substituted-heteroaryl.

Further additional precursor compounds are C₃-C₆-cycloalkylhalides optionally substituted with a substituent group such as halogen, C₃-C₆-cycloalkyl, aryl, substituted-aryl, heteroaryl, and substituted-heteroaryl.

Also, however, the compound 44 may be treated with a formylating agent or an acylating agent of the formula X-C(O)-R', wherein X is halogen and R' is as defined previously, or O-(C(O)-R')₂ to prepare the appropriate derivative wherein R¹ is formyl or C(O)-R', respectively, to give compound 45. Alternately, compound 44 can be reacted with carbonyldiimidazole to give an intermediate compound 45 wherein R¹ is imidazolylcarbonyl, and this intermediate is reacted with an amine having the formula HNR'R", to give the compound 45 wherein R¹ is C(O)-NR'R". Further, compound 44 can be reacted with an alcohol of the formula HOR' to give a compound wherein R¹ is C(O)-OR', wherein R' is as previously defined, to give a compound 45 wherein R¹ is C(O)-O-R'.

Compound 44 can also be reacted with a substituted or unsubstituted aryl alcohol in the presence of a homologating agent such as formaldehyde or paraformaldehyde to give a compound wherein R¹ is methyl substituted with substituted aryl.

Compound 44 can be treated again with N-iodosuccinimide or with iodine in the presence of light to give compound 46 wherein both R¹ and R² are H. Compound 46 may then be treated in the presence of base with one of the R¹-precursor reagents described previously to give compound 47.

Compound 47 may then be treated with a R²-precursor reagent similar to the R¹-precursor reagent described previously and under similar conditions to give the appropriately disubstituted compound 48.

In the instance wherein R¹ and R² taken together may be -(CH₂)ₚ-, wherein p is 3-7, which taken together with the nitrogen atom to which they are attached thus forms a heterocyclic ring containing one nitrogen atom and from 3 to 7 carbon atoms, the precursor can be a suitable alkyl dihalide, such as 1,3-dibromopropane, 1,4-dibromobutane, 1,5-dibromopentane, 1,6-dibromohexane, or 1,7-dibromoheptane, for example.

When R^{p} of formula (I)-(V) is a hydroxy protecting group such as acetate or benzoate, the compound may be deprotected by treatment with methanol or ethanol to give a compound of formula (I) wherein R^{p} is hydrogen. When R^{p} is a trialkylsilyl group, the compound may be deprotected by treatment with fluoride in THF or acetonitrile to give compound of formula (I)-(V) wherein R^{p} is hydrogen.

The foregoing may be better understood by reference to the following examples which are presented for illustration and not to limit the scope of the inventive concept.

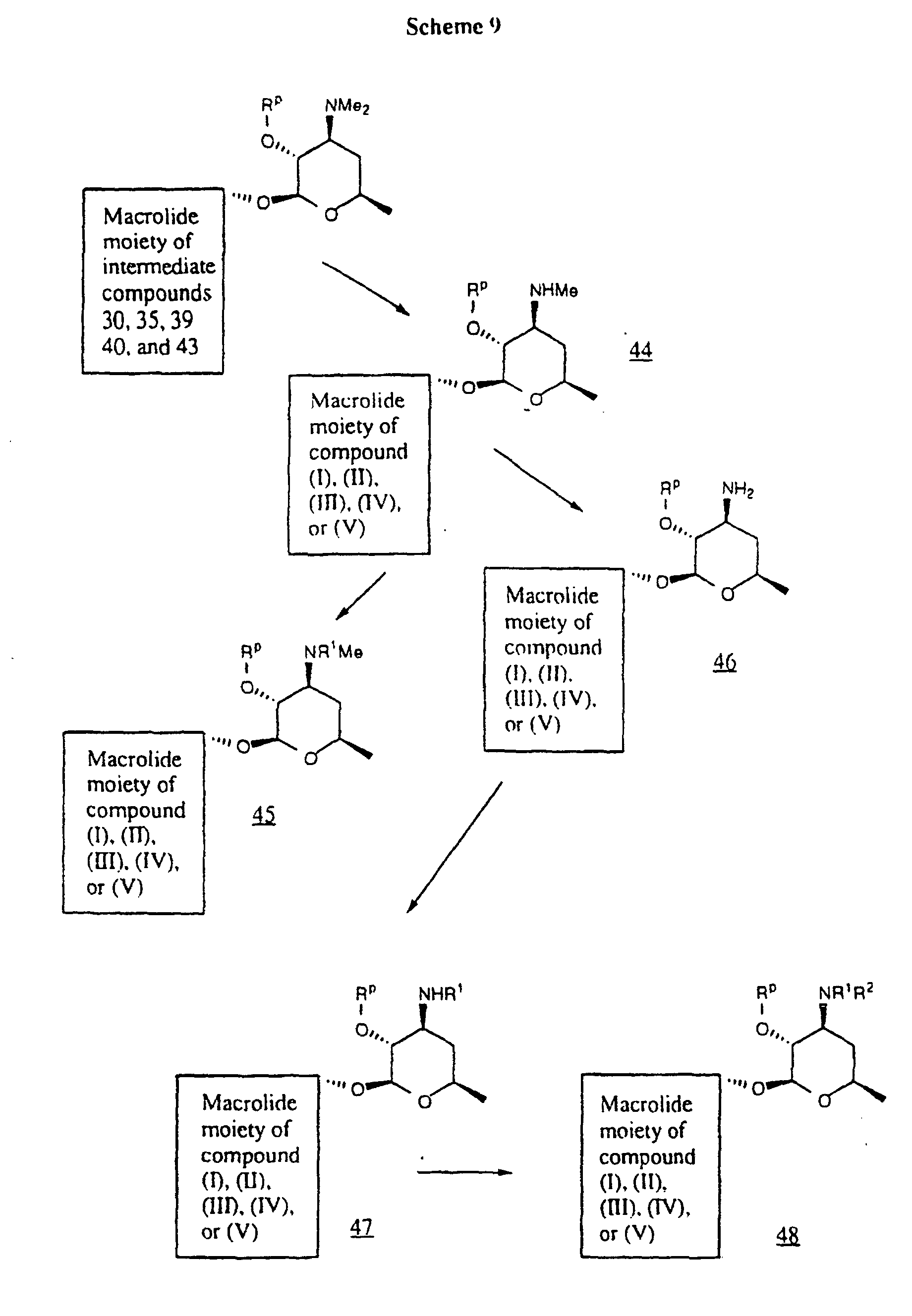

### Example I

### Compound of Formula (I), R is -CH₂CH=CH-(3-quinolyl), R^{p} is H, R¹ is methyl R² is hydrogen

### Step 1a: Compound 4 from Scheme 1, V is N-O-(1-isopropoxycyclohexyl), R is allyl, R^{p} is trimethylsilyl

To a 0 °C solution of 2',4"-*bis*-*O*-trimethylsilylerythromycin A 9-[O-(1-isopropoxycyclohexyl)oxime (1.032 g, 1.00 mmol), prepared according to the method of U.S. Pat. No. 4.990.602 in 5 mL of DMSO and 5 mL of THF was added freshly distilled allyl bromide (0.73 mL, 2.00 mmol). After approximately 5 minutes, a solution of potassium *tert*-butoxide (1M 2.0 mL, 2.0 mL) in 5 mL of DMSO and 5 mL of THF was added dropwise over 4 hours. The reaction mixture was taken up in ethyl acetate and washed with water and brine. The organic phase was concentrated in vacuo to give the desired compound (1.062 g) as a white foam.

### Step 1b: Compound 5 from Scheme 1, V is NOH, R is allyl

To a solution of the compound resulting from step 1a (1.7g) in 17 mL of acetonitrile and 8.5 mL of water was added 9 mL of acetic acid at ambient temperature. After several hours at ambient temperature, the reaction mixture was diluted with 200 mL of toluene and concentrated in vacuo. The residue obtained was found to contain unreacted starting material, so additional acetonitrile (15 mL), water (70 mL) and HOAc (2 mL) was added. After 2 hours, an additional 1 mL aliquot of HOAc was added. After approximately three more hours, the reaction mixture was placed in the freezer overnight. The reaction mixture was allowed to warm to ambient temperature, diluted with 200 mL of toluene and concentrated in vacuo. The residue was chased twice with toluene and dried to constant weight (1.524 g).

### Step 1c: Compound 6 from Scheme 1, R is allyl

The compound resulting from step 1b (1.225 g) in 16 mL of 1:1 ethanol-water was treated with NaHSO₃ (700 mg) and formic acid (141 µL) and warmed at 86 °C for 2.5 hours. The reaction mixture was allowed to cool to ambient temperature, diluted with 5-6 mL of water, basified with 1N NaOH to pH 9-10 and extracted with ethyl acetate. The combined organic extracts were washed with brine (2x), dried over MgSO₄, filtered and concentrated in vacuo. The crude material was purified by column chromatography eluting with 1% MeOH in methylene chloride containing 1% ammonium hydroxide to give 686 mg (57%) of the title compound. ¹³C NMR (CDCl₃) δ 219.3 (C-9), 174.8 (C-1), 135.5 (C-17), 116.3 (C-(18), 101.9 (C-1'), 95.9 (C-1"), 79.7 (C-5), 78.8 (C-6), 78.5 (C-3), 74.1 (C-12), 72.4 (C-3"), 70.6 (C-11), 68.1 (C-5'), 65.5 (C-16), 65.1 (C2'), 49.0 (C-3" O-CH₃), 45.0 (C-2), 44.1 (C-8), 39.7 (NMe₂), 37.9 (C-4), 37.1 (C-10), 34.6 (C-2"), 28.4 (C-4'), 21.0, 20.6 (C-3" CH3, C-6' CH₃), 20.8 (C-14), 18.3 (C-6"), 18.1 (C-8 CH₃), 15.7, 15.6 (C-2 CH₃, C-6 CH₃), 11.9 (C-10 CH₃), 10.1 (C-15), 8.9 (C-4 CH₃), MS (FAB)+ m/e 774 (M+H)⁺, 812 (M+K)⁺.

### Step 1d: Compound 28 from Scheme 4, R is allyl

To a suspension of the compound prepared in step 1c (7.73 g. 10.0 mmol) in ethanol (25 mL) and water (75 mL) was added aqueous 1 M HCl (18 mL) over 10 minutes. The reaction mixture was stirred for 9 hours at ambient temperature and then was left standing in the refrigerator overnight. Aqueous 2 M NaOH (9 mL. 18 mmol) which resulted in the formation of a white precipitate. The mixture was diluted with water and filtered. The solid was washed with water and dried under vacuum to give the des-cladinosyl compound 7 (3.11 g).

### Step 1e: Compound 29 from Scheme 4, R is allyl, R^{p} is benzoyl

To a solution of the product of step 1d (2.49 g. 4.05 mmol) in dichloromethane (20 mL) was added benzoic anhydride (98%, 1.46 g, 6.48 mmol) and triethylamine (0.90 mL, 6.48 mmol) and the white suspension was stirred for 26 hours at ambient temperature. Aqueous 5% sodium carbonate was added and the mixture was stirred for 20 minutes. The mixture was extracted with dichloromethane. The organic phase was washed with aqueous 5% sodium bicarbonate and brine, dried over sodium sulfate and concentrated in vacuo to give a white foam. Chromatography on silica gel (30% acetone-hexanes) gave the title compound (2.46 g) as a white solid.

### Step 1f: Compound 30 from Scheme 4, R is allyl, R^{p} is benzoyl

To a -10 °C solution under N₂ of N-chlorosuccinimide (0.68 g, 5.07 mmol) in dichloromethane (20 mL) was added dimethylsulfide (0.43 mL, 5.92 mmol) over 5 minutes. The resulting white slurry was stirred for 20 minutes at - 10 °C and then a solution of the compound resulting from step 1e (2.43 g, 3.38 mmol) in dichloromethane (20 mL) was added and the reaction mixture was stirred for 30 minutes at -10 to -5 °C. Triethylamine (0.47 mL, 3.38 mmol) was added dropwise over 5 minutes and the reaction mixture was stirred for 30 minutes at 0 °C. The reaction mixture was extracted with dichloromethane. The organic phase was washed twice with aqueous 5% sodium bicarbonate and once with brine, dried over sodium sulfate, and concentrated in vacuo to give a white foam. Chromatography on silica gel (30% acetone-hexanes) gave the title compound (2.27 g) as a white foam.

### Step 1g: Compound of Formula (1), R is allyl, R^{p} is benzoyl, R¹ is methyl, R² is hydrogen

To a sample of the compound from step 1f (215 mg, 0,30 mmol) in acetonitrile (10mL) at 0 °C under nitrogen was added N-iodosuccinimide (101mg, 0.45 mmol), and the mixture was warmed to room temperature. After 5 hours dichloromethane (50 mL) was added, and the mixture was washed with 1: 15% NaHSO3/Na2CO₃ (pH 9) and brine, dried (Na2SO4) and concentrated. The residue was chromatographed on silica gel, eluting with 3:7 acetone/hexane to give the crude product. This material was dissolved in THF (5 mL) and stirred with 5% Na2CO3 (5 mL) for 2 hours. The mixture was diluted with ethyl acetate (30 mL), and the resulting solution was washed with 5% Na2CO3 and brine, dried (Na2SO4) and concentrated. The residue was chromatographed on silica gel, eluting with 3:7 acetone/hexane to give the title compound (75.5 mg).

### Step 1h: Compound of Formula (I), R is allyl, R^{p} is H, R¹ is methyl, R² is hydrogen

The compound from step 1g was heated at reflux in methanol under nitrogen for 6 hours, then the solvent was removed. The residue was chromatographed on silica gel, eluting with 95:5:0.5 dichlomethane/methanol/NH4OH to give the title compound (48.7 mg), Anal. Calcd, for C₃₁H₅₃NO₁₀·0,5 H₂O: C. 61.16; H, 8.94; N, 2.30; Found: C. 61,33; H, 8.89; N, 2.24. MS m/e 600 (M+H)⁺.

### Step 1i : Compound of Formula (I), R is -CH₂CH=CH-(3-quinolyl), R^{p} is H,R¹ is methyl, R² is hydrogen

A mixture of the compound from Step 1h, palladium(II)acetate and tri-o-tolylphosphine in acetonitrile (400 mL) is flushed with nitrogen. To this solution is added 3-bromoquinoline and triethylamine. The reaction mixture is heated at 50°C for I hour and stirred at 90°C for 4 days. The reaction mixture is taken up in ethyl acetate and washed with aqueous 5% sodium bicarbonate and brine, dried over sodium sulfate, filtered, and concentrated *in vacuo.* Chromatography on silica gel gives the title compound.

### Example 2

### Compound of formula (II), R is -CH₂CH=CH-(3-quinolyl), R^{p} is acetyl, R¹ is H, R² is CH₃, W is absent, R^{w} is H

### Step 2a. Compound 31 from Scheme 5; R is-CH₂CH=CH₂, R^{p} is acetyl

To a sample of the compound from Example 1 step c (405.2 g, 528 mmol) in dichloromethane (20 mL) was added dimethylaminopyridine(0.488 g, 4 mmol) and acetic anhydride (3.39 mL, 36 mmol), and the mixture was stirred at room temperature for 3 hours. The mixture was diluted with methylene chloride, then washed with 5% aqueous sodium bicarbonate and brine and dried over Na₂SO₄. The residue was dried and recrystallized from acetonitrile to give the title compound (491 g). MS m/e 857 (M+H)⁺.

### Step 2b. Compound 32 from Scheme 5; R is -CH₂CH=CH₂, R^{p} is acetyl

To a sample of the compound from step 2a (85.8 g. 100 mmol) in dry THF (500 mL) cooled to -40 °C and flushed with nitrogen was added sodium bis(trimethylsilyl)amide (125 mL, 125 mmol) over 20 minutes, and the mixture was stirred at -40 °C for 40 minutes. To this mixture was added a solution of carbonyldiimidazole (3.65 g, 22.56 mmol) in 5:3 THF/DMF (800 mL) under nitrogen at -40 °C over 30 minutes, and the mixture was stirred at -20 °C for 30 minutes. The mixture was stirred at room temperature for 27 hours, then diluted with ethyl acetate. The mixture was washed with 5% sodium bicarbonate and brine, dried over Na₂SO₄, and concentrated to give the title compound (124g), which was taken directly to the next step.

### Step 2c. Compound 33 from Scheme 5; R is -CH₂CH=CH₂, R^{p} is acetyl, W is absent, R^{w} is H

The compound from step 2h (124 g) was dissolved in 9:1 acetonitrile/THF (1100 mL), ammonium hydroxide (28%, 200 mL) was added, and the mixture was stirred at room temperature under nitrogen for 8 days. The solvent was removed, and the residue was dissolved in ethyl acetate. This solution was washed with 5% sodium bicarbonate and brine, dried over Na₂SO₄, and concentrated to give the title compound, MS (FAB)+ m/e 882 (M+H)⁺.

### Step 2d. Compound 34 from Scheme 5; R is -CH-₂CH=CH₂, R^{P} is acetyl, W is absent, R^{w} is H

To a sample of the compound from step 2c (69.0 g, 78.2 mmol) suspended in ethanol (200 mL) and diluted with water (400 mL) was added HCl (0.972 N. 400 mL) dropwise over 20 minutes. The mixture was stirred for 4 hours, and additional HCl was added (4 N. 100 mL) over 20 minutes. The mixture was stirred for 18 hours, cooled to 0 °C, then NaOH (4 N, 200 mL) was added over 30 minutes to approximately pH 9. The title compound was isolated by filtration (35.56 g)

### Step 2e. Compound 35 from Scheme 5; R is -CH₂CH=CH₂ R^{P} is acetyl, W is absent, R^{w} is H

To a -10 °C solution under nitrogen of N-chlorosuccinimide (2.37 g, 17.8 mmol) in dichloromethane (80 mL) was added dimethylsulfide (1.52 mL, 20.8 mmol) over 5 minutes. The resulting white slurry was stirred for 10 minutes at 10 °C, a solution of the compound from step 2d (8.10 g, 11.9 mmol) in dichloromethane (60 mL) was added and the reaction mixture was stirred for 30 minutes at -10 to -5 °C. Triethylamine (1.99 mL. 14.3 mmol) was added dropwise over 10 minutes and the reaction mixture was stirred for 1 hour at 0°C. The reaction mixture was extracted with dichloromethane. The organic phase was washed with aqueous 5% sodium bicarbonate and brine, dried over sodium sulfate, and concentrated in vacuo to give a white foam. Chromatography on silica gel (eluting with 50:50:0.5 acetone/hexanes/ammonium hydroxide) gave the title compound (8.27 g) as a white foam. Anal. Calcd. for C₃₅H₅₆N₂O₁₁: C. 61.75; H, 8.29; N. 4.11: Found: C, 62.25: H, 8.50; N, 4.28.

### Step 2f, Compound 35 from Scheme 5; R is -CH₂CH=CH-(3-quinolyl), R^{p} is acetyl, W is absent, R^{w} is H

A mixture of the compound from Step 2e (46.36 g. 68.2 mmol), palladium(II)acetate (3.055 g, 13.6 mmol), and tri-o-tolylphosphine (8.268 g. 27.2 mmol) in acetonitrile (400 mL) was flushed with nitrogen. To this solution was added 3-bromoquinoline(18.45 mL, 136 mmol) and triethylamine (18.92 mL, 13.6 mmol) via syringe . The reaction mixture was heated at 50 °C for 1 hour and stirred at 90 °C for 4 days. The reaction mixture was taken up in ethyl acetate and washed with aqueous 5% sodium bicarbonate and brine, dried over sodium sulfate, filtered, and concentrated in vacuo, Chromatography on silica gel (eluting with 50:50:0.5 acetone/hexanes/aminonium hydroxide) gave the title compound (46.56 g) as a white foam. MS m/e 808 (M+H)⁺.

### Step 2g Compound of formula (II), R is -CH₂CH=CH-(3-quinolyl), R^{p} is acetyl, R¹ is H, R² is CH₃, W is absent, R^{w} is H

To a sample of the compound from step 2f (2.65 g. 3.3 mmol) in dry acetonitrile (110 mL) at 0 °C under nitrogen was added N-iodosuccinimide (0.887 g, 3.94 mmol) in portions, and the mixture was held at 5 °C overnight. Then the mixture was again cooled to 0°C, and additional N-iodosuccinimide (371 mg) was added. The mixture was then allowed to warm to ambient temperature, diluted with methanol and stirred overnight. The solvent was removed under vacuum, and the residue was dissolved in dichloromethane. The solution was washed with 5% Na₂CO₃ and brine, dried (Na₂SO₄) and concentrated. The residue was chromatographed on silica gel, eluting with 5-10 % methanol in dichloromethane containing 0.5 % NH₄POH. The product was rechromatographed with 1:1:0.5 to 3:1;).5 acetone/hexane/NH4OH to give the title compound (260 mg).
MS m/e 794 (M+H)⁺.

### Example 3

### Compound of Formula (II); R is -CH₂CH=CH-(3-quinolyl), R^{p} is H, W is absent, R^{w} is H, R¹ is H, R² is CH₃-

### Step 3a, Compound 35 from Scheme 5; R is -CH₂CH=CH-(3-quinolyl), R^{p} is H, W is absent, R^{w} is H

A sample of the compound from Example 2, Step 2f was stirred in methanol overnight. The solvent was removed, and the product was used without further purification.

### Step 3b Compound of formula (II), R is -CH₂CH=CH-(3-quinolyl), R^{p} is H, R¹ is H, R² is CH₃, W is absent, R^{w} is H

To a sample of the compound from Step 3a (382 mg, 0.500 mmol) in dry acetonitrile (20 mL) at 0 °C under nitrogen was added N-iodosuccinimide (125 mg, 0.600 mmol), and the mixture was allowed to warm to room temperature. After standing overnight, the mixture was diluted with ethyl acetate. The solution was washed with 5% Na₂SO₃, 5% Na₂CO₃ and brine, dried (Na₂SO₄) and concentrated. The residue was chromatographed on silica gel, eluting with 5-10 % methanol in dichloromethane containing 0.5 % dimethylamine to give the title compound (201 mg). High Res. M.S. calcd for C₄₁H₅₇N₃O₁₀: 752.4122; observed: 752,4145.

### Example 4

### Compound of Formula (II); R is -CH₂CH=CH-(3-quinolyl), R^{P} is H, W is absent, R^{w} is H, R¹ is acetyl, R² is CH₃-

To a sample of the compound from Example 3 (193 mg. 0.260 mmol) in dichloromethane at 0 °C was added triethylamine (0.109 mL, 0.780 mmol). The solution was stirred for 5 minutes, then acetic anhydride ((0.024 mL, 0.260 mmol) was added, and the mixture was stirred for 2 hours. Another portion of acetic anhydride was added (0.005 mL), then the mixture was stirred at room temperature overnight and at reflux for 30 minutes. The mixture was diluted with ethyl acetate, and this solution was washed with 5% aqueous Na₂CO₃ and brine, dried (Na₂SO₄) and concentrated. The residue was chromatographed on silica gel, eluting with 5% methanol in dichloromethane containing 0.5 % NH₄OH to give the title compound (91.7 mg). MS m/e 794 (M+H)⁺.

### Example 5

### Compound of Formula (II); R is -CH₂CH=CH-(3-quinolyl), R^{p} is H, W is absent, R^{w} is H, R¹ is CH₂C(O)-O-CH₂CH₃, R² is CH₃.

To a sample of the compound from Example 3 (120 mg, 0.160 mmol) in acetonitrile was added NaHCO₃ (67.2 mg, 0.800 mmol) and ethyl bromoacetate (0.020 mL, 0.180 mmol), and the mixture was stirred under nitrogen at room temperature for 4 days. The mixture was diluted with ethyl acetate, and this solution was washed with 5% aqueous NaHCO₃ and brine, dried (Na₂SO₄) and concentrated. The residue was chromatographed on silica gel, eluting with 5-10% methanol in dichloromethane containing 0.5 % NH₄OH to give the title compound (60 mg). MS m/e 838 (M+H)⁺.

### Example 6

### Compound of Formula (II); R is -CH₂CH=CH-(3-quinolyl), R^{p} is H, W is absent, R^{w} is H, R¹ is CH₂CH=CH₂, R² is CH₃

To a sample of the compound from Example 3 (120 mg, 0.160 mmol) in acetonitrile was added NaHCO₃ (67.2 mg, 0.800 mmol) and allyl bromide (0.016 mL, 0.180 mmol), and the mixture was stirred under nitrogen at room temperature for 4 days. The mixture was diluted with ethyl acetate, and this solution was washed with 5% aqueous NaHCO₃ and brine, dried (Na₂SO₄) and concentrated. The residue was chromatographed on silica gel, eluting with 5-10% methanol in dichloromethane containing 0.5 % NH₄OH to give the title compound (69 mg). MS m/e 792 (M+H)⁺.

### Example 7

### Compound of Formula (II); R is -CH₂CH=CH-(3-quinolyl), R^{p} is H, W is absent, R^{w} is H, R¹ is CH₂CH₂F, R² is CH₃

To a sample of the compound from Example 2 (150 mg, 0.200 mmol) in acetonitrile was added NaHCO₃ (84 mg, 1.00 mmol) and 1-bromo-2-fluoroethane (0.016 mL, 0.220 mmol), and the mixture was stirred under nitrogen at room temperature for 4 hours. Another portion of 1-bromo-2-fluoroethane (0.010 mL, 0.100 mmol) was added, then the mixture was stirred at room temperature overnight and at reflux for 2 hours. Another portion of 1-bromo-2-fluoroethane (0.005 mL, 0.050 mmol) was added, then the mixture was stirred at reflux overnight. The mixture was diluted with ethyl acetate, and this solution was washed with 5% aqueous NaHCO₃ and brine, dried (Na₂SO₄) and concentrated. The residue was chromatographed on silica gel, eluting with 5-10% methanol in dichloromethane containing 0.5 % NH₄OH to give the title compound (73.3 mg). MS m/e 798 (M+H)⁺.

### Example 8

### Compound of Formula (II); R is -CH₂CH=CH-(3-quinolyl R^{p} is H, W is absent R^{w} is H, R¹ is CH₂-phenyl, R² is CH₃

To a sample of the compound from Example 2 (150 mg, 0.200 mmol) in acetonitrile was added NaHCO₃ (84 mg, 1.00 mmol) and benzyl bromide (0.020 mL, 0.220 mmol), and the mixture was stirred under nitrogen at room temperature for 48 hours. The mixture was diluted with ethyl acetate, and this solution was washed with 5% aqueous NaHCO₃ and brine, dried (Na₂SO₄) and concentrated. The residue was chromatographed on silica gel, eluting with 5-10% methanol in dichloromethane containing 0.5 % NH₄OH to give the title compound 118 mg). MS m/e 842 (M+H)⁺.

### Example 9

### Compound of Formula (II); R is -CH₂CH=CH-(3-quinolyl), R^{p} is H, W is absent, R^{w} is H, R¹ is CH₂-CN, R² is CH₃-

To a sample of the compound from Example 3 (150 mg, 0.200 mmol) in acetonitrile was added NaHCO₃ (84 mg, 1.00 mmol) and bromoacetonitrile (0.015 mL, 0.220 mmol), and the mixture was stirred under nitrogen at room temperature for 48 hours. The mixture was diluted with ethyl acetate, and this solution was washed with 5% aqueous NaHCO₃ and brine, dried (Na₂SO₄) and concentrated. The residue was chromatographed on silica gel, eluting with 5-10% methanol in dichloromethane containing 0.5 % NH₄OH to give the title compound (106.7 mg). MS m/e 791 (M+H)⁺.

### Example 10

### Compound of Formula (II); R is -CH₂CH=CH-(3-quinolyl), R^{p} is H, W is absent, R^{w} is H, R¹ is CH₂-C≡CH, R² is CH₃₋

To a sample of the compound from Example 3 (150 mg. 0.200 mmol) in acetonitrile was added NaHCO₃ (84 mg, 1.00 mmol) and propargyl bromide (80% in toluene, 0.026 mL, 0.220 mmol), and the mixture was stirred under nitrogen at room temperature for 48 hours. The mixture was diluted with ethyl acetate, and this solution was washed with 5% aqueous NaHCO₃ and brine, dried (Na₂SO₄) and concentrated. The residue was chromatographed on silica gel, eluting with 5% methanol in dichloromethane containing 0.5 % NH₄OH to give the title compound (90 mg). MS m/e 790 (M+H)⁺.

### Example 11

### Compound of Formula (II); R is -CH₂CH=CH-(3-quinolyl), R^{p} is H, W is absent, R^{w} is H, R¹ is CH₂CH₂CH₃, R² is CH₃.

To a sample of the compound from Example 3 (150 mg, 0.200 mmol) in acetonitrile was added NaHCO₃ (84 mg, 1.00 mmol) and 1-bromopropane (0.020 mL, 0.220 mmol), and the mixture was stirred under nitrogen at room temperature for 48 hours and at 60 °C for 16 hours. The mixture was diluted with ethyl acetate, and this solution was washed with 5% aqueous NaHCO₃ and brine, dried (Na₂SO₄) and concentrated. The residue was chromatographed on silica gel, eluting with 5% methanol in dichloromethane containing 0.5% NH₄OH to give the title compound (80 mg). MS m/e 794 (M+H)⁺.

### Example 12

### Compound of Formula (II); R is -CH₂CH=CH-(3-quinolyl), R^{p} is H, W is absent, R^{w} is H, R¹ is CH₂-cyclopropyl, R² is CH₃.

To a sample of the compound from Example 3 (150 mg, 0.200 mmol) in acetonitrile was added NaHCO₃ (84 mg, 1.00 mmol) and (bromomethyl)cyclopropane (0.021 mL, 0.220 mmol), and the mixture was stirred under nitrogen at room temperature for 48 hours. The mixture was diluted with ethyl acetate, and this solution was washed with 5% aqueous NaHCO₃ and brine, dried (Na₂SO₄) and concentrated. The residue was chromatographed on silica gel, eluting with 5% methanol in dichloromethane containing 0.5 % NH₄OH to give the title compound (90.5 mg), MS m/e 806 (M+H)⁺.

### Example 13

### Compound of Formula (II); R is -CH₂CH=CH-(3-quinolyl), R^{p} is H, W is absent, R^{w} is H, R¹ is cyclopropyl, R² is CH₃

To a sample of the compound from Example 3 (150 mg, 0.200 mmol) in methanol was added acetic acid (0.114 mL, 2.00 mmol) and ((1-ethoxycyclopropyl)oxy)trimethylsilane (0.200 mL, 1.00 mmol), and the mixture was stirred under nitrogen. NaBH₃CN (63 mg, 1.00 mmol) was added under nitrogen, and the mixture was stirred at room temperature for 2 hours and at reflux for 12 hours. The mixture was diluted with ethyl acetate, and this solution was washed with 5% aqueous Na₂CO₃ and brine, dried (Na₂SO₄) and concentrated. The residue was chromatographed on silica gel, eluting with 5% methanol in dichloromethane containing 0.5 % NH₄OH to give the title compound (54.4 mg). MS m/e 792 (M+H)⁺.

### Example 14

### Compound of Formula (II); R is -CH₂CH=CH-(3-quinolyl), R^{p} is H, W is absent, R^{w} is H, R¹ is CH₂-(3-pyridyl), R² is CH₃

To a sample of the compound from Example 3 (150) mg, 0.200 mmol) in methanol was added acetic acid (0.114 mL, 2.00 mmol) and 3-pyridine carboxaldehyde (0.094 mL, 1.00 mmol), and the mixture was stirred at 0 °C under nitrogen. NaBH₃CN (63 mg, 1.00 mmol) was added under nitrogen, and the mixture was allowed to warm to room temperature and stirred for 6 hours. The mixture was diluted with ethyl acetate, and this solution was washed with 5% aqueous Na₂CO₃ and brine, dried (Na₂SO₄) and concentrated. The residue was chromatographed on silica gel, eluting with 5% methanol in dichloromethane containing 0.5 *%* NH₄OH to give the title compound (132 mg), MS m/e 843 (M+H)⁺.

### Example 15

### Compound of Formula (II); R is -CH₂CH=CH-(3-quinolyl), R^{p} is H, W is absent, R^{w} is H, R¹ is CH₂-(cyclo-C₃H₅), R² is CH₃

A solution of a sample of Example 1 (150 mg, 0.20 mmol) in acetonitrile (2 mL) at room temperature under N₂ was treated sequentially with NaHCO₃ (84 mg, 1.1 mmol) and (bromomethyl)cyclopropane (21 µL, 0.22 mmol), stirred at room temperature for 18 hours, treated with an additional equivalent of (bromomethyl)cyclopropane, stirred for 18 hours, treated with N,N-diisopropylethylamine (174 µL, 1.1 mmol) and an additional 2 equivalents of (bromomethyl)cyclopropane, stirred for 4 days, diluted with ethyl acetate (10 mL). washed sequentially with 5% NaHCO₃ and brine, dried (Na₂SO₄), filtered, and concentrated. The residue was purified by column chromatography on silica gel with 1% methanol in methylene chloride containing 1% ammonium hydroxide to provide 90.5 mg of the desired compound as a white solid. MS (ESI(+)) 806 (M+H)⁺.

### Example 16

### Compound of Formula (II); R is -CH₂CH=CH-(3-quinolyl), R^{p} is H, W is absent, R^{w} is H, R¹ is CH₂CH₂CH₃, R² is CH₃

A solution of a sample of Example 1 (150 mg, 0.20 mmol) in acetonitrile (2 mL) at room temperature under N₂ was treated sequentially with NaHCO₃ (84 mg, 1.1 mmol) and 1-bromopropane (20 µL, 0.22 mmol), stirred at room temperature for 18 hours, treated with an additional equivalent of 1-bromopropane, stirred for 18 hours, treated with N,N-diisopropylethylamine (174 µL. 1.1 mmol), warmed to 60 °C for 18 hours, cooled to room temperature, treated with an additional 2 equivalents of 1-bromopropane, diluted with ethyl acetate (10 mL), washed sequentially with 5% NaHCO₃ and brine, dried (Na₂SO₄), filtered. and concentrated. The residue was purified by column chromatography on silica gel with 1 % methanol in methylene chloride containing 1% ammonium hydroxide to provide 80 mg of the desired compound as a white solid. MS (ESI(+)) 794 (M+H)⁺.

### Example 17

### Compound of Formula (II); R is -CH₂CH=CH-(3-quinolyl), R^{p} is H, W is absent, R^{w} is H, R¹ is CH₂CH=CHC₆H₅, R² is CH₃

A solution of a sample of Example 1 (260.64 mg, 0.33 mmol) in acetonitrile (2 mL) at at room temperature under N₂ was treated with K₂CO₃ (230 mg, 1.1 mmol) and cinnamyl bromide (55.5 µL. 0.37 mmol), stirred at room temperature for 48 hours, diluted with ethyl acetate (10 mL), washed sequentially with 5% NaHCO₃ and brine, dried (Na₂SO₄), filtered, and concentrated. The residue was purified by column chromatography on silica gel with 5% methanol in methylene chloride containing 1% ammonium hydroxide to provide 180 mg of the desired compound as a white solid. MS (ESI(+)) 869 (M+H)⁺.

### Example 18

### Compound of Formula (II); R is -CH₂CH=CH-(3-quinolyl), R^{p} is H, W is absent, R^{w} is H, R¹ is CH₂C(=CH₂)C(O)OCH₃, R² is CH₃

A solution of a sample of Example 1 (260.64 mg, 0.33 mmol) in acetonitrile (2 mL) at at room temperature under N₂ was treated with K₂CO₃ (230 mg, 1.1 mmol) and methyl-2-(bromomethyl)acrylate (45.08 µL), stirred at room temperature for 48 hours, diluted with ethyl acetate, washed sequentially with 5% NaHCO₃ and brine, dried (Na₂SO₄), filtered, and concentrated. The residue was purified by column chromatography on silica gel with 5% methanol in methylene chloride containing 1% ammonium hydroxide to provide 233 mg of the desired compound as a white solid. MS (ESI(+)) 850 (M+H)⁺.

### Example 19

### Compound of Formula (II); R is -CH₂CH=CH-(3-quinolyl), R^{p} is H, W is absent, R^{w} is H, R¹ is CH₂C(=CH₂)CH₃,R² is CH₃

A solution of a sample of Example 1 (260.64 mg, 0.33 mmol) in acetonitrile (2 mL) at at room temperature under N₂ was treated with K₂CO₃ (230 mg, 1.1 mmol) and 3-bromo-2-methylpropene (37.81 µL), stirred at room temperature for 48 hours, diluted with ethyl acetate, washed sequentially with 5% NaHCO₃ and brine, dried (Na₂SO₄), filtered, and concentrated. The residue was purified by column chromatography on silica gel with 5% methanol in methylene chloride containing 1 % ammonium hydroxide to provide 176.4 mg of the desired compound as a white solid. MS (ESI(+)) 804 (M+H)⁺.

### Example 20

### Compound of Formula (II); R is -CH₂CH=CH-(3-quinolyl), R^{p} is H, W is absent, R^{w} is H, R¹ is cyclo-C₃H₅, R² is CH₃

A solution of a sample of Example 1 (150mg, 0.200 mmol) in methanol (5 mL) at room temperature under N₂ was treated sequentially with acetic acid (114 µL, 2.00 mmol), [(1-ethyoxycyclopropyl)oxy]trimethylsilane (200 µL, 1.00 mmol), and NaBH₃CN (63 mg, 1.00 mmol), stirred at room temperature for two hours, heated to reflux for 12 hours. diluted with ethyl acetate (30 mL), washed sequentially with 5% Na₂CO₃ and brine, dried (Na₂SO₄), filtered, and concentrated. The residue was purified by column chromatography on silica gel with a gradient of 2% methanol in methylene chloride to 5% methanol in methylene chloride containing 1% ammonium hydroxide to provide 54.4 mg of the desired compound as a white solid. MS (ESI(+)) 792 (M+H)⁺. HRMS (ESI(+)) m/z calcd for C₄₄H₆₁N₃O₁₀: 814.4249 (M+Na)⁺. Found 814.4243.

### Example 21

### Compound of Formula (II); R is -CH₂CH=CH-(3-quinolyl), R^{p} is H, W is absent, R^{w} is H, R¹ is CH₂-(3-pyridyl), R² is CH₃

A solution of a sample of Example 1 in methanol (5 mL) at 0°C under N₂ was treated sequentially with acetic acid (114 µL, 2.00 mmol), 3-pyridinecarboxaldehyde (94 µl, 1.00 mmol), and sodium cyanoborohydride (63 mg 1.00 mmol), warmed to room temperature with stirring over 18 hours, diluted with ethyl acetate (30 mL), washed sequentially with 5% Na₂CO₃, 2% tris(hydroxymethyl)aminomethane, and brine, dried (Na₂SO₄), filtered, and concentrated. The residue was purified by column chromatography on silica gel with 5% methanol in methylene chloride containing 1% ammonium hydroxide to provide 132 mg (78%) of the desired compound as an off-white foam.
MS (APCI) 843 (M+H)+.
HRMS (ESI(+)) m/z calcd for C₄₇H₆₃N₄O₁₀: 843,4544 (M+H)⁺. Found: 843,4562. Anal, calcd for: C, 66.96; H, 7.41, N, 6.65. Found C. 66.97; H, 7.45; N, 6.57.

### Example 22

### Compound of Formula (II); R is -CH₂CH=CH-(3-quinolyl), R^{p} is H, W is absent, R^{w} is H, R¹ is CH₂-(3-hydroxyphenyl), R² is CH₃

A solution of a sample of Example 1 (150 mg, 0.200 mmol) in methanol (5 mL) at 0°C under N₂ was treated with 3-hydroxybenzaldehyde (122 mg, 1.0 mmol), stirred for 5-10 minutes, treated with acetic acid (114 µL, 2.00 mmol), stirred at 0°C for 10-15 minutes, treated with sodium cyanoborohydride (63 mg, 1.00 mmol), warmed to room temperature over 18 hours. stirred for 48 hours, treated with ethyl acetate (20 mL), washed sequentially with 5% NaHCO₃, 2% tris(hydroxymethyl)aminomethane, and brine. If any aqueous extract was too basic (pH 10-12) and contained product, it was treated with NH₄Cl and back-extracted with ethyl acetate. The combined organic extracts were washed with brine, dried (Na₂SO₄), filtered, and concentrated. The residue was purified by column chromatography on silica gel with a gradient of 5% methanol in methylene chloride containing 1% ammonium hydroxide to provide to provide 97.1 mg of the desired compound as a yellow solid. MS (ESI(+)) m/z 858 (M+H)⁺.

### Example 23

### Compound of Formula (II); R is -CH₂CH=CH-(3-quinolyl), R^{p} is H, W is absent, R^{w} is H, R¹ is CH₂-(2-hydroxy-3-tert-butyl-5-methylphenyl), R² is CH₃

A solution of a sample of Example 1 (28 mg, 0.037 mmol) and 3-*tert*-butyl-5-methylphenol (1.5-2.0 equivalents) in toluene (1 mL) in a 1 dram vial was treated with paraformaldehyde (2 equivalents), warmed to 90 °C for 18 hours, and concentrated. If necessary, the vial was uncapped and warmed to permit the toluene to evaporate and drive the reaction to completion. The residue was purified by column chromatography on silica gel with acetone to provide the desired product. MS (ESI(+)) m/z 928 (M+H)⁺.

### Example 24

### Compound of Formula (II); R is -CH₂CH=CH-(3-quinolyl), R^{p} is H, W is absent, R^{w} is H, R¹ is CH₂-(2-hydroxy-3,4-dimethylphenyl), R² is CH₃

A sample of Example 1, paraformaldehyde, and 3,4-dimethylphenol were processed as described in Example 9 to provide the desired compound.
MS (ESI(+)) m/z 886 (M+H)⁺.

### Example 25

### Compound of Formula (II); R is -CH₂CH=CH-(3 -quinolyl), R^{p} is H, W is absent R^{w} is H, R¹ is CH₂-(2-hydroxy-3-methoxy-5-(2-propenyl)phenyl), R² is-CH₃

A sample of Example 1 (28 mg, 0.037 mmol), paraformaldehyde, and 3-allyl-5-methoxyphenol were processed as described in Example 9 to provide the desired compound. MS (ESI(+)) m/z 928 (M+H)⁺.

### Example 26

### Compound of Formula (II); R is -CH₂CH=CH-(3-quinolyl), R^{p} is H, W is absent, R^{w} is H, R¹ is CH₂-(2-hydroxy-3-methoxy-5-methylphenyl), R² is CH₃

A sample of Example 1 (28 mg. 0.037 mmol), paraformaldehyde, and 3-methoxy-5-methylphenol were processed as described in Example 9 to provide the desired compound.
MS (ESI(+)) m/z 902 (M+H)⁺.

### Example 27

### Compound of Formula (II); R is -CH₂CH=CH-(3-quinolyl), R^{p} is H, W is absent, R^{w} is H, R¹ is CH₂-(2-hydroxy-5-cyclopentylphenyl), R² is CH₃

A sample of Example 1 (28 mg, 0.037 mmol), paraformaldehyde, and 3-cyclopentylphenol were processed as described in Example 9 to provide the desired compound.
MS (ESI(+)) m/z 926 (M+H)⁺.

### Example 28

### Compound of Formula (II); R is -CH₂CH=CH-(3-quinolyl), R^{p} is H, W is absent, R^{w} is H, R¹ is CH₂-(2-hydroxy-5-carboxamidophenyl), R² is CH₃

A sample of Example 1 (28 mg, 0.037 mmol), paraformaldehyde, and 3-hydroxybenzamide were processed as described in Example 9 to provide the desired compound.
MS (ESI(+)) m/z 901 (M+H)⁺.

### Example 29

### Compound of Formula (II); R is -CH₂CH=CH-(3-quinolyl), R^{p} is H, W is absent, R^{w} is H, R¹ is is CH₂-(2-hydroxy-3-methoxy-5-(2-methoxycarbonylethyl)phenyl), R² is CH₃

A sample of Example 1 (28 mg, 0.037 mmol), paraformaldehyde, and 3-(3-hydroxyphenyl)-propionic acid methyl ester were processed as described in Example 9 to provide the desired compound.
MS (ESI(+)) m/z 944 (M+H)⁺.

### Example 30

### Compound of Formula (II); R is -CH₂CH=CH-(3-quinolyl), R^{p} is H, W is absent, R^{w} is H, R¹ is CH₂-(2-hydroxy-3-methyl-5-fluorophenyl), R² is CH₃

A sample of Example 1 (28 mg, 0.037 mmol), paraformaldehyde, and 3-fluoro-5-methylphenol were processed as described in Example 9 to provide the desired compound.
MS (ESI(+)) m/z 890 (M+H)⁺.

### Example 31

### Compound of Formula (II); R is -CH₂CH=CH-(3-quinolyl), R^{p} is H, W is absent, R^{w} is H R¹ is CH₂-(2-hydroxy-3-methoxy-5-acetylphenyl), R² is CH₃

A sample of Example 1 (28 mg, 0.037 mmol), paraformaldehyde, and 1-(3-hydroxy-5-methoxy-phenyl)ethanone were processed as described in Example 9 to provide the desired compound.
MS (ESI(+)) m/z 930 (M+H)⁺.

### Example 32

### Compound of Formula (II); R is -CH₂CH=CH-(3-quinolyl), R^{p} is H, W is absent, R^{w} is H, R¹ is CH₂-(2-hydroxy-3-bromophenyl),R² is CH₃

A sample of Example 1 (28 mg, 0.037 mmol), paraformaldehyde, and 3-bromophenol were processed as described in Example 9 to provide the desired compound.
MS (ESI(+)) m/z 936 (M+H)⁺.

### Example 33

### Compound of Formula (II); R is -CH₂CH=CH-(3-quinolyl), R^{p} is H, W is absent, R^{w} is H, R¹ is CH₂-(2-hydroxy-3-methoxy-5-alkoxycarbonylphenyl), R² is CH₃

A sample of Example 1 (28 mg, 0.037 mmol), paraformaldehyde, and 3-hydroxy-5-methoxybenzoic acid methyl ester were processed as described in Example 9 to provide the desired compound.
MS (ESI(+)) m/z 946 (M+H)⁺.

### Example 34

### Compound of Formula (II): R is -CH₂CH=CH-(3-quinolyl), R^{p} is H, W is absent, R^{w} is H, R¹ is CH₂-(2-hydroxy-3-ethylphenyl), R² is CH₃

A sample of Example 1 (28 mg. 0.037 mmol), paraformaldehyde, and 3-ethylphenol were processed as described in Example 9 to provide the desired compound.
MS (ESI(+)) m/z 886 (M+H)⁺.

### Example 35

### Compound of Formula (II); R is -CH₂CH=CH-(3-quinolyl), R^{p} is H, W is absent, R^{w} is H, R¹ is CH₂-(2-hydroxy-5-isobutylphenyl), R² is CH₃

A sample of Example 1 (28 mg, 0.037 mmol), paraformaldehyde, and 3-*sec*-butylphenol were processed as described in Example 9 to provide the desired compound.
MS (ESI(+)) m/z 914 (M+H)⁺.

### Example 36

### Compound of Formula (II); R is -CH₂CH=CH-(3-quinolyl), R^{p} is H, W is absent, R^{w} is H R¹ is CH₂-(2-hydroxy-3-methyl-5-diethylamino-6-methylphenyl), R² is CH₃

A sample of Example 1 (28 mg, 0.037 mmol), paraformaldehyde, and 3-diethylaminomethyl-2,5-dimethylphenol were processed as described in Example 9 to provide the desired compound.
MS (ESI(+)) m/z 971 (M+H)⁺.

### Example 37

### Compound of Formula (II); R is -CH₂CH=CH-(3-quinolyl), R^{p} is H, W is absent, R^{w} is H, R¹ is CH₂-(2-hydroxy-4-methyl-5-bromo-6-methylphenyl), R² is CH₃

A sample of Example 1 (28 mg, 0.037 mmol), paraformaldehyde, and 3-bromo-2,4-dimethylphenol were processed as described in Example 9 to provide the desired compound.
MS (ESI(+)) m/z 964 (M+H)⁺.

### Example 38

### Compound of Formula (II); R is -CH₂CH=CH-(3-quinolyl), R^{p} is H, W is absent, R^{w} is H, R¹ is CH₂-(2-hydroxy-3-hydroxymethylphenyl), R² is CH₃

A sample of Example 1 (28 mg, 0.037 mmol), paraformaldehyde, and 3-hydroxymethylphenol were processed as described in Example 9 to provide the desired compound.

## Claims

1. A compound selected from the group consisting of 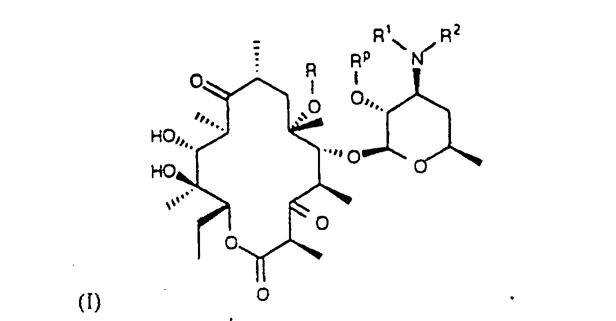 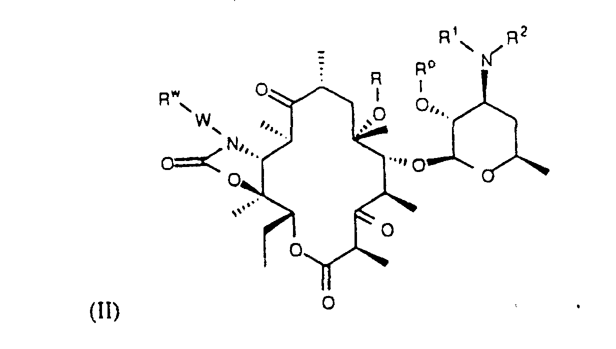 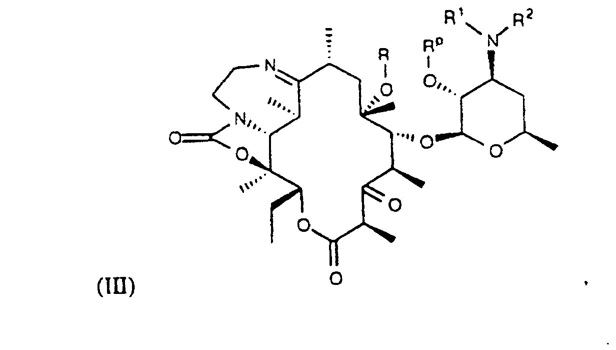 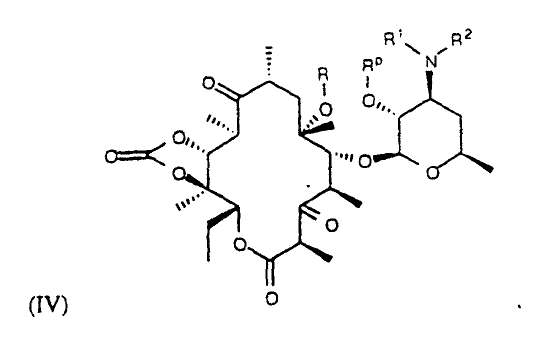 and 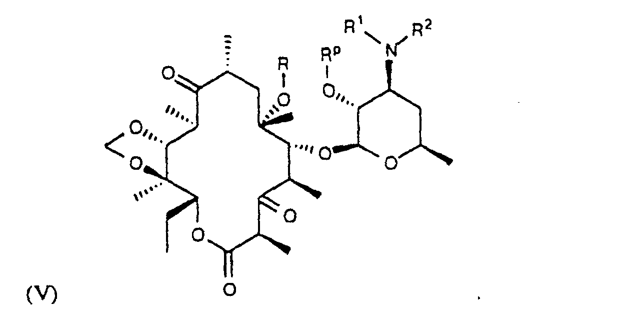 or a pharmaceutically acceptable salt, ester or prodrug thereof,
wherein
R¹ and R², with the proviso that R¹ and R² are not both methyl, are independently selected from the group consisting of
(1) hydrogen,
(2) C₁-C₆-alkyl optionally substituted with a substituent selected from the group consisting of
(a) halogen,
(b) C₃-C₆-cycloalkyl,
(c) aryl,
(d) substituted aryl,
(e) heteroaryl,
(f) substituted heteroaryl,
(g) -CHO,
(h) -C(O)-C₁-C₆-alkyl, and
(i) -C(O)-NR'R", wherein R' and R" are independently selected from the group consisting of hydrogen, C₁-C₃-alkyl, C₁-C₃-alkyl substituted with aryl, substituted aryl, heteroaryl, and substituted heteroaryl,
(3) C₂-C₆-alkyl optionally substituted with a substituent selected from the group consisting of
(a) C₁-C₆-alkoxy,
(b) -NR'R", wherein R' and R" are as previously defined,
(c) -NH-C(O)-C₁-C₆-alkyl,
(d) -NH-C(O)-O-C₁-C₆-alkyl,
(e) -O-C(O)-O-C₁-C₆-alkyl,
(f) -O-C(O)-C₁-C₆-alkyl,
(g) -CH(=N-O-C₁-C₆-alkyl),
(h) -C(=N-O-C₁-C₆-alkyl)-C₁-C₆-alkyl,
(i) -CH(=N-NH-C₁-C₆-alkyl), and
(j) -C(=N-NH-C₁-C₆-alkyl)-C₁-C₆-alkyl,
(4) C₃-C₆-alkenyl optionally substituted with a substituent selected from the group consisting of
(a) halogen,
(b) C₃-C₆-cycloalkyl,
(c) aryl,
(d) substituted aryl,
(e) heteroaryl,
(f) substituted heteroaryl,
(g) -NH-C(O)-C₁-C₆-alkyl,
(h) -NH-C(O)-O-C₁-C₆-alkyl,
(i) -O-C(O)-O-C₁-C₆-alkyl,
(j) -O-C(O)-C₁-C₆-alkyl,
(k) -CHO,
(l) -C(O)-C₁-C₆-alkyl,
(m) -C(O)-NR'R", wherein R' and R" are as previously defined,
(n) -CH(=N-O-C₁-C₆-alkyl),
(o) -C(=N-O-C₁-C₆-alkyl)-C₁-C₆-alkyl,
(p) -CH(=N-NH-C₁-C₆-alkyl),
(q) -C(=N-NH-C₁-C₆-alkyl)-C₁-C₆-alkyl, and
(r) -C(O)-O-C₁-C₆-alkyl,
(5) C₃-C₆-alkynyl optionally substituted with a substituent selected from the group consisting of
(a) halogen,
(b) C₃-C₆-cycloalkyl,
(c) aryl,
(d) substituted aryl,
(e) heteroaryl, and
(f) substituted heteroaryl,
(6) C₃-C₆-cycloalkyl,
(7) -CHO,
(8) -C(O)-C₁-C₆-alkyl,
(9) -C(O)-NR'R", wherein R' and R" are as previously defined, and
(10) -C(O)-O-C₁-C₆-alkyl,
or R¹ and R² taken together may be -(CH₂)ₚ-, wherein p is 3-to-7, which taken together with the nitrogen atom to which they are attached, thus form a heterocyclic ring containing one nitrogen atom and from 3 to 7 carbon atoms:
R is selected from the group consisting of
(1) methyl substituted with a substituent selected from the group consisting of
(a) -CN,
(b) -F,
(c) -CO₂R³ wherein R³ is C₁-C₃-alkyl, aryl-substituted C₁-C₃-alkyl, or heteroaryl-substituted C₁-C₃-alkyl,
(d) -S(O)ₙ-R³ wherein n is 0, 1, or 2. and R³ is as previously defined,
(e) -NH-C(O)-R³ where R³ is as previously defined,
(f) -NH-C(O)-NR⁴R⁵ wherein R⁴ and R⁵ are independently selected from the group consisting of
(i) hydrogen,
(ii) C₁-C₃-alkyl,
(iii) C₁-C₃-alkyl substituted with aryl,
(iv) C₁-C₃-alkyl substituted with substituted aryl,
(v) C₁-C₃-alkyl substituted with heteroaryl, and
(vi) C₁-C₃-alkyl substituted with substituted heteroaryl,
(g) aryl,
(h) substituted aryl,
(i) heteroaryl,
and
(j) substituted heteroaryl,
(2) C₂-C₁₀-alkyl,
(3) C₂-C₁₀-alkyl substituted with one or more substituents selected from the group consisting of
(a) halogen,
(b) hydroxy,
(c) C₁-C₃-alkoxy,
(d) C₁-C₃-alkoxy-C₁-C₃-alkoxy,
(e) oxo,
(f) -N₃,
(g) -CHO,
(h) -O-SO₂-(substituted C₁-C₆-alkyl),
(i) -NR⁶R⁷ wherein R⁶ and R⁷ are selected from the group consisting of
(i) hydrogen,
(ii) C₁-C₁₂-alkyl,
(iii) substituted C₁-C₁₂-alkyl,
(iv) C₁-C₁₂-alkenyl,
(v) substituted C₁-C₁₂-alkenyl,
(vi) C₁-C₁₂-alkynyl,
(vii) substituted C₁-C₁₂-alkynyl,
(viii) aryl,
(ix) C₃-C₈-cycloalkyl,
(x) substituted C₃-C₈-cycloalkyl,
(xi) substituted aryl,
(xii) heterocycloalkyl,
(xiii) substituted heterocycloalkyl,
(xiv) C₁-C₁₂-alkyl substituted with aryl,
(xv) C₁-C₁₂-alkyl substituted with substituted aryl,
(xvi) C₁-C₁₂-alkyl substituted with heterocycloalkyl,
(xvii) C₁-C₁₂-alkyl substituted with substituted heterocycloalkyl,
(xviii) C₁-C₁₂-alkyl substituted with C₃-C₈-cycloalkyl,
(xix) C₁-C₁₂-alkyl substituted with substituted C₃-C₈-cycloalkyl,
(xx) heteroaryl,
(xxi) substituted heteroaryl,
(xxii) C₁-C₁₂-alkyl substituted with heteroaryl, and
(xxiii) C₁-C₁₂-alkyl substituted with substituted heteroaryl,
or
R⁶ and-R⁷ are taken together with the atom to which they are attached to form a 3-10 membered heterocycloalkyl ring which may be substituted with one or more substituents independently selected from the group consisting of
(i) halogen,
(ii) hydroxy,
(iii) C₁-C₃-alkoxy,
(iv) C₁-C₃-alkoxy-C₁-C₃-alkoxy,
(v) oxo,
(vi) C₁-C₃-alkyl,
(vii) halo-C₁-C₃-alkyl,
and
(viii) C₁-C₃-alkoxy-C₁-C₃-alkyl,
(j) -CO₂R³ wherein R³ is as previously defined,
(k) -C(O)-NR⁴R⁵ wherein R⁴ and R⁵ are as previously defined,
(l) =N-O-R³ wherein R³ is as previously defined,
(m) -C≡N,
(n) -O-S(O)ₙ-R³ wherein n and R³ are as previously defined,
(o) aryl,
(p) substituted aryl,
(q) heteroaryl,
(r) substituted heteroaryl,
(s) C₃-C₈-cycloalkyl,
(t) substituted C₃-C₈-cycloalkyl,
(u) C₁-C₁₂-alkyl substituted with heteroaryl,
(v) heterocycloalkyl,
(w) substituted heterocycloalkyl,
(x) -NH-C(O)-R³ where R³ is as previously defined,
(y) -NH-C(O)-NR⁴R⁵ wherein R⁴ and R⁵ are as previously defined,
(z) =N-NR⁶R⁷ wherein R⁶ and R⁷ are as previously defined,
(aa) =N-R³ wherein R³ is as previously defined,
(bb) =N-NH-C(O)-R⁴ wherein R⁴ is as previously defined,
and
(cc) =N-NH-C(O)-NR⁴R⁵ wherein R⁴ and R⁵ are as previously defined,
(4) C₃-alkenyl substituted with a moiety selected from the group consisting of
(a) halogen,
(b) -CHO,
(c) -CO₂R³ where R³ is as previously defined,
(d) -C(O)-R⁴ where R⁴ is as previously defined,
(e) -C(O)-NR⁴R⁵ wherein R⁴ and R⁵ are as previously defined,
(f) -C≡N,
(g) aryl,
(h) substituted aryl,
(i) heteroaryl,
(j) substituted heteroaryl,
(k) C₃-C₇-cycloatkyl,
and
(l) C₁-C₁₂-alkyl substituted with heteroaryl,
(5) C₄-C₁₀-alkenyl,
(6) C₄-C₁₀-alkenyl substituted with one or more substituents selected from the group consisting of
(a) halogen,
(b) C₁-C₃-alkoxy,
(c) oxo,
(d) -CHO,
(e) -CO₂R³ where R³ is as previously defined,
(f) -C(O)-NR⁴R⁵ wherein R⁴ and R⁵ are as previously defined,
(g) -NR⁶R⁷ wherein R⁶ and R⁷ are as previously defined,
(h) =N-O-R³ wherein R³ is as previously defined,
(i) -C≡N,
(j) -O-S(O)ₙ-R³ wherein n is 0,1 or 2 and R³ is as previously defined,
(k) aryl,
(l) substituted aryl,
(m) heteroaryl,
(n) substituted heteroaryl,
(o) C₃-C₇-cycloalkyl,
(p) C₁-C₁₂-alkyl substituted with heteroaryl,
(q) -NH-C(O)-R³ where R³ is as previously defined,
(r) -NH-C(O)-NR⁴R⁵ wherein R⁴ and R⁵ are as previously defined,
(s) =N-NR⁶R⁷ wherein R⁶ and R⁷ are as previously defined,
(t) =N-R³ wherein R³ is as previously defined,
(u) =N-NH-C(O)-R³ where R³ is as previously defined,
and
(v) =N-NH-C(O)-NR⁴R⁵ wherein R⁴ and R⁵ are as previously defined,
(7) C₃-C₁₀-alkynyl,
and
(8) C₃-C₁₀-alkynyl substituted with one or more substituents selected from the group contisting of
(a) trialkylsilyl,
(b) aryl,
(c) substituted aryl,
(d) heteroaryl,
and
(e) substituted heteroaryl,
with the proviso that when R is allyl and R¹ is methyl, R² is not H;
R^{p} is hydrogen or a hydroxy protecting group;
R^{w} is selected from the group consisting of
(1) hydrogen,
(2) C₁-C₆-alkyl, optionally substituted with one or more substituents selected from the group consisting of
(a) aryl,
(b) substituted aryl,
(c) heteroaryl,
(d) substituted heteroaryl,
(3) a group selected from option (2) as previously defined further substituted with - CH₂-M-R⁸, wherein M is selected from the group consisting of
(i) -O-,
(ii) -NH-,
(iii) -N(CH₃)-,
(iv) -S(O)ₙ-, wherein n is as described previously,
(v) -NH-C(O)-, and
(vi) -C(O)-NH-,
and
R⁸ is selected from the group consisting of
(i) -(CH₂)ₙ-aryl, wherein n is as described previously,
(ii) -(CH₂)ₙ-substituted aryl, wherein n is as described previously,
(iii) -(CH₂)ₙ-heteroaryl, wherein n is as described previously,
(iv) -(CH₂)ₙ-substituted heteroaryl, wherein n is as described previously,
and
(v) -(CH₂)ₙ-heterocycloalkyl, wherein n is as described previously;
and
W is absent or is selected from the group consisting of -O-, -NH- and -N(CH₃)-

2. A compound according to Claim 1 which is selected from the group consisting of
Compound of Formula (I), R is -CH₂CH=CH-(3-quinolyl), R^{p} is H, R is methyl, R² is hydrogen;
Compound of formula (II), R is -CH₂CH=CH-(3-quinolyl), R^{p} is acetyl, R¹ is H, R² is CH₃, W is absent, R^{w} is H;
Compound of Formula (II); R is -CH₂CH=CH-(3-quinolyl), R^{p} is H, W is absent, R^{w} is H, R¹ is H, R² is CH₃;
Compound of Formula (II); R is -CH₂CH=CH-(3-quinolyl), R^{p} is H, W is absent, R^{w} is H, R¹ is acetyl, R² is CH₃;
Compound of Formula (II); R is -CH₂CH=CH-(3-quinolyl), R^{p} is H, W is absent, R^{w} is H, R¹ is CH₂C(O)-O-CH₂CH₃, R² is CH₃;
Compound of Formula (II); R is -CH₂CH=CH-(3-quinolyl), R^{p} is H, W is absent, R^{w} is H, R¹ is CH₂CH=CH₂, R² is CH₃;
Compound of Formula (II); R is -CH₂CH=CH-(3-quinolyl), R^{p} is H, W is absent, R^{w} is H, R¹ is CH₂CH₂F, R² is CH₃;
Compound of Formula (II); R is -CH₂CH=CH-(3-quinolyl), R^{p} is H, W is absent, R^{w} is H, R¹ is CH₂-phenyl, R² is CH₃;
Compound of Formula (II); R is -CH₂CH=CH-(3-quinolyl), R^{p} is H, W is absent, R^{w} is H, R¹ is CH₂-CN, R² is CH₃;
Compound of Formula (II); R is -CH₂CH=CH-(3-quinolyl), R^{p} is H, W is absent, R^{w} is H, R¹ is CH₂-C≡CH, R² is CH₃;
Compound of Formula (II); R is -CH₂CH=CH-(3-quinolyl), R^{p} is H, W is absent, R^{w} is H, R¹ is CH₂CH₂CH₃, R² is CH₃;
Compound of Formula (II); R is -CH₂CH=CH-(3-quinolyl), R^{p} is H, W is absent, R^{w} is H, R¹ is CH₂-cyclopropyl, R² is CH₃;
Compound of Formula (II); R is -CH₂CH=CH-(3-quinolyl), R^{p} is H, W is absent, R^{w} is H, R¹ is cyclopropyl, R² is CH₃;
Compound of Formula (II); R is -CH₂CH=CH-(3-quinolyl), R^{p} is H, W is absent, R^{w} is H, R¹ is CH₂-(3-pyridyl), R² is CH₃;
Compound of Formula (II); R is -CH₂CH=CH-(3-quinolyl), R^{p} is H, W is absent, R^{w} is H, R¹ is CH₂-(cyclo-C₃H₅), R² is CH₃;
Compound of Formula (II); R is -CH₂CH=CH-(3-quinolyl), R^{p} is H, W is absent, R^{w} is H, R¹ is CH₂CH₂CH₃, R² is CH₃;
Compound of Formula (II); R is -CH₂CH=CH-(3-quinolyl), R^{p} is H, W is absent, R^{w} is H, R¹ is CH₂CH=CHC₆H₅, R² is CH₃;
Compound of Formula (II): R is -CH₂CH=CH-(3-quinolyl), R^{p} is H, W is absent, R^{w} is H, R¹ is CH₂C(=CH₂)C(O)OCH₃, R² is CH₃;
Compound of Formula (II): R is -CH₂CH=CH-(3-quinolyl), R^{p} is H, W is absent, R^{w} is H, R¹ is CH₂C(=CH₂)CH₃, R² is CH₃;
Compound of Formula (II): R is -CH₂CH=CH-(3-quinolyl), R^{p} is H, W is absent, R^{w} is H, R¹ is cyclo-C₃H₅, R² is CH₃;
Compound of Formula (II): R is -CH₂CH=CH-(3-quinolyl), R^{p} is H, W is absent, R^{w} is H, R¹ is CH₂-(3-pyridyl), R² is CH₃;
Compound of Formula (II): R is -CH₂CH=CH-(3-quinolyl), R^{p} is H, W is absent, R^{w} is H, R¹ is CH₂-(3-hydroxyphenyl), R² is CH₃;
Compound of Formula (II): R is -CH₂CH=CH-(3-quinolyl), R^{p} is H, W is absent, R^{w} is H, R¹ is CH₂-(2-hydroxy-3-*tert*-butyl-5-methylphenyl), R² is CH₃;
Compound of Formula (II): R is -CH₂CH=CH-(3-quinolyl), R^{p} is H, W is absent, R^{w} is H, R¹ is CH₂-(2-hydroxy-3,4-dimethylphenyl), R² is CH₃;
Compound of Formula (II): R is -CH₂CH=CH-(3-quinolyl), R^{p} is H, W is absent, R^{w} is H, R¹ is CH₂-(2-hydroxy-3-methoxy-5-(2-propenyl)phenyl),
R² is CH₃;
Compound of Formula (II): R is -CH₂CH=CH-(3-quinolyl), R^{p} is H, W is absent, R^{w} is H, R¹ is CH₂-(2-hydroxy-3-methoxy-5-methylphenyl), R² is CH₃;
Compound of Formula (II): R is -CH₂CH=CH-(3-quinolyl), R^{p} is H, W is absent, R^{w} is H, R¹ is CH₂-(2-hydroxy-5-cyclopentylphenyl), R² is CH₃;
Compound of Formula (II): R is -CH₂CH=CH-(3-quinolyl), R^{p} is H, W is absent, R^{w} is H, R¹ is CH₂-(2-hydroxy-5-carboxamidophenyl), R² is CH₃;
Compound of Formula (II): R is -CH₂CH=CH-(3-quinolyl). R^{p} is H, W is absent, R^{w} is H, R¹ is CH₂-(2-hydroxy-3-methoxy-5-(2-methoxycarbonylethyl)phenyl), R² is CH₃;
Compound of Formula (II): R is -CH₂CH=CH-(3-quinolyl), R^{p} is H, W is absent, R^{w} is H, R is CH₂-(2-hydroxy-3-methyl-5-fluorophenyl), R² is CH₃;
Compound of Formula (II): R is -CH₂CH=CH-(3-quinolyl), R^{p} is H, W is absent, R^{w} is H, R¹ is CH₂-(2-hydroxy-3-methoxy-5-acetylphenyl), R² is CH₃;
Compound of Formula (II); R is -CH₂CH=CH-(3-quinolyl), R^{p} is H, W is absent, R^{w} is H, R¹ is CH₂-(2-hydroxy-3-bromophenyl), R² is CH₃;
Compound of Formula (II); R is -CH₂CH=CH-(3-quinolyl), R^{p} is H, W is absent, R^{w} is H, R is CH₂-(2-hydroxy-3-methoxy-5-alkoxycarbonylphenyl),
R² is CH₃:
Compound of Formula (II): R is -CH₂CH=CH-(3-quinolyl), R^{p} is H, W is absent, R^{w} is H, R¹ is CH₂-(2-hydroxy-3-ethylphenyl), R² is CH₃;
Compound of Formula (II): R is -CH₂CH=CH-(3-quinolyl), R^{p} is H, W is absent, R^{w} is H, R¹ is CH₂-(2-hydroxy-5-isobutylphenyl), R² is CH₃;
Compound of Formula (II): R is -CH₂CH=CH-(3-quinolyl), R^{p} is H, W is absent, R^{w} is H, R¹ is CH₂-(2-hydroxy-3-methyl-5-diethylamino-6-methylphenyl), R² is CH₃;
Compound of Formula (II): R is -CH₂CH=CH-(3-quinolyl), R^{p} is H, W is absent, R^{w} is H, R¹ is CH₂-(2-hydroxy-4-methyl-5-bromo-6-methylphenyl),
R² is CH₃; and
Compound of Formula (II): R is -CH₂CH=CH-(3-quinolyl), R^{p} is H, W is absent, R^{w} is H, R¹ is CH₂-(2-hydroxy-3-hydroxymethylphenyl), R² is CH₃.

3. A pharmaceutical composition for treating bacterial infections comprising a therapeutically effective amount of a compound of Claim 1 or a pharmaceutically acceptable salt or ester thereof in combination with a pharmaceutically acceptable carrier.

4. Use of a compound of Claim 1 or a pharmaceutically acceptable salt or ester thereof for manufacturing a medicament for treating bacterial infections by administration to a mammal in need of such treatment of a pharmaceutical composition containing a therapeutically effective amount of said compound or pharmaceutically acceptable salt or ester thereof.

5. A compound according to Claim 1 having the formula (I)

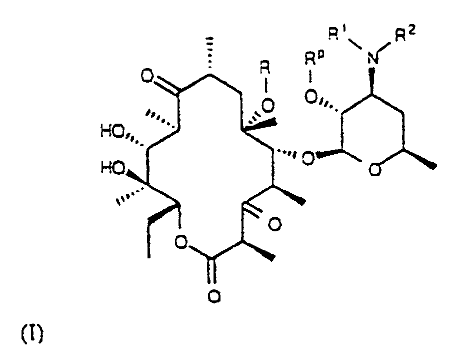

6. A compound according to Claim 1 having the formula (II)

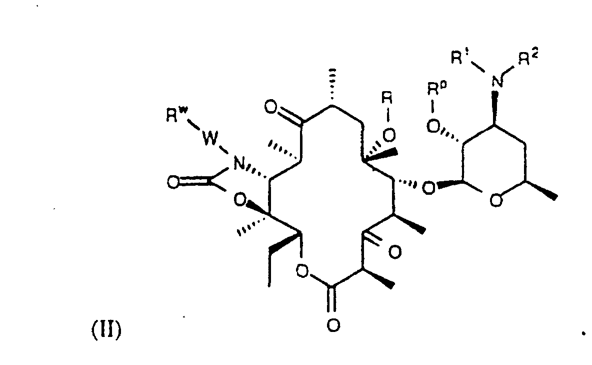

7. A compound according to Claim 1 having the formula (III)

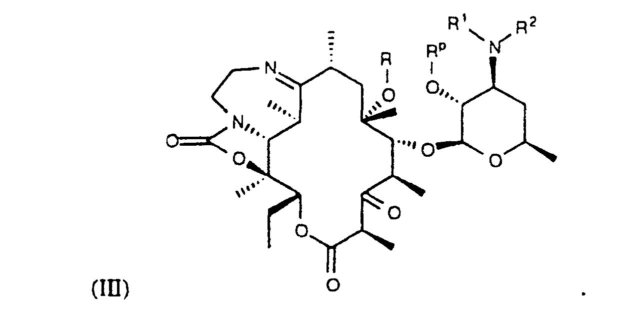

8. A compound according to Claim 1 having the formula (IV)

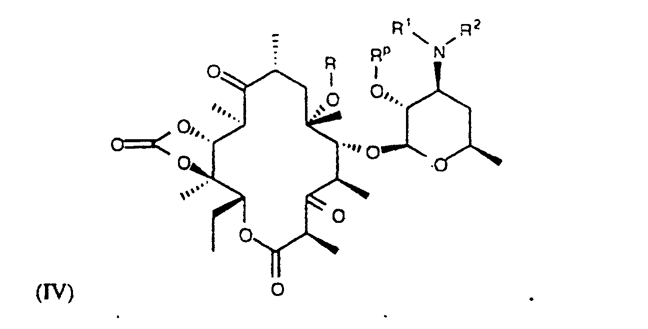

9. A compound according to Claim 1 having the formula (V)

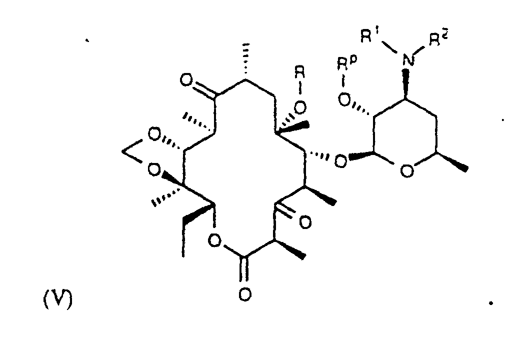

10. A process for preparing a compound selected from the group consisting of 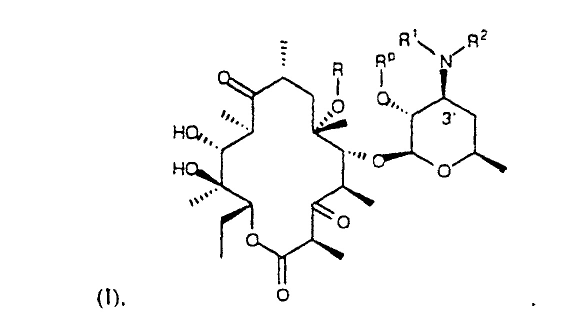 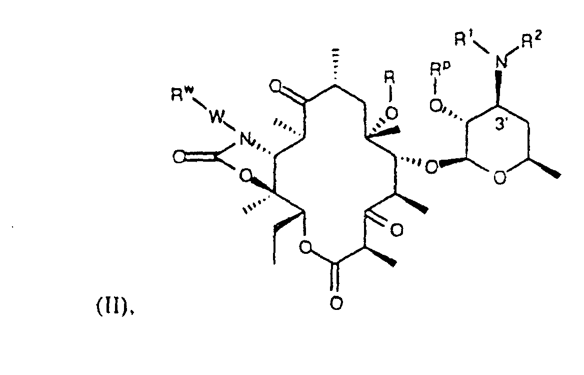 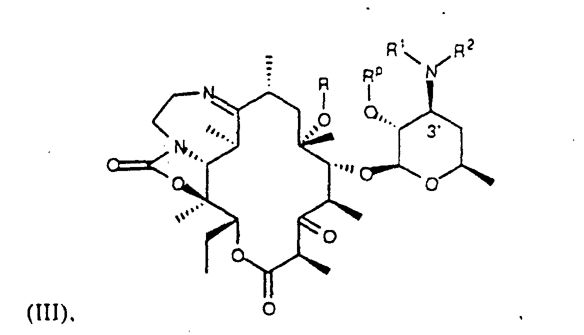 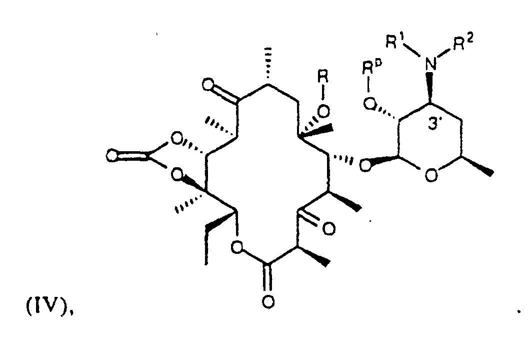 and 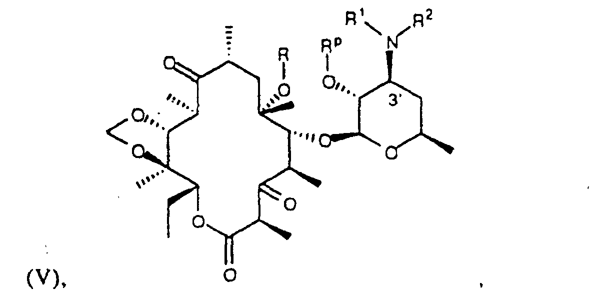 wherein
R¹ and R², with the proviso that R¹ and R² are not both methyl, are independently selected from the group consisting of
(1) hydrogen,
(2) C₁-C₆-alkyl optionally substituted with a substituent selected from the group consisting of
(a) halogen,
(b) C₃-C₆-cycloalkyl,
(c) aryl,
(d) substituted aryl,
(e) heteroaryl,
(f) substituted heteroaryl,
(g) -CHO,
(h) -C(O)-C₁-C₆-alkyl, and
(i) -C(O)-NR'R", wherein R' and R" are independently selected from the group consisting of hydrogen. C₁-C₃-alkyl, C₁-C₃-alkyl substituted with aryl, substituted aryl, heteroaryl, and substituted heteroaryl.
(3) C₂-C₆-alkyl optionally substituted with a substituent selected from the group consisting of
(a) C₁-C₆-alkoxy,
(b) -NR'R", wherein R' and R" are as previously defined,
(c) -NH-C(O)-C₁-C₆-alkyl,
(d) -NH-C(O)-O-C₁-C₆-alkyl,
(e) -O-C(O)-O-C₁-C₆-alkyl,
(f) -O-C(O)-C₁-C₆-alkyl,
(g) -CH(=N-O-C₁-C₆-alkyl),
(h) -C(=N-O-C₁-C₆-alkyl)-C₁-C₆-alkyl,
(i) -CH(=N-NH-C₁-C₆-alkyl), and
(j) -C(=N-NH-C₁-C₆-alkyl)-C₁-C₆-alkyl,
(4) C₃-C₆-alkenyl optionally substituted with a substituent selected from the group consisting of
(a) halogen,
(b) C₃-C₆-cycloalkyl,
(c) aryl,
(d) substituted aryl,
(e) heteroaryl,
(f) substituted heteroaryl,
(g) -NH-C(O)-C₁-C₆-alkyl,
(h) -NH-C(O)-O-C₁-C₆-alkyl,
(i) -O-C(O)-O-C₁-C₆-alkyl,
(j) -O-C(O)-C₁-C₆-alkyl,
(k) -CHO,
(l) -C(O)-C₁-C₆-alkyl,
(m) -C(O)-NR'R", wherein R' and R" are as previously defined,
(n) -CH(=N-O-C₁-C₆-alkyl),
(o) -C(=N-O-C₁-C₆-alkyl)-C₁-C₆-alkyl,
(p) -CH(=N-NH-C₁-C₆-alkyl),
(q) -C(=N-NH-C₁-C₆-alkyl)-C₁-C₆-alkyl, and
(r) -C(O)-O-C₁-C₆-alkyl,
(5) C₃-C₆-alkynyl optionally substituted with a substituent selected from the group consisting of
(a) halogen,
(b) C₃-C₆-cycloalkyl,
(c) aryl,
(d) substituted aryl,
(e) heteroaryl, and
(f) substituted heteroaryl,
(6) C₃-C₆-cycloalkyl,
(7) -CHO,
(8) -C(O)-C₁-C₆-alkyl,
(9) -C(O)-NR'R". wherein R' and R" are as previously defined, and
(10) -C(O)-O-C₁-C₆-alkyl,
or R¹ and R² taken together may be -(CH₂)ₚ-, wherein p is 3-to-7, which taken together with the nitrogen atom to which they are attached, thus form a heterocyclic ring containing one nitrogen atom and from 3 to 7 carbon atoms:
R is selected from the group consisting of
(1) methyl substituted with a substituent selected from the group consisting of
(a) -CN,
(b) -F,
(c) -CO₂R³ wherein R³ is C₁-C₃-alkyl, aryl-substituted C₁-C₃-alkyl, or heteroaryl-substituted C₁-C₃-alkyl,
(d) -S(O)ₙ-R³ wherein n is 0,1, or 2, and R³ is as previously defined,
(e) -NH-C(O)-R³ where R³ is as previously defined,
(f) -NH-C(O)-NR⁴R⁵ wherein R⁴ and R⁵ are independently selected from the group consisting of
(i) hydrogen,
(ii) C₁-C₃-alkyl,
(iii) C₁-C₃-alkyl substituted with aryl,
(iv) C₁-C₃-alkyl substituted with substituted aryl,
(v) C₁-C₃-alkyl substituted with heteroaryl, and
(vi) C₁-C₃-alkyl substituted with substituted heteroaryl,
(g) aryl,
(h) substituted aryl,
(i) heteroaryl,
and
(j) substituted heteroaryl,
(2) C₂-C₁₀-alkyl,
(3) C₂-C₁₀-alkyl substituted with one or more substituents selected from the group consisting of
(a) halogen,
(b) hydroxy,
(c) C₁-C₃-alkoxy,
(d) C₁-C₃-alkoxy-C₁-C₃-alkoxy,
(e) oxo,
(f) -N₃,
(g) -CHO,
(h) -O-SO₂-(substituted C₁-C₆-alkyl),
(i) -NR⁶R⁷ wherein R⁶ and R⁷ are selected from the group consisting of
(i) hydrogen,
(ii) C₁-C₁₂-alkyl,
(iii) substituted C₂-C₁₂-alkyl,
(iv) C₁-C₁₂-alkenyl,
(v) substituted C₁-C₁₂-alkenyl,
(vi) C₁-C₁₂-alkynyl,
(vii) substituted C₁-C₁₂-alkynyl,
(viii) aryl,
(ix) C₃-C₈-cycloalkyl,
(x) substituted C₃-C₈-cycloalkyl,
(xi) substituted aryl,
(xii) heterocycloalkyl,
(xiii) substituted heterocycloalkyl,
(xiv) C₁-C₁₂-alkyl substituted with aryl,
(xv) C₁-C₁₂-alkyl substituted with substituted aryl,
(xvi) C₁-C₁₂-alkyl substituted with heterocycloalkyl,
(xvii) C₁-C₁₂-alkyl substituted with substituted heterocycloalkyl.
(xviii) C₁-C₁₂-alkyl substituted with C₃-C₈-cycloalkyl,
(xix) C₁-C₁₂-alkyl substituted with substituted C₃-C₈-cycloalkyl,
(xx) heteroaryl,
(xxi) substituted heteroaryl,
(xxii) C₁-C₁₂-alkyl substituted with heteroaryl,
and
(xxiii) C₁-C₁₂-alkyl substituted with substituted heteroaryl,
or
R⁶ and R⁷ are taken together with the atom to which they are attached to form a 3-10 membered heterocycloalkyl ring which may be substituted with one or more substituents independently detected from the group consisting of
(i) halogen,
(ii) hydroxy,
(iii) C₁-C₃-alkoxy,
(iv) C₁-C₃-alkoxy-C₁-C₃-alkoxy,
(v) oxo,
(vi) C₁-C₃-alkyl,
(vii) halo-C₁-C₃-alkyl,
and
(vii) C₁-C₃-alkoxy-C₁-C₃-alkyl,
(j) -CO₂R³ wherein R³ is as previously defined,
(k) -C(O)-NR⁴R⁵ wherein R⁴ and R⁵ are as previously defined,
(l) =N-O-R³ wherein R³ is as previously defined,
(m) -C≡N,
(n) -O-S(O)ₙ-R³ wherein n and R³ are as previously defined,
(o) aryl,
(p) substituted aryl,
(q) heteroaryl,
(r) substituted heteroaryl,
(s) C₃-C₈-cycloalkyl,
(t) substituted C₃-C₈-cycloalkyl,
(u) C₁-C₁₂-alkyl substituted with heteroaryl,
(v) heterocycloalkyl,
(w) substituted heterocycloalkyl,
(x) -NH-C(O)-R³ where R³ is as previously defined,
(y) -NH-C(O)=NR⁴R⁵ wherein R⁴ and R⁵ are as previously defined,
(z) =N-NR⁶R⁷ wherein R⁶ and R⁷ are as previously defined,
(aa) =N-R³ wherein R³ is as previously defined,
(bb) =N-NH-C(O)-R⁴ wherein R⁴ is as previously defined,
and
(cc) =N-NH-C(O)-NR⁴R⁵ wherein R⁴ and R⁵ are as previously defined.
(4) C₃-alkenyl substituted with a moiety selected from the group consisting of
(a) halogen,
(b) -CHO,
(c) -CO₂R³ where R³ is as previously defined,
(d) -C(O)-R⁴ where R⁴ is as previously defined,
(e) -C(O)-NR⁴R⁵ wherein R⁴ and R⁵ are as previously defined,
(f) -C≡N,
(g) aryl,
(h) substituted aryl,
(i) heteroaryl,
(j) substituted heteroaryl,
(k) C₃-C₇-cycloalkyl,
and
(I) C₁-C₁₂-alkyl substituted with heteroaryl,
(5) C₄-C₁₀-alkenyl,
(6) C₄-C₁₀-alkenyl substituted with one or more substituents selected from the group consisting of
(a) halogen,
(b) C₁-C₃-alkoxy,
(c) oxo,
(d) -CHO,
(e) -CO₂R³ where R³ is as previously defined,
(f) -C(O)-NR⁴R⁵ wherein R⁴ and R⁵ are as previously defined,
(g) -NR⁶R⁷ wherein R⁶ and R⁷ are as previously defined,
(h) =N-O-R³ wherein R³ is as previously defined,
(i) -C≡N,
(j) -O-S(O)ₙ-R³ wherein n is 0,1, or 2 and R³ is as previously defined,
(k) aryl,
(I) substituted aryl,
(m) heteroaryl,
(n) substituted heteroaryl,
(o) C₃-C₇-cycloalkyl,
(p) C₁-C₁₂-alkyl substituted with heteroaryl,
(q) -NH-C(O)-R³ where R³ is as previously defined,
(r) -NH-C(O)-NR⁴R⁵ wherein R⁴ and R⁵ are as previously defined,
(s) =N-NR⁶R⁷ wherein R⁶ and R⁷ are as previously defined,
(t) =N-R³ wherein R³ is as previously defined,
(u) =N-NH-C(O)-R³ where R³ is as previously defined,
and
(v) =N-NH-C(O)-NR⁴R⁵ wherein R⁴ and R⁵ are as previously defined,
(7) C₃-C₁₀-alkynyl,
and
(8) C₃-C₁₀-alkynyl substituted with one or more substituents selected from the group consisting of
(a) trialkylsilyl,
(b) aryl,
(c) substituted aryl,
(d) heteroaryl,
and
(e) substituted heteroaryl,
with the proviso that when R is allyl and R¹ is methyl, R² is not H;
R^{p} is hydrogen or a hydroxy protecting group;
R^{w} is selected from the group consisting of
(1) hydrogen,
(2) C₁-C₆-alkyl, optionally substituted with one or more substituents selected from the group consisting of
(a) aryl,
(b) substituted aryl,
(c) heteroaryl,
(d) substituted heteroaryl,
(3) a group selected from option (2) as previously defined further substituted with -CH₂-M-R⁸, wherein M is selected from the group consisting of
(i) -O-,
(ii) -NH-,
(ii) -N(CH₃)-,
(iv) -S(O)ₙ-, wherein n is as described previously,
(v) -NH-C(O)-, and
(vi) -C(O)-NH-,
and
R⁸ is selected from the group consisting of
(i) -(CH₂)ₙ-aryl, wherein n is as described previously,
(ii) -(CH₂)ₙ-substituted aryl, wherein n is as described previously,
(iii) -(CH₂)ₙ-heteroaryl, wherein n is as described previously,
(iv) -(CH₂)ₙ-substituted heteroaryl, wherein n is as described previously, and
(v) -(CH₂)ₙ-heterocycloalkyl, wherein n is as described previously;
and
W is absent or is selected from the group consisting of -O-, -NH- and -N(CH₃)-, the method comprising:
(a) sequentially desmethylating 3'-nitrogen of a compound selected from the group consisting of 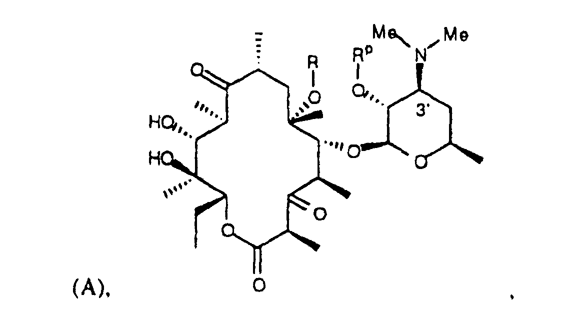 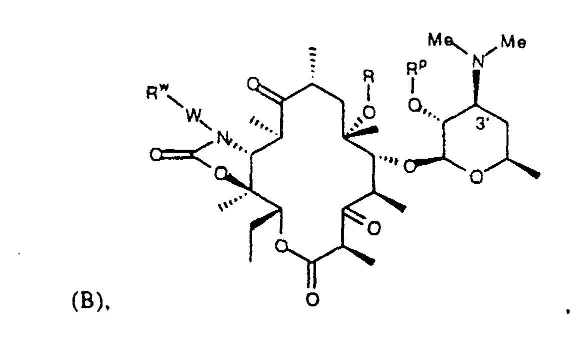 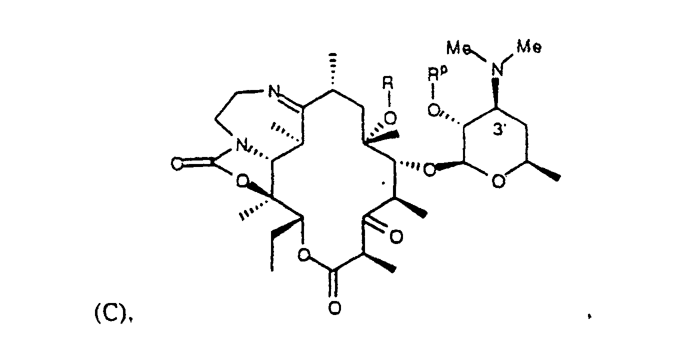 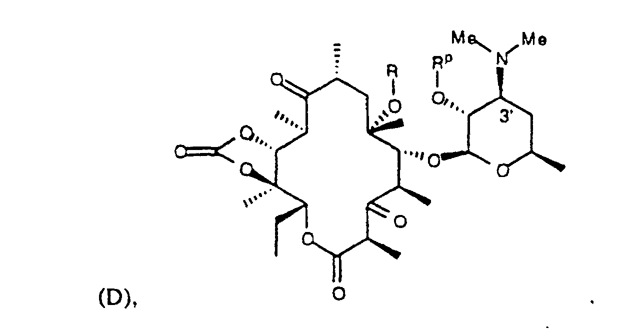 and 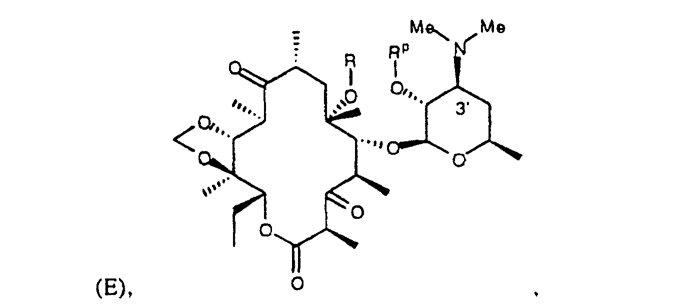 wherein R, and R^{p} are as defined previously; and
(b) sequentially reacting the compound from step (a) with a R¹-and a R²-precursor compound.

11. The process of Claim 10, wherein the desmethylation of the 3'-nitrogen is obtained by reacting the compound with N-iodosuccinimide to afford a corresponding compound having a 3'-NHCH₃ group.

12. The process of Claim 11, wherein in step (b), the compound is reacted with a R¹-precursor selected from the group consisting of
(i) R¹-X wherein R¹ is as defined previously and X is a halide or sulfonate leaving group,
(ii) an aldehyde of formula R*-CHO followed by reduction to give R*-CH₂, the R¹ moiety described previously,
(iii) carbonyldiimidazole to give an intermediate compound wherein R¹ is imidazolylcarbonyl and reacting this intermediate with an amine having the formula HNR'R", wherein R' and R" are as defined previously, to give a compound wherein R¹ is C(O)-NR'R",
(iv) an alcohol of the formula HOR', wherein R' is as previously defined, to give a compound wherein R¹ is C(O)-OR',
(v) an acylating agent of the formula X-C(O)-R', wherein X is halogen and R' is as defined previously, or O-(C(O)-R')₂ to give a compound wherein R¹ is C(O)R', and
(vi) a substituted or unsubstituted aryl alcohol and a homologating agent selected from formaldehyde or paraformaldehyde to give a compound wherein R¹ is methyl substituted with substituted aryl.

13. The process of Claim 11, further comprising treating the compound with iodosuccinimide or iodine in presence of light to afford corresponding compound having a 3'-NH₂ group.

14. The process of Claim 13, further comprising treating the compound with a R¹-precursor to afford a compound having a 3'-NHR¹CH₃ group, wherein the R¹-precursor is selected from the group consisting of
(i) R¹-X wherein R¹ is as defined previously and X is a halide or sulfonate leaving group,
(ii) an aldehyde of formula R*-CHO followed by reduction to give R*-CH₂, the R¹ moiety described previously,
(iii) carbonyldiimidazole to give an intermediate compound wherein R¹ is imidazolylcarbonyl and reacting this intermediate with an amine having the formula HNR'R", wherein R' and R" are as defined previously, to give a compound wherein R¹ is C(O)-NR'R",
(iv) an alcohol of the formula HOR', wherein R' is as previously defined, to give a compound wherein R¹ is C(O)-OR',
(v) an acylating agent of the formula X-C(O)-R', wherein X is halogen and R' is as defined previously, or O-(C(O)-R')₂ to give a compound wherein R¹ is C(O)R', and
(vi) a substituted or unsubstituted aryl alcohol and a homologating-agent selected from formaldehyde or paraformaldehyde to give a compound wherein R¹ is methyl substituted with substituted aryl.

15. The process of Claim 14, further comprising treating the compound with a R²-precursor compound to afford a compound having a 3'-NR¹R² group, wherein the R²-precursor is selected from the group consisting of
(i) R²-X wherein R² is as defined previously and X is a halide or sulfonate leaving group,
(ii) an aldehyde of formula R*-CHO followed by reduction to give R*-CH₂-, the R² moiety described previously,
(iii) carbonyldiimidazole to give an intermediate compound wherein R² is imidazolylcarbonyl and reacting this intermediate with an amine having the formula HNR'R", wherein R' and R" are as previously defined, to give a compound wherein R² is C(O)-NR'R",
(iv) an alcohol of the formula HOR' to give a compound wherein R² is C(O)-OR',
(v) an acylating agent of the formula X-C(O)-R', wherein X is halogen and R' is as defined previously, or O-(C(O)-R')₂ to give a compound wherein R² is C(O)R', and
(vi) a substituted or unsubstituted aryl alcohol and a homologating agent selected from formaldehyde or paraformaldehyde to give a compound wherein in R¹ is methyl substituted with substituted aryl.

## Patentansprüche

1. Eine Verbindung gewählt aus der Gruppe bestehend aus 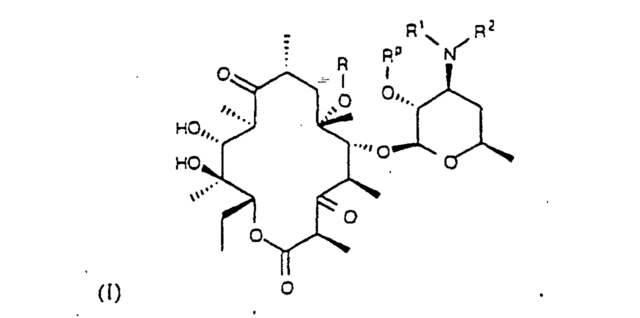 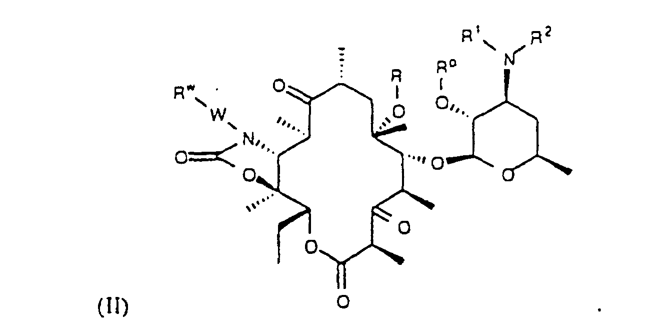 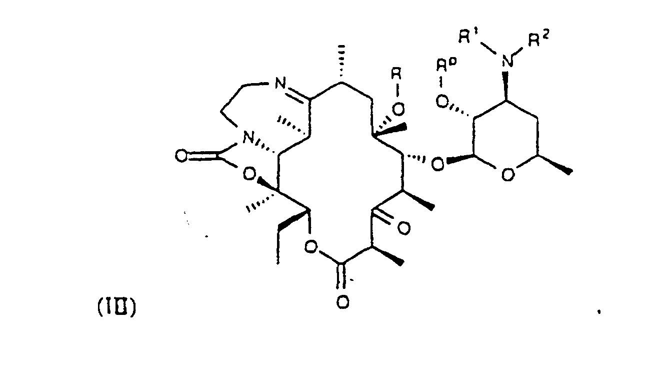 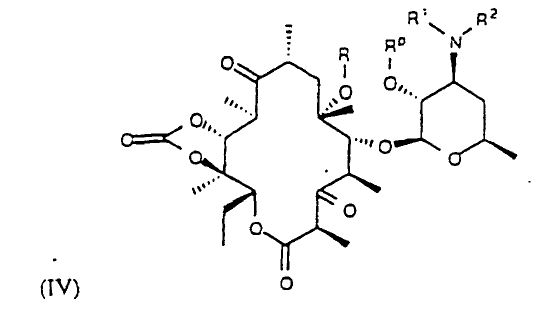 und 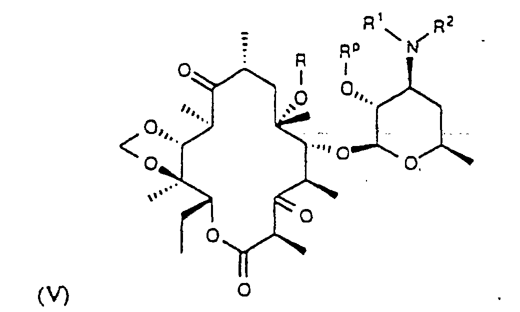 oder ein pharmazeutisch verträgliches Salz, Ester oder Prodrug davon,
worin
R¹ und R², unter der Voraussetzung, daß R¹ und R² nicht beide Methyl sind, unabhängig gewählt sind aus der Gruppe bestehend aus
(1) Wasserstoff,
(2) C₁-C₆-Alkyl, wahlweise substituiert mit einem Substituenten gewählt aus der Gruppe bestehend aus
(a) Halogen,
(b) C₃-C₆-Cycloalkyl,
(c) Aryl,
(d) substituiertem Aryl,
(e) Heteroaryl,
(f) substituiertem Heteroaryl,
(g) -CHO,
(h) -C (O) -C₁-C₆-Alkyl, und
(i) -C(O)-NR'R'', worin R' und R" unabhängig gewählt sind aus der Gruppe bestehend aus Wasserstoff, C₁-C₃-Alkyl, C₁-C₃-Alkyl substituiert mit Aryl, substituiertem Aryl, Heteroaryl und substituiertem Heteroaryl,
(3) C₂-C₆-Alkyl, wahlweise substituiert mit einem Substituenten gewählt aus der Gruppe bestehend aus
(a) C₁-C₆-Alkoxy,
(b) -NR'R'', worin R' und R" wie vorher definiert sind,
(c) -NH-C (O) -C₁-C₆-Alkyl,
(d) -NH-C (O) -O-C₁-C₆-Alkyl,
(e) -O-C (O) -O-C₁-C₆-Alkyl,
(f) -O-C(O)-C₁-C₆-Alkyl,
(g) -CH (=N-O-C₁-C₆-Alkyl),
(h) -C (=N-O-C₁-C₆-Alkyl) -C₁-C₆-alkyl,
(i) -CH (=N-NH-C₁-C₆-Alkyl), und
(j) -C (=N-NH-C₁-C₆-Alkyl) -C₁-C₆-alkyl,
(4) C₃-C₆-Alkenyl wahlweise substituiert mit einem Substituenten gewählt aus der Gruppe bestehend aus
(a) Wasserstoff,
(b) C₃-C₆-Cycloalkyl,
(c) Aryl,
(d) substituiertem Aryl,
(e) Heteroaryl,
(f) substituiertem Heteroaryl,
(g) -NH-C (O) -C₁-C₆-Alkyl,
(h) -NH-C (O) -O-C₁-C₆-Alkyl,
(i) -O-C (O) -O-C₁-C₆-Alkyl,
(j) -O-C (O) -C₁-C₆-Alkyl,
(k) -CHO-
(l) -C(O)-C₁-C₆-Alkyl,
(m) -C(O)-NR'R'', worin R' und R'' wie vorher definiert sind
(n) -CH (=N-O-C₁-C₆-Alkyl),
(o) -C (=N-O-C₁-C₆-Alkyl) -C₁-C₆-alkyl,
(p) -CH (=N-NH-C₁-C₆-Alkyl),
(q) -C(=N-NH-C₁-C₆-Alkyl)-C₁-C₆-alkyl, und
(r) -C(O)-O-C₁-C₆-Alkyl,
(5) C₃-C₆-Alkinyl, wahlweise substituiert mit einem Substituenten, gewählt aus der Gruppe bestehend aus
(a) Halogen,
(b) C₃-C₆-Cycloalkyl,
(c) Aryl,
(d) substituiertes Aryl,
(e) Heteroaryl, und
(f) substituiertes Heteroaryl,
(6) C₃-C₆-Cycloalkyl,
(7) -CHO,
(8) -C (O) -C₁-C₆-Alkyl,
(9) -C(O)-NR'R'', worin R' und R" wie vorher definiert sind und
(10) -C (O) -O-C₁-C₆-Alkyl,
oder R¹ und R² zusammengenommen können -(CH₂)ₚ- sein, worin p 3 bis 7 ist, welche zusammengenommen mit dem Stickstoffatom, an welches sie gebunden sind, folglich einen heterozykischen Ring bilden, der ein Stickstoffatom und von 3 bis 7 Kohlenstoffatome enthält;
R ist gewählt aus der Gruppe bestehend aus
(1) Methyl substituiert mit einem Substituenten gewählt aus der Gruppe bestehend aus
(a) -CN,
(b) -F,
(c) -CO₂R³, worin R³ C₁-C₃-Alkyl, Aryl-substituiertes C₁-C₃-Alkyl oder Heteroaryl-substituiertes C₁-C₃-Alkyl ist,
(d) -S(O)ₙ-R³, worin n 0, 1 oder 2 ist, und R³ ist wie vorher definiert,
(e) -NH-C(O)-R³, worin R³ wie vorher definiert ist,
(f) -NH-C(O)-NR⁴R⁵, worin R⁴ und R⁵ unabhängig gewählt sind aus der Gruppe bestehend aus
(i) Wasserstoff,
(ii) C₁-C₃-Alkyl,
(iii) C₁-C₃-Alkyl substituiert mit Aryl,
(iv) C₁-C₃-Alkyl substituiert mit substituiertem Aryl,
(v) C₁-C₃-Alkyl substituiert mit Heteroaryl,
und
(vi) C₁-C₃-Alkyl substituiert mit substituiertem Heteroaryl,
(g) Aryl,
(h) substituiertem Aryl,
(i) Heteroaryl,
und
(j) substituiertem Heteroaryl,
(2) C₂-C₁₀-Alkyl,
(3) C₂-C₁₀-Alkyl substituiert mit einem oder mehreren Substituenten gewählt aus der Gruppe bestehend aus
(a) Halogen,
(b) Hydroxy,
(c) C₁-C₃-Alkoxy,
(d) C₁-C₃-Alkoxy-C₁-C₃-alkoxy,
(e) Oxo,
(f) -N₃,
(g) -CHO,
(h) -O-SO₂-(substituiertes C₁-C₆-Alkyl), (i) -NR⁶R⁷, worin R⁶ und R⁷ gewählt sind aus der Gruppe bestehend aus
(i) Wasserstoff,
(ii) C₁-C₁₂-Alkyl,
(iii) substituiertes C₁-C₁₂-Alkyl,
(iv) C₁-C₁₂-Alkenyl,
(v) substituiertes C₁-C₁₂-Alkenyl,
(vi) C₁-C₁₂-Alkinyl,
(vii) substituiertes C₁-C₁₂-Alkinyl,
(viii) Aryl,
(ix) C₃-C₈-Cycloalkyl,
(x) substituiertes C₃-C₈-Cycloalkyl,
(xi) substituiertes Aryl,
(xii) Heterocycloalkyl,
(xiii) substituiertes Heterocycloalkyl,
(xiv) C₁-C₁₂-Alkyl substituiert mit Aryl,
(xv) C₁-C₁₂-Alkyl substituiert mit substituiertem Aryl,
(xvi) C₁-C₁₂-Alkyl substituiert mit Heterocycloalkyl,
(xvii) C₁-C₁₂-Alkyl substituiert mit substituiertem Heterocycloalkyl,
(xviii) C₁-C₁₂-Alkyl substituiert mit C₃-C₈-Cycloalkyl,
(xix) C₁-C₁₂-Alkyl substituiert mit substituiertem C₃-C₈-Cycloalkyl,
(xx) Heteroaryl,
(xxi) substituiertes Heteroaryl,
(xxii) C₁-C₁₂-Alkyl substituiert mit Heteroaryl, und
(xxiii) C₁-C₁₂-Alkyl substituiert mit substituiertem Heteroaryl,
oder
R⁶ und R⁷ zusammengenommen mit dem Atom, an welches sie gebunden sind, bilden einen 3-10 gliedrigen Heterocycloalkylring, der substituiert sein kann mit einem oder mehreren Substituenten unabhängig gewählt aus der Gruppe bestehend aus
(i) Halogen,
(ii) Hydroxy,
(iii) C₁-C₃-Alkoxy,
(iv) C₁-C₃-Alkoxy-C₁-C₃-alkoxy,
(v) Oxo,
(vi) C₁-C₃-Alkyl,
(vii) Halo-C₁-C₃-alkyl,
und
(viii) C₁-C₃-Alkoxy-C₁-C₃-alkyl,
(j) -CO₂R³, worin R³ wie vorher definiert ist,
(k) -C(O)-NR⁴R⁵, worin R⁴ und R⁵ wie vorher definiert sind,
(l) =N-O-R³, worin R³ wie vorher definiert ist,
(m) C≡N,
(n) -O-S(O)ₙ-R³, worin n und R³ wie vorher definiert sind,
(o) Aryl,
(p) substituiertem Aryl,
(q) Heteroaryl,
(r) substituiertem Heteroaryl,
(s) C₃-C₈-Cycloalkyl,
(t) substituiertem C₃-C₈-Cycloalkyl,
(u) C₁-C₁₂-Alkyl substituiert mit Heteroaryl,
(v) Heterocycloalkyl,
(w) substituiertem Heterocycloalkyl,
(x) -NH-C(O)-R³, worin R³ wie vorher definiert ist,
(y) -NH-C(O)-NR⁴R⁵, worin R⁴ und R⁵ wie vorher definiert sind,
(z) =N-NR⁶R⁷, worin R⁶ und R⁷ wie vorher definiert sind,
(aa) =N-R³, worin R³ wie vorher definiert ist,
(bb) =N-NH-C(O)-R⁴, worin R⁴ wie vorher definiert ist, und
(cc) =N-NH-C(O)-NR⁴R⁵, worin R⁴ und R⁵ wie vorher definiert sind,
(4) C₃-Alkenyl substituiert mit einem Anteil gewählt aus der Gruppe bestehend aus
(a) Halogen,
(b) -CHO,
(c) -CO₂R³, worin R³ wie vorher definiert ist,
(d) -C(O)-R⁴, worin R⁴ wie vorher definiert ist,
(e) -C(O)-NR⁴R⁵, worin R⁴ und R⁵ wie vorher definiert sind,
(f) -C≡N,
(g) Aryl,
(h) substituiertem Aryl,
(i) Heteroaryl, .
(j) substituiertem Heteroaryl,
(k) C₃-C₇-Cycloalkyl,
und
(l) C₁-C₁₂-Alkyl substituiert mit Heteroaryl,
(5) C₉-C₁₀-Alkenyl,
(6) C₄-C₁₀-Alkenyl substituiert mit einem oder mehreren Substituenten gewählt aus der Gruppe bestehend aus
(a) Halogen,
(b) C₁-C₃-Alkoxy,
(c) Oxo,
(d) -CHO,
(e) -CO₂R³, worin R³ wie vorher definiert ist,
(f) -C(O)-NR⁴R⁵, worin R⁴ und R⁵ wie vorher definiert sind,
(g) -NR⁶R⁷, worin R⁶ und R⁷ wie vorher definiert sind,
(h) =N-O-R³, worin R³ wie vorher definiert ist,
(i) -C≡N,
(j) -O-S (O)ₙ-R³, worin n 0, 1 oder 2 ist und R³ ist wie vorher definiert,
(k) Aryl,
(l) substituiertem Aryl,
(m) Heteroaryl,
(n) substituiertem Heteroaryl,
(o) C₃-C₇-Cycloalkyl,
(p) C₁-C₁₂-Alkyl substituiert mit Heteroaryl,
(q) -NH-C(O)-R³, worin R³ wie vorher definiert ist,
(r) -NH-C(O)-NR⁴R⁵, worin R⁴ und R⁵ wie vorher definiert sind,
(s) =N-NR⁶R⁷, worin R⁶ und R⁷ wie vorher definiert sind,
(t) =N-R³, worin R³ wie vorher definiert ist,
(u) =N-NH-C(O)-R³, worin R³ wie vorher definiert ist,
und
(v) =N-NH-C(O)-NR⁴R⁵, worin R⁴ und R⁵ wie vorher definiert sind,
(7) C₃-C₁₀-Alkinyl,
und
(8) C₃-C₁₀-Alkinyl substituiert mit einem oder mehreren Substituenten gewählt aus der Gruppe bestehend aus
(a) Trialkylsilyl,
(b) Aryl,
(c) substituiertem Aryl,
(d) Heteroaryl,
und
(e) substituiertem Heteroaryl,
unter der Voraussetzung, daß, wenn R Allyl ist und R¹ Methyl ist, R² nicht H ist;
R^{p} ist Wasserstoff oder eine Hydroxyschutzgruppe;
R^{w} ist gewählt aus der Gruppe bestehend aus
(1) Wasserstoff,
(2) C₁-C₆-Alkyl, wahlweise substituiert mit einem oder mehreren Substituenten gewählt aus der Gruppe bestehend aus
(a) Aryl,
(b) substituiertem Aryl,
(c) Heteroaryl,
(d) substituiertem Heteroaryl,
(3) einer Gruppe gewählt aus Option (2) wie vorher definiert, weiter substituiert mit -CH₂-M-R⁸, worin M gewählt ist aus der Gruppe bestehend aus
(i) -O-,
(ii) -NH-,
(iii) -N(CH₃)-,
(iv) -S(O)ₙ-, worin n wie vorher beschrieben ist,
(v) -NH-C(O)-, und
(vi) -C(O)-NH-
und
R⁸ ist gewählt aus der Gruppe bestehend aus
(i) -(CH₂)ₙ-Aryl, worin n wie vorher beschrieben ist,
(ii) -(CH₂)ₙ-substituiertes Aryl, worin n wie vorher beschrieben ist,
(iii) -(CH₂)ₙ-Heteroaryl, worin n wie vorher beschrieben ist,
(iv) -(CH₂)ₙ-substituiertes Heteroaryl, worin n wie vorher beschrieben ist,
und
(v) -(CH₂)ₙ-Heterocycloalkyl, worin n wie vorher beschrieben ist, und
W ist abwesend oder gewählt aus der Gruppe bestehend aus -O-, -NH- und -N(CH₃)-.

2. Eine Verbindung gemäß Anspruch 1, die gewählt ist aus der Gruppe bestehend aus
Verbindung von Formel (I), R ist -CH₂CH=CH-(3-Chinolyl), R^{p} ist H, R¹ ist Methyl, R² ist Wasserstoff;
Verbindung von Formel (II), R ist -CH₂CH=CH-(3-Chinolyl), R^{p} ist Acetyl, R¹ ist H, R² ist CH₃, W ist abwesend, R^{w} ist H;
Verbindung von Formel (II), R ist -CH₂CH=CH-(3-Chinolyl), R^{p} ist H, W ist abwesend, R^{w} ist H, R¹ ist H, R² ist CH₃;
Verbindung von Formel (II), R ist -CH₂CH=CH-(3-Chinolyl), R^{p} ist H, W ist abwesend, R^{w} ist H, R¹ ist Acetyl, R² ist CH₃;
Verbindung von Formel (II), R ist -CH₂CH=CH-(3-Chinolyl), R^{p} ist H, W ist abwesend, R^{w} ist H, R¹ ist CH₂C(O)-O-CH₂CH₃, R² ist CH₃;
Verbindung von Formel (II), R ist -CH₂CH=CH-(3-Chinolyl), R^{p} ist H, W ist abwesend, R^{w} ist H, R¹ ist CH₂CH=CH₂, R² ist CH₃;
Verbindung von Formel (II), R ist -CH₂CH=CH-(3-Chinolyl), R^{p} ist H, W ist abwesend, R^{w} ist H, R¹ ist CH₂CH₂F, R² ist CH₃;
Verbindung von Formel (II), R ist -CH₂CH=CH-(3-Chinolyl), R^{p} ist H, W ist abwesend, R^{w} ist H, R¹ ist CH₂-Phenyl, R² ist CH₃;
Verbindung von Formel (II), R ist -CH₂CH=CH-(3-Chinolyl), R^{p} ist H, W ist abwesend, R^{w} ist H, R¹ ist CH₂-CN, R² ist CH₃;
Verbindung von Formel (II), R ist -CH₂CH=CH-(3-Chinolyl), R^{p} ist H, W ist abwesend, R^{w} ist H, R¹ ist CH₂-C=CH, R² ist CH₃;
Verbindung von Formel (II), R ist -CH₂CH=CH-(3-Chinolyl), R^{p} ist H, W ist abwesend, R^{w} ist H, R¹ ist CH₂CH₂CH₃, R² ist CH₃;
Verbindung von Formel (II), R ist -CH₂CH=CH-(3-Chinolyl), R^{p} ist H, W ist abwesend, R^{w} ist H, R¹ ist CH₂-Cyclopropyl, R² ist CH₃;
Verbindung von Formel (II), R ist -CH₂CH=CH-(3-Chinolyl), R^{p} ist H, W ist abwesend, R^{w} ist H, R¹ ist Cyclopropyl, R² ist CH₃;
Verbindung von Formel (II), R ist -CH₂CH=CH-(3-Chinolyl), R^{p} ist H, W ist abwesend, R^{w} ist H, R¹ ist CH₂-(3-Pyridyl), R² ist CH₃;
Verbindung von Formel (II), R ist -CH₂CH=CH-(3-Chinolyl), R^{p} ist H, W ist abwesend, R^{w} ist H, R¹ ist CH₂-Cyclo-C₃H₅, R² ist CH₃;
Verbindung von Formel (II), R ist -CH₂CH=CH-(3-Chinolyl), R^{p} ist H, W ist abwesend, R^{w} ist H, R¹ ist CH₂CH₂CH₃, R² ist CH₃;
Verbindung von Formel (II), R ist -CH₂CH=CH-(3-Chinolyl), R^{p} ist H, W ist abwesend, R^{w} ist H, R¹ ist CH₂CH=CHC₆H₅, R² ist CH₃;
Verbindung von Formel (II), R ist -CH₂CH=CH-(3-Chinolyl), R^{p} ist H, W ist abwesend, R^{w} ist H, R¹ ist CH₂C(=CH₂)C(O)OCH₃, R² ist CH₃;
Verbindung von Formel (II), R ist -CH₂CH=CH-(3-Chinolyl), R^{p} ist H, W ist abwesend, R^{w} ist H, R¹ ist CH₂C(=CH₂)CH₃, R² ist CH₃;
Verbindung von Formel (II), R ist -CH₂CH=CH-(3-Chinolyl), R^{p} ist H, W ist abwesend, R^{w} ist H, R¹ ist Cyclo-C₃H₅, R² ist CH₃;
Verbindung von Formel (II), R ist -CH₂CH=CH-(3-Chinolyl), R^{p} ist H, W ist abwesend, R^{w} ist H, R¹ ist CH₂-(3-Pyridyl), R² ist CH₃;
Verbindung von Formel (II), R ist -CH₂CH=CH-(3-Chinolyl), R^{p} ist H, W ist abwesend, R^{w} ist H, R¹ ist CH₂-(3-Hydroxyphenyl), R² ist CH₃;
Verbindung von Formel (II), R ist -CH₂CH=CH-(3-Chinolyl), R^{p} ist H, W ist abwesend, R^{w} ist H, R¹ ist CH₂-(2-Hydroxy-3-*tert*-butyl-5-methylphenyl), R² ist CH₃;
Verbindung von Formel (II), R ist -CH₂CH=CH-(3-Chinolyl), R^{p} ist H, W ist abwesend, R^{w} ist H, R¹ ist CH₂-(2-Hydroxy-3,4-dimethylphenyl), R² ist CH₃;
Verbindung von Formel (II), R ist -CH₂CH=CH-(3-Chinolyl), R^{p} ist H, W ist abwesend, R^{w} ist H, R¹ ist CH₂-(2-Hydroxy-3-methoxy-5-(2-propenyl)phenyl), R² ist CH₃;
Verbindung von Formel (II), R ist -CH₂CH=CH-(3-Chinolyl), R^{p} ist H, W ist abwesend, R^{w} ist H, R¹ ist CH₂-(2-Hydroxy-3-methoxy-5-methylphenyl), R² ist CH₃;
Verbindung von Formel (II), R ist -CH₂CH=CH-(3-Chinolyl), R^{p} ist H, W ist abwesend, R^{w} ist H, R¹ ist CH₂-(2-Hydroxy-5-cyclopentylphenyl), R² ist CH₃;
Verbindung von Formel (II), R ist -CH₂CH=CH-(3-Chinolyl), R^{p} ist H, W ist abwesend, R^{w} ist H, R¹ ist CH₂-(2-Hydroxy-5-carboxamidophenyl), R² ist CH₃;
Verbindung von Formel (II), R ist -CH₂CH=CH-(3-Chinolyl), R^{p} ist H, W ist abwesend, R^{w} ist H, R¹ ist CH₂-(2-Hydroxy-3-methoxy-5-(2-methoxycarbonylethyl)phenyl), R² ist CH₃;
Verbindung von Formel (II), R ist -CH₂CH=CH-(3-Chinolyl), R^{p} ist H, W ist abwesend, R^{w} ist H, R¹ ist CH₂-(2-Hydroxy-3-methyl-5-fluorphenyl), R² ist CH₃;
Verbindung von Formel (II), R ist -CH₂CH=CH-(3-Chinolyl), R^{p} ist H, W ist abwesend, R^{w} ist H, R¹ ist CH₂- (2-Hydroxy-3-methoxy-5-acetylphenyl), R² ist CH₃;
Verbindung von Formel (II), R ist -CH₂CH=CH-(3-Chinolyl), R^{p} ist H, W ist abwesend, R^{w} ist H, R¹ ist CH₂-(2-Hydroxy-3-promophenyl), R² ist CH₃;
Verbindung von Formel (II), R ist -CH₂CH=CH-(3-Chinolyl), R^{p} ist H, W ist abwesend, R^{w} ist H, R¹ ist CH₂-(2-Hydroxy-3-methoxy-5-alkoxycarbonylphenyl), R² ist CH₃;
Verbindung von Formel (II), R ist -CH₂CH=CH-(3-Chinolyl), R^{p} ist H, W ist abwesend, R^{w} ist H, R¹ ist CH₂-(2-Hydroxy-3-ethylphenyl) , R² ist CH₃;
Verbindung von Formel (II), R ist -CH₂CH=CH-(3-Chinolyl), R^{p} ist H, W ist abwesend, R^{w} ist H, R¹ ist CH₂-(2-Hydroxy-5-isobutylphenyl), R² ist CH₃;
Verbindung von Formel (II), R ist -CH₂CH=CH-(3-Chinolyl), R^{p} ist H, W ist abwesend, R^{w} ist H, R¹ ist CH₂-(2-Hydroxy-3-methyl-5-diethylamino-6-methylphenyl), R² ist CH₃;
Verbindung von Formel (II), R ist -CH₂CH=CH-(3-Chinolyl), R^{p} ist H, W ist abwesend, R^{w} ist H, R¹ ist CH₂-(2-Hydroxy-4-methyl-5-brom-6-methylphenyl), R² ist CH₃; und
Verbindung von Formel (II), R ist -CH₂CH=CH-(3-Chinolyl), R^{p} ist H, W ist abwesend, R^{w} ist H, R¹ ist CH₂-(2-Hydroxy-3-hydroxymethylphenyl), R² ist CH₃.

3. Eine pharmazeutische Zusammensetzung zur Behandlung bakterieller Infektionen, die-eine therapeutisch wirksame Menge einer Verbindung von Anspruch 1 oder ein pharmazeutisch verträgliches Salz oder Ester davon in Kombination mit einem pharmazeutisch verträglichen Träger umfaßt.

4. Verwendung einer Verbindung gemäß Anspruch 1 oder eines pharmazeutisch verträglichen Salzes oder Esters davon, zur Herstellung eines Medikaments zur Behandlung bakterieller Infektionen durch Verabreichen einer pharmazeutischen Zusammensetzung, die eine therapeutisch wirksame Menge der Verbindung oder ein pharmazeutisch verträgliches Salz oder Ester davon enthält, an ein Säugetier, das eine solche Behandlung benötigt.

5. Eine Verbindung gemäß Anspruch 1, mit der Formel (I)

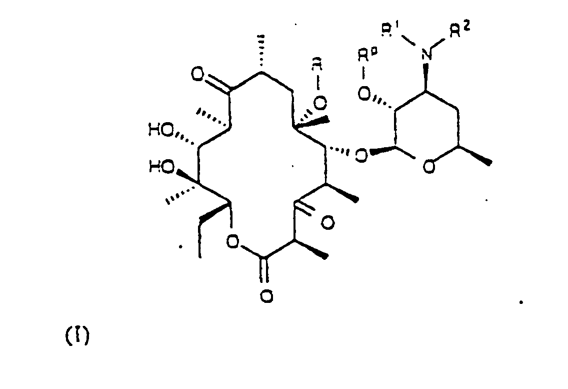

6. Eine Verbindung gemäß Anspruch 1 mit der Formel (II)

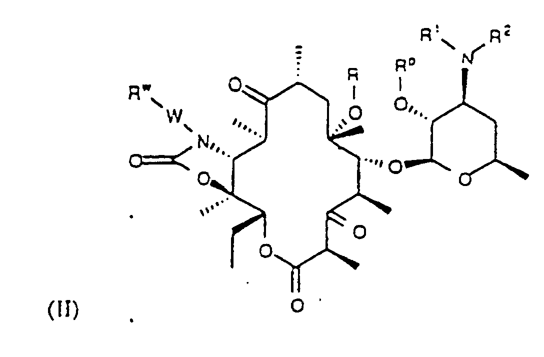

7. Eine Verbindung gemäß Anspruch 1 mit der Formel (III)

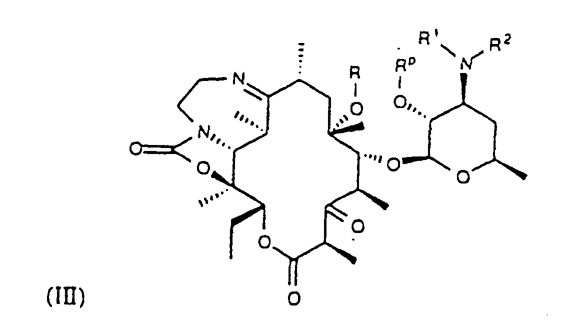

8. Eine Verbindung gemäß Anspruch 1, mit der Formel (IV)

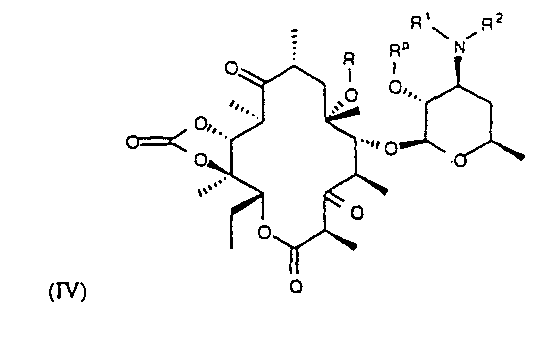

9. Eine Verbindung gemäß Anspruch 1, mit der Formel (V)

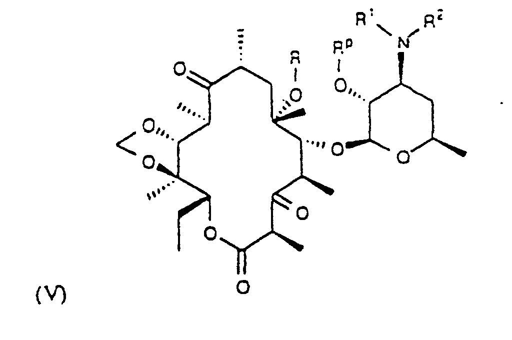

10. Ein Verfahren zur Herstellung einer Verbindung gewählt aus der Gruppe bestehend aus 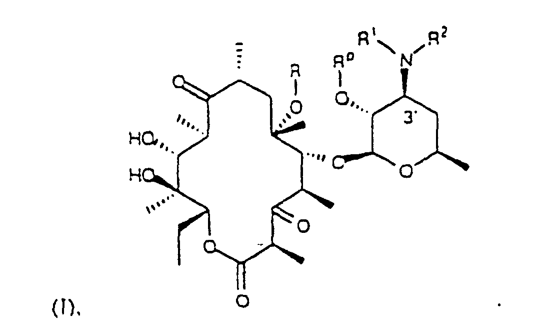 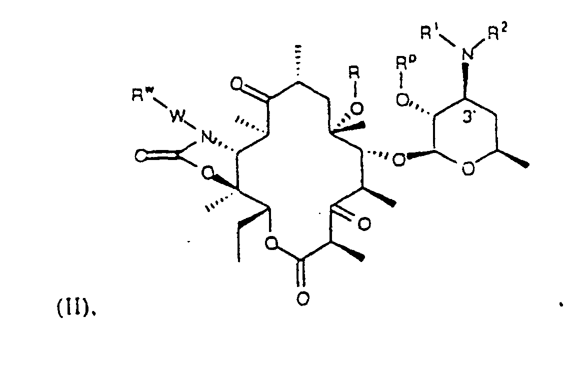 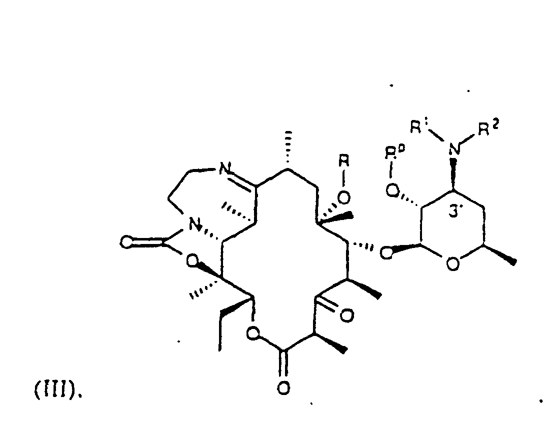 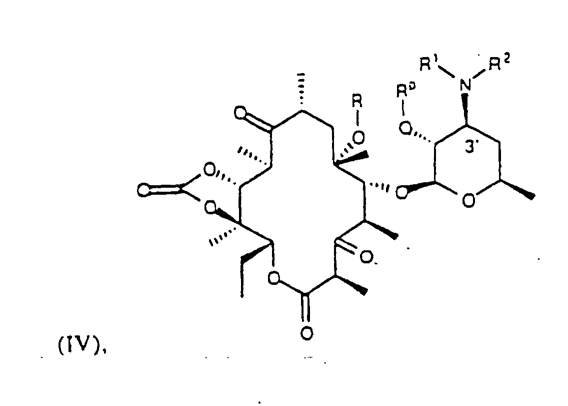 und 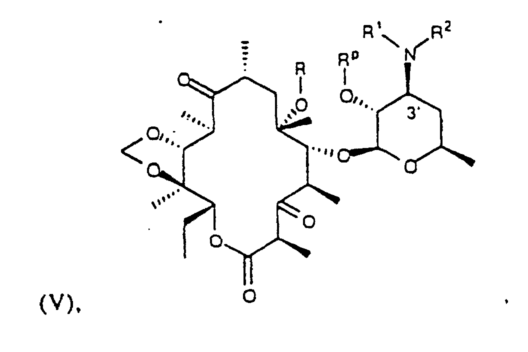 worin
R¹ und R², unter der Voraussetzung, daß R¹ und R² nicht beide Methyl sind, unabhängig gewählt sind aus der Gruppe bestehend aus
(1) Wasserstoff,
(2) C₁-C₆-Alkyl, wahlweise substituiert mit einem Substituenten gewählt aus der Gruppe bestehend aus
(a) Halogen,
(b) C₃-C₆-Cycloalkyl,
(c) Aryl,
(d) substituiertes Aryl,
(e) Heteroaryl,
(f) substituiertes Heteroaryl,
(g) -CHO,
(h) -C(O)-C₁-C₆-Alkyl, und
(i) -C(O)-NR'R", worin R' und R'' unabhängig gewählt sind aus der Gruppe bestehend aus Wasserstoff, C₁-C₃-Alkyl, C₁-C₃-Alkyl substituiert mit Aryl, substituiertem Aryl, Heteroaryl und substituiertem Heteroaryl;
(3) C₂-C₆-Alkyl, wahlweise substituiert mit einem Substituenten gewählt aus der Gruppe bestehend aus
(a) C₁-C₆-Alkoxy,
(b) -NR'R'', worin R' und R'' wie vorher definiert sind,
(c) -NH-C (O) -C₁-C₆-Alkyl,
(d) -NH-C (O) -O-C₁-C₆-Alkyl,
(e) -O-C (O)-O-C₁-C₆-Alkyl,
(f) -O-C(O)-C₁-C₆-Alkyl,
(g) -CH (=N-O-C₁-C₆-Alkyl),
(h) -C (=N-O-C₁-C₆-Alkyl) -C₁-C₆-alkyl,
(i) -CH (=N-NH-C₁-C₆-Alkyl), und
(j) -C (=N-NH-C₁-C₆-Alkyl) -C₁-C₆-alkyl,
(4) C₃-C₆-Alkenyl wahlweise substituiert mit einem Substituenten gewählt aus der Gruppe bestehend aus
(a) Halogen,
(b) C₃-C₆-Cycloalkyl,
(c) Aryl,
(d) substituiertem Aryl,
(e) Heteroaryl,
(f) substituiertem Heteroaryl,
(g) -NH-C (O) -C₁-C₆-Alkyl,
(h) -NH-C(O) -O-C₁-C₆-Alkyl,
(i) -O-C (O)-O-C₁-C₆-Alkyl,
(j) -O-C (O)-C₁-C₆-Alkyl,
(k) -CHO,
(l) -C (O)-C₁-C₆-Alkyl,
(m) -C(O)-NR'R'', worin R' und R'' wie vorher definiert sind,
(n) -CH ( =N-O-C₁-C₆-Alkyl) ,
(o) -C (=N-O-C₁-C₆-Alkyl) -C₁-C₆-alkyl,
(p) -CH (=N-NH-C₁-C₆-alkyl) ,
(q) -C(=N-NH-C₁-C₆-Alkyl)-C₁-C₆-alkyl, und
(r) -C ( O ) -O-C₁-C₆-Alkyl .
(5) C₃-C₆-Alkinyl wahlweise substituiert mit einem Substituenten gewählt aus der Gruppe bestehend aus
(a) Halogen,
(b) C₃-C₆-Cycloalkyl,
(c) Aryl,
(d) substituiertes Aryl,
(e) Heteroaryl, und
(f) substituiertes Heteroaryl,
(6) C₃-C₆-Cycloalkyl,
(7) -CHO,
(8) -C (O) -C₁-C₆-Alkyl,
(9) -C(O)-NR'R", worin R' und R" wie vorher definiert sind, und
(10) -C(O)-O-C₁-C₆-Alkyl,
oder R¹ und R² zusammengenommen können -(CH₂)ₚ- sein, worin p 3 bis 7 ist, welche zusammengenommen mit dem Stickstoffatom, an welches sie gebunden sind, folglich einen heterozyklischen Ring bilden, der ein Stickstoffatom und von 3 bis 7 Kohlenstoffatome enthält;
R ist gewählt aus der Gruppe bestehend aus
(1) Methyl substituiert mit einem Substituenten gewählt aus der Gruppe bestehend aus
(a) -CN,
(b) -F,
(c) -CO₂R³, worin R³ C₁-C₃-Alkyl, Aryl-substituiertes C₁-C₃-Alkyl oder Heteroaryl-substituiertes C₁-C₃-Alkyl ist,
(d) -S(O)ₙ-R₃, worin n 0, 1 oder 2 ist und R³ ist wie vorher definiert,
(e) -NH-C(O)-R³, worin R³ wie vorher definiert ist,
(f) -NH-C(O)-NR⁴R⁵, worin R⁴ und R⁵ unabhängig gewählt sind aus der Gruppe bestehend aus
(i) Wasserstoff,
(ii) C₁-C₃-Alkyl,
(iii) C₁-C₃-Alkyl substituiert mit Aryl,
(iv) C₁-C₃-Alkyl substituiert mit substituiertem Aryl,
(v) C₁-C₃-Alkyl substituiert mit Heteroaryl, und
(vi) C₁-C₃-Alkyl substituiert mit substituiertem Heteroaryl,
(g) Aryl,
(h) substituiertem Aryl,
(i) Heteroaryl,
und
(j) substituiertem Heteroaryl,
(2) C₂-C₁₀-Alkyl,
(3) C₂-C₁₀-Alkyl substituiert mit einem oder mehreren Substituenten gewählt aus der Gruppe bestehend aus
(a) Halogen,
(b) Hydroxy,
(c) C₁-C₃-Alkoxy,
(d) C₁-C₃-Alkoxy-C₁-C₃-alkoxy,
(e) Oxo,
(f) -N₃,
(g) -CHO,
(h) -O-SO₂- (substituiertes C₁-C₆-Alkyl) ,
(i) -NR⁶R⁷, worin R⁶ und R⁷ gewählt sind aus der Gruppe bestehend aus
(i) Wasserstoff,
(ii) C₁-C₁₂-Alkyl,
(iii) substituiertem C₁-C₁₂-Alkyl,
(iv) C₁-C₁₂-Alkenyl,
(v) substituiertem C₁-C₁₂-Alkenyl,
(vi) C₁-C₁₂-Alkinyl,
(vii) substituiertem C₁-C₁₂-Alkinyl,
(viii) Aryl,
(ix) C₃-C₈-Cycloalkyl,
(x) substituiertem C₃-C₈-Cycloalkyl,
(xi) substituiertem Aryl,
(xii) Heterocycloalkyl,
(xiii) substituiertem Heterocycloalkyl,
(xiv) C₁-C₁₂-Alkyl substituiert mit Aryl,
(xv) C₁-C₁₂-Alkyl substituiert mit substituiertem Aryl,
(xvi) C₁-C₁₂-Alkyl substituiert mit Heterocycloalkyl,
(xvii) C₁-C₁₂-Alkyl substituiert mit substituiertem Heterocycloalkyl,
(xviii) C₁-C₁₂-Alkyl substituiert mit C₃-C₈-Cycloalkyl,
(xix) C₁-C₁₂-Alkyl substituiert mit substituiertem C₃-C₈-Cycloalkyl,
(xx) Heteroaryl,
(xxi) substituiertem Heteroaryl,
(xxii) C₁-C₁₂-Alkyl substituiert mit Heteroaryl, und
(xxiii) C₁-C₁₂-Alkyl substituiert mit substituiertem Heteroaryl,
oder
R⁶ und R⁷ zusammengenommen mit dem Atom,
an welches sie gebunden sind, bilden einen 3-10 gliedrigen Heterocycloalkylring, der substituiert sein kann mit einem oder mehreren Substituenten unabhängig gewählt aus der Gruppe bestehend aus
(i) Halogen,
(ii) Hydroxy,
(iii) C₁-C₃-Alkoxy,
(iv) C₁-C₃-Alkoxy-C₁-C₃-alkoxy,
(v) Oxo,
(vi) C₁-C₃-Alkyl,
(vii) Halo-C₁-C₃-alkyl,
und
(vii) C₁-C₃-Alkoxy-C₁-C₃-alkyl,
(j) -CO₂R³, worin R³ wie vorher definiert ist,
(k) -C(O) -NR⁴R⁵, worin R⁴ und R⁵ wie vorher definiert sind,
(l) =N-O-R³, worin R³ wie vorher definiert ist,
(m) C=N,
(n) -O-S(O)ₙ-R³, worin n und R³ wie vorher definiert sind,
(o) Aryl,
(p) substituiertem Aryl,
(q) Heteroaryl,
(r) substituiertem Heteroaryl,
(s) C₃-C₈-Cycloalkyl,
(t) substituiertem C₃-C₈-Cycloalkyl,
(u) C₁-C₁₂-Alkyl substituiert mit Heteroaryl,
(v) Heterocycloalkyl,
(w) substituiertem Heterocycloalkyl,
(x) -NH-C(O)-R³, worin R³ wie vorher definiert ist,
(y) -NH-C(O)-NR⁴R⁵, worin R⁴ und R⁵ wie vorher definiert sind,
(z) =N-NR⁶R⁷, worin R⁶ und R⁷ wie vorher definiert sind,
(aa) =N-R³, worin R³ wie vorher definiert ist,
(bb) =N-NH-C(O)-R⁴, worin R⁴ wie vorher definiert ist,
und
(cc) =N-NH-C(O)-NR⁴R⁵, worin R⁴ und R⁵ wie vorher definiert sind,
(4) C₃-Alkenyl substituiert mit einem Anteil gewählt aus der Gruppe bestehend aus
(a) Halogen,
(b) -CHO,
(c) -CO₂R³, worin R³ wie vorher definiert ist,
(d) -C (O) -R⁴, worin R⁴ wie vorher definiert ist,
(e) -C (O) -NR⁴R⁵, worin R⁴ und R⁵ wie vorher definiert sind,
(f) -C≡N,
(g) Aryl,
(h) substituiertem Aryl,
(i) Heteroaryl,
(j) substituiertem Heteroaryl,
(k) C₃-C₇-Cycloalkyl,
und
(l) C₁-C₁₂-Alkyl substituiert mit Heteroaryl,
(5) C₄-C₁₀-Alkenyl,
(6) C₄-C₁₀-Alkenyl substituiert mit einem oder mehreren Substituenten gewählt aus der Gruppe bestehend aus
(a) Halogen,
(b) C₁-C₃-Alkoxy,
(c) Oxo,
(d) -CHO,
(e) -CO₂R³, worin R³ wie vorher definiert ist,
(f) -C(O)-NR⁴R-⁵, worin R⁴ und R⁵ wie vorher definiert sind,
(g) -NR⁶R⁷, worin R⁶ und R⁷ wie vorher definiert sind,
(h) =N-O-R³, worin R³ wie vorher definiert ist,
(i) -C≡N,
(j) -O-S(O)ₙ-R³, worin n 0, 1 oder 2 ist und R³ ist wie vorher definiert,
(k) Aryl,
(l) substituiertem Aryl,
(m) Heteroaryl,
(n) substituiertem Heteroaryl,
(o) C₃-C₇-Cycloalkyl,
(p) C₁-C₁₂-Alkyl substituiert mit Heteroaryl,
(q) -NH-C(O)-R³, worin R³ wie vorher definiert ist,
(r) -NH-C(O)-NR⁴R⁵, worin R⁴ und R⁵ wie vorher definiert sind,
(s) =N-NR⁶R⁷, worin R⁶ und R⁷ wie vorher definiert sind,
(t) =N-R³, worin R³ wie vorher definiert ist,
(u) =N-NH-C(O)-R³, worin R³ wie vorher definiert ist, und
(v) =N-NH-C(O)-NR⁴R⁵, worin R⁴ und R⁵ wie vorher definiert sind,
(7) C₃-C₁₀-Alkinyl,
und
(8) C₃-C₁₀-Alkinyl substituiert mit einem oder mehreren Substituenten gewählt aus der Gruppe bestehend aus
(a) Trialkylsilyl,
(b) Aryl,
(c) substituiertem Aryl,
(d) Heteroaryl,
und
(e) substituiertem Heteroaryl,
unter der Voraussetzung, daß, wenn R Allyl ist und R¹ Methyl ist, R² nicht H ist;
R^{p} ist Wasserstoff oder eine Hydroxyschutzgruppe;
R^{w} ist gewählt aus der Gruppe bestehend aus
(1) Wasserstoff,
(2) C₁-C₆-Alkyl, wahlweise substituiert mit einem oder mehreren
Substituenten gewählt aus der Gruppe bestehend aus
(a) Aryl,
(b) substituiertem Aryl,
(c) Heteroaryl,
(d) substituiertem Heteroaryl,
(3) eine Gruppe gewählt aus Option (2) wie vorher definiert, weiter substituiert mit -CH₂-M-R⁸, worin M gewählt ist aus der Gruppe bestehend aus
(i) -O-,
(ii) -NH-,
(ii) -N(CH₃)-,
(iv) -S(O)ₙ-, worin n wie vorher beschrieben ist,
(v) -NH-C(O)-, und
(vi) -C(O)-NH-
und
R⁸ ist gewählt aus der Gruppe bestehend aus
(i) -(CH₂)ₙ-Aryl, worin n wie vorher beschrieben ist,
(ii) -(CH₂)ₙ-substituiertes Aryl, worin n wie vorher beschrieben ist,
(iii) -(CH₂)ₙ-Heteroaryl, worin n wie vorher beschrieben ist,
(iv) -(CH₂)ₙ-substituiertes Heteroaryl, worin n wie vorher beschrieben ist,
und
(v) -(CH₂)ₙ-Heterocycloalkyl, worin n wie vorher beschrieben ist,
und
W ist abwesend oder gewählt aus der Gruppe bestehend aus -O-, -NH- und -N(CH₃)-, wobei das Verfahren folgendes umfaßt:
(a) sequentielle Desmethylierung des 3'-Stickstoff einer Verbindung, die gewählt ist aus der Gruppe bestehend aus 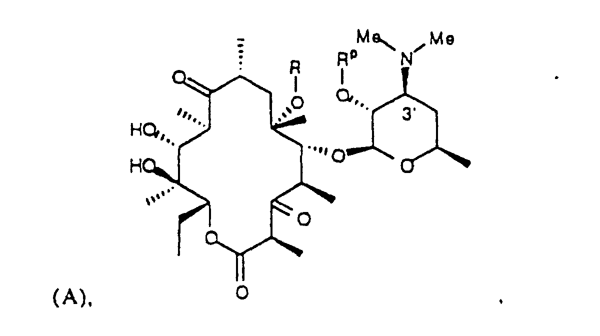 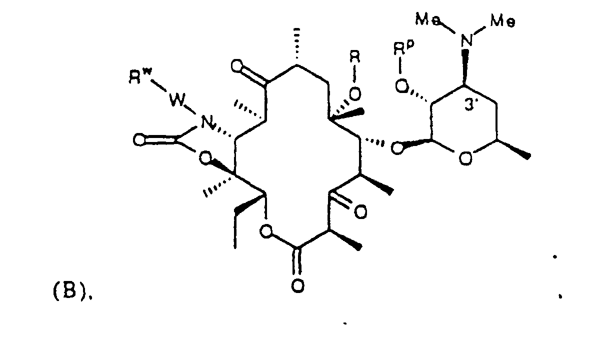 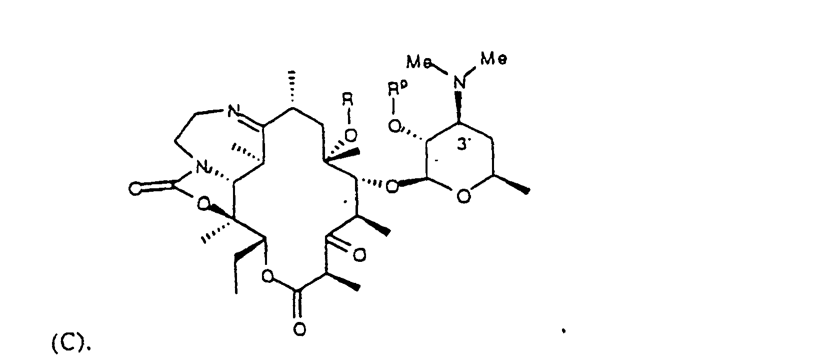 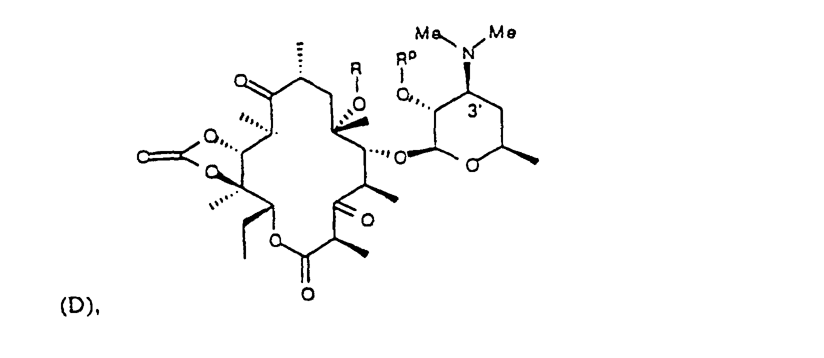 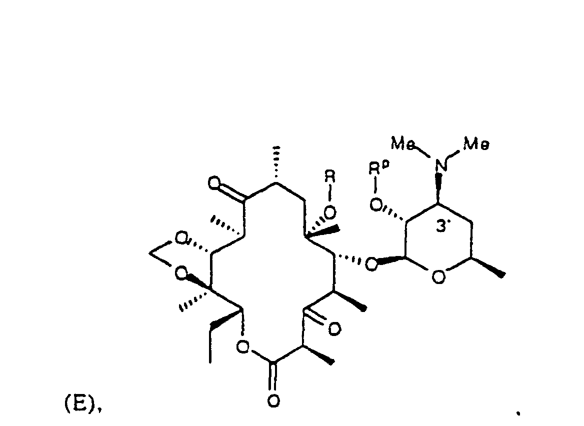 und
worin R und R^{p} wie vorher definiert sind; und
(b) sequentielles Reagieren der Verbindung aus. Schritt (a) mit einer R¹- und einer R² -Vorläuferverbindung.

11. Das Verfahren gemäß Anspruch 10, worin die Desmethylierung des 3'-Stickstoffs erhalten wurde durch Reagieren der Verbindung mit N-Jodsuccinimid, um eine entsprechende Verbindung zu liefern, die eine 3'-NHCH₃ Gruppe hat.

12. Das Verfahren gemäß Anspruch 11, worin in Schritt (b) die Verbindung mit einem R¹-Vorläufer reagiert wird, gewählt aus der Gruppe bestehend aus
(i) R¹-X, worin R¹ wie vorher definiert ist und X ist eine Halid- oder Sulfonatabgangsgruppe.
(ii) einem Aldehyd von Formel R*-CHO, gefolgt von Reduktion, um R*-CH₂ zu ergeben, wobei der R¹ Anteil zuvor beschrieben ist,
(iii) Carbonyldiimidazol, um eine Intermediatverbindung zu ergeben,
worin R¹ Imidazolylcarbonyl ist, und Reagieren dieses Intermediates mit einem Amin, das die Formel HNR'R'' hat, worin R' und R'' wie vorher definiert sind, um eine Verbindung zu ergeben, worin R¹ C(O)-NR'R" ist.
(iv) einem Alkohol der Formel HOR', worin R' wie vorher definiert ist, um eine Verbindung zu ergeben, worin R¹ C(O)-OR' ist.
(v) einem Acylierungsmittel der Formel X-C(O)-R', worin X Halogen ist und R' ist wie vorher definiert, oder O-(C(O)-R')₂, um eine Verbindung zu ergeben, worin R¹ C(O)R' ist, und
(vi) einem substituierten oder unsubstituierten Arylalkohol
und einem Homologisierungsmittel gewählt aus Formaldehyd oder Paraformaldehyd, um eine Verbindung zu ergeben, worin R¹ Methyl substituiert mit substituiertem Aryl ist.

13. Das Verfahren gemäß Anspruch 11, das weiter das Behandeln der Verbindung mit Jodsuccinimid oder Jod in der Anwesenheit von Licht umfaßt, um eine entsprechende Verbindung zu liefern, die eine 3'-NH₂ Gruppe hat.

14. Das Verfahren gemäß Anspruch 13, das weiter das Behandeln der Verbindung mit einem R¹-Vorläufer umfaßt, um eine Verbindung zu liefern, die eine 3'-NHR¹CH₃ Gruppe hat, worin der R¹-Vorläufer gewählt ist aus der Gruppe bestehend aus
(i) R¹-X, worin R¹ wie vorher definiert ist, und X ist eine Halid- oder Sulfonatabgangsgruppe,
(ii) einem Aldehyd von Formel R*-CHO, gefolgt von Reduktion, um R*-CH₂ zu ergeben, wobei der R¹ Anteil vorher beschrieben ist,
(iii) Carbonyldiimidazol, um eine Intermediatverbindung zu ergeben,
worin R¹ Imidazolylcarbonyl ist, und Reagieren dieses Intermediats mit einem Amin, das die Formel HNR'R'' hat, worin R' und R'' wie vorher definiert sind, um eine Verbindung zu ergeben, worin R¹ C(O)-NR'R'' ist.
(iv) einem Alkohol der Formel HOR', worin R' wie vorher definiert ist, um eine Verbindung zu ergeben, worin R¹ C(O)-OR' ist.
(v) einem Alkylierungsmittel der Formel X-C(O)-R', worin X Halogen ist und R' ist wie vorher definiert, oder O-(C(O)-R')₂, um eine Verbindung zu ergeben, worin R¹ C(O)R' ist, und
(vi) einem substituierten oder unsubstituierten Arylalkohol und einem Homologisierungsmittel gewählt aus Formaldehyd oder Paraformaldehyd, um eine Verbindung zu ergeben, worin R¹ Methyl substituiert ist mit substituiertem Aryl.

15. Das Verfahren gemäß Anspruch 14, das weiter das Behandeln der Verbindung mit einer R²-Vorläufer-Verbindung umfaßt, um eine Verbindung zu liefern, die eine 3'-NHR¹R² Gruppe hat, worin der R²-Vorläufer gewählt ist aus der Gruppe bestehend aus
(i) R²-X, worin R² wie zuvor definiert ist, und X ist eine Halid- oder Sulfonatabgangsgruppe,
(ii) einem Aldehyd von Formel R*-CHO, gefolgt von Reduktion, um R*-CH₂- zu ergeben, wobei der R² Anteil vorher beschrieben ist,
(iii) Carbonyldiimidazol, um eine Intermediatverbindung zu ergeben,
worin R² Imidazolylcarbonyl ist und Reagieren dieses Intermediates mit einem Amin, das die Formel HNR'R'' hat, worin R' und R'' wie vorher definiert sind, um eine Verbindung zu ergeben, worin R² C(O)-NR'R'' ist.
(iv) einem Alkohol der Formel HOR', um eine Verbindung zu ergeben, worin R² C(O)-OR' ist.
(v) einem Acylierungsmittel der Formel X-C(O)-R', worin X Halogen ist und R' ist wie vorher definiert, oder O-(C(O)-R')_{2'} um eine Verbindung zu ergeben, worin R² C(O)R' ist, und
(vi) einem substituierten oder unsubstituierten Arylalkohol und einem Homologisierungsmittel gewählt aus Formaldehyd oder Paraformaldehyd, um eine Verbindung zu ergeben, worin R¹ Methyl substituiert mit substituiertem Aryl ist.

## Revendications

1. Composé choisi dans le groupe constitué par 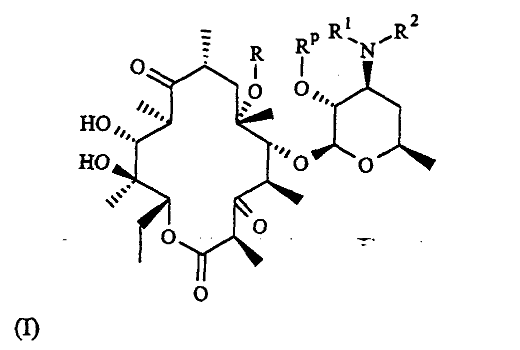 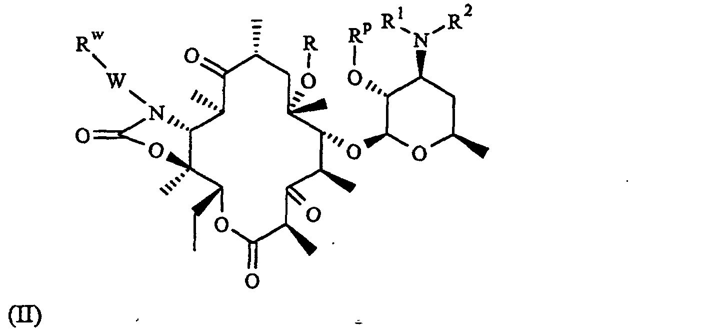 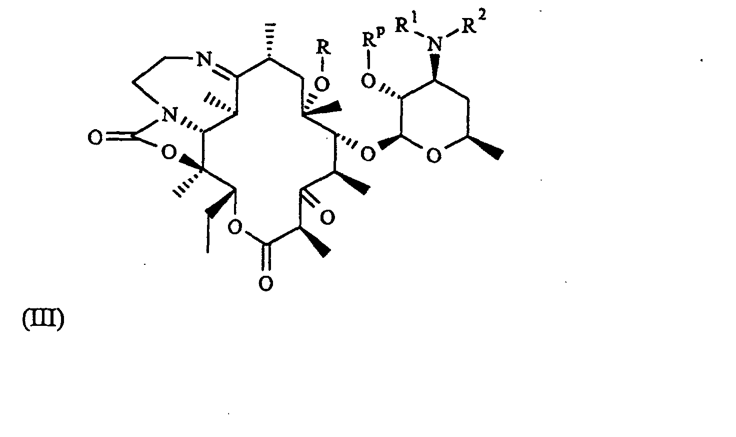 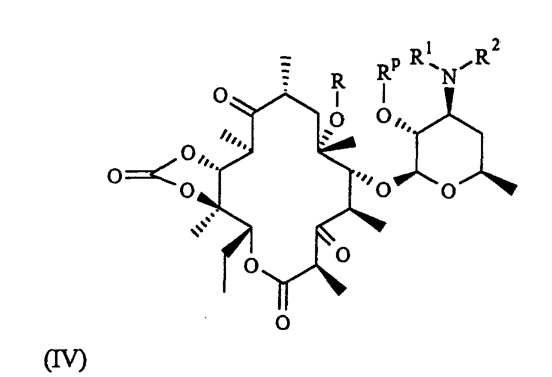 et 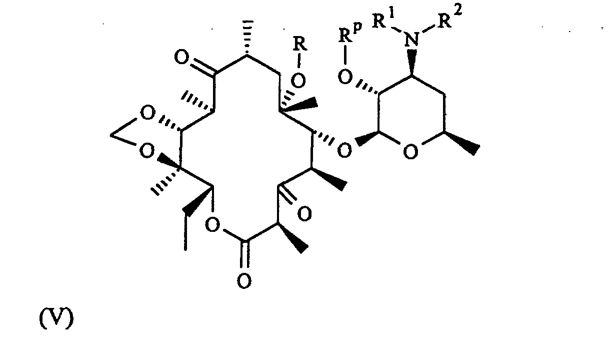 ou un de ses sels, esters ou promédicaments pharmaceutiquement acceptables, où
R¹ et R², à condition que R¹ et R² ne soient pas tous les deux un groupe méthyle, sont choisis indépendamment dans le groupe constitué par
(1) un atome d'hydrogène,
(2) un groupe alkyle en C₁-C₆ éventuellement substitué par un substituant choisi dans le groupe constitué par
(a) un atome d'halogène,
(b) un groupe cycloalkyle en C₃-C₆,
(c) aryle,
(d) aryle substitué,
(e) hétéroaryle,
(f) hétéroaryle substitué,
(g) -CHO,
(h) -C(O)-(alkyle en C₁-C₆) et
(i) -C(O)-NR'R", dans lequel R' et R" sont choisis indépendamment dans le groupe constitué par un atome d'hydrogène, un groupe alkyle en C₁-C₃, alkyle en C₁-C₃ substitué par un groupe aryle, aryle substitué, hétéroaryle et hétéroaryle substitué,
(3) un groupe alkyle en C₂-C₆ éventuellement substitué par un substituant choisi dans le groupe constitué par un groupe
(a) alcoxy en C₁-C₆,
(b) -NR'R", dans lequel R' et R" sont tels que définis précédemment,
(c) -NH-C(O)-(alkyle en C₁-C₆),
(d) -NH-C(O)-O-(alkyle en C₁-C₆),
(e) -O-C(O)-O-(alkyle en C₁-C₆),
(f) -O-C(O)-(alkyle en C₁-C₆),
(g) -CH(=N-O-(alkyle en C₁-C₆)),
(h) -C(=N-O-(alkyle en C₁-C₆))-(alkyle en C₁-C₆),
(i) -CH(=N-NH-(alkyle en C₁-C₆)) et
(j) -C(=N-NH-(alkyle en C₁-C₆)-(alkyle en C₁-C₆),
(4) un groupe alcényle en C₃-C₆ éventuellement substitué par un substituant choisi dans le groupe constitué par
(a) un atome d'halogène,
(b) un groupe cycloalkyle en C₃-C₆,
(c) aryle,
(d) aryle substitué,
(e) hétéroaryle,
(f) hétéroaryle substitué,
(g) -NH-C(O)-(alkyle en C₁-C₆),
(h) -NH-C(O)-O-(alkyle en C₁-C₆),
(i) -O-C(O)-O-(alkyle en C₁-C₆,
(j) -O-C(O)-(alkyle en C₁-C₆),
(k) -CHO,
(l) -C(O)-(alkyle en C₁-C₆),
(m) -C(O)-NR'R", dans lequel R' et R" sont tels que définis précédemment,
(n) -CH(=N-O-(alkyle en C₁-C₆)),
(o) -C(=N-O-(alkyle en C₁-C₆))-(alkyle en C₁-C₆),
(p) -CH(=N-NH-(alkyle en C₁-C₆)),
(q) -C(=N-NH-(alkyle en C₁-C₆)-(alkyle en C₁-C₆) et
(r) -C(O)-O-(alkyle en C₁-C₆),
(5) un groupe alcynyle en C₃-C₆ éventuellement substitué par un substituant choisi dans le groupe constitué par
(a) un atome d'halogène,
(b) un groupe cycloalkyle en C₃-C₆,
(c) aryle,
(d) aryle substitué,
(e) hétéroaryle et
(f) hétéroaryle substitué,
(6) un groupe cycloalkyle en C₃-C₆,
(7) un groupe -CHO,
(8) un groupe -C(O)-(alkyle en C₁-C₆),
(9) un groupe -C(O)-NR'R", dans lequel R' et R" sont tels que définis précédemment et
(10) un groupe -C(O)-O-(alkyle en C₁-C₆)
ou R¹ et R² pris ensemble peuvent être un groupe -(CH₂)ₚ- , dans lequel p est 3 à 7, qui pris ensemble avec l'atome d'azote auquel ils sont liés, forme ainsi un noyau hétérocyclique contenant un atome d'azote et 3 à 7 atomes de carbone ;
R est choisi dans le groupe constitué par
(1) un groupe méthyle substitué par un substituant choisi dans le groupe constitué par un groupe
(a) -CN,
(b) -F,
(c) *-*CO₂R³ dans lequel R³ est un groupe alkyle en C₁-C₃, alkyle en C₁-C₃ substitué par un groupe aryle ou alkyle en C₁-C₃ substitué par un groupe hétéroaryle,
(d) -S(O)ₙ-R³ dans lequel n est égal à 0, 1 ou 2 et R³ est tel que défini précédemment,
(e) -NH-C(O)-R³ où R³ est tel que défini précédemment,
(f) -NH-C(O)-NR⁴R⁵ dans lequel R⁴ et R⁵ sont choisis indépendamment dans le groupe constitué par
(i) un atome d'hydrogène,
(ii) un groupe alkyle en C₁-C₃,
(iii) alkyle en C₁-C₃ substitué par un groupe aryle,
(iv) alkyle en C₁-C₃ substitué par un groupe aryle substitué,
(v) alkyle en C₁-C₃ substitué par un groupe hétéroaryle et
(vi) alkyle en C₁-C₃ substitué par un groupe hétéroaryle substitué,
(g) aryle,
(h) aryle substitué,
(i) hétéroaryle,
et
(j) hétéroaryle substitué,
(2) un groupe alkyle en C₂-C₁₀,
(3) un groupe alkyle en C₂-C₁₀ substitué par un ou plusieurs substituants choisis dans le groupe constitué par
(a) un atome d'halogène,
(b) un groupe hydroxy,
(c) alcoxy en C₁-C₃,
(d) (alcoxy en C₁-C₃)-(alcoxy en C₁-C₃),
(e) oxo,
(f)-N₃,
(g) -CHO,
(h) -O-SO₂-(alkyle en C₁-C₆ substitué),
(i) -NR⁶R⁷ dans lequel R⁶ et R⁷ sont choisis dans le groupe constitué par
(i) un atome d'hydrogène,
(ii) un groupe alkyle en C₁-C₁₂,
(iii) alkyle en C₁-C₁₂ substitué,
(iv) alcényle en C₁-C₁₂,
(v) alcényle en C₁-C₁₂ substitué,
(vi) alcynyle en C₁-C₁₂,
(vii) alcynyle en C₁-C₁₂ substitué,
(viii) aryle,
(ix) cycloalkyle en C₃-C₈,
(x) cycloalkyle en C₃-C₈ substitué,
(xi) aryle substitué,
(xii) hétérocycloalkyle,
(xiii) hétérocycloalkyle substitué,
(xiv) alkyle en C₁-C₁₂ substitué par un groupe aryle,
(xv) alkyle en C₁-C₁₂ substitué par un groupe aryle substitué,
(xvi) alkyle en C₁-C₁₂ substitué par un groupe hétérocycloalkyle,
(xvii) alkyle en C₁-C₁₂ substitué par un groupe hétérocycloalkyle substitué,
(xviii) alkyle en C₁-C₁₂ substitué par un groupe cycloalkyle en C₃-C₈,
(xix) alkyle en C₁-C₁₂ substitué par un groupe cycloalkyle en C₃-C₈ substitué,
(xx) hétéroaryle,
(xxi) hétéroaryle substitué,
(xxii) alkyle en C₁-C₁₂ substitué par un groupe hétéroaryle,
et
(xxiii) alkyle en C₁-C₁₂ substitué par un groupe hétéroaryle substitué
ou
R⁶ et R⁷ pris ensemble avec l'atome auquel ils sont liés forment un noyau hétérocycloalkyle à 3 à 10 chaînons qui peut être substitué par un ou plusieurs substituants choisis indépendamment dans le groupe constitué par
(i) un atome d'halogène,
(ii) un groupe hydroxy,
(iii) alcoxy en C₁-C₃,
(iv) (alcoxy en C₁-C₃)-(alcoxy en C₁-C₃),
(v) oxo,
(vi) alkyle en C₁-C₃,
(vii) halogénoalkyle en C₁-C₃
et
(viii) (alcoxy en C₁-C₃)-(alkyle en C₁-C₃),
(j) -CO₂R³ dans lequel R³ est tel que défini précédemment,
(k) -C(O)-NR⁴R⁵ dans lequel R⁴ et R⁵ sont tels que définis précédemment,
(l) N-O-R³ dans lequel R³ est tel que défini précédemment,
(m) -C≡N,
(n) -O-S(O)ₙ-R³ dans lequel n et R³ sont tels que définis précédemment,
(o) aryle,
(p) aryle substitué,
(q) hétéroaryle,
(r) hétéroaryle substitué,
(s) cycloalkyle en C₃-C₈,
(t) cycloalkyle en C₃ C₈ substitué,
(u) alkyle en C₁-C₁₂ substitué par un groupe hétéroaryle,
(v) hétérocycloalkyle,
(w) hétérocycloalkyle substitué,
(x) -NH-C(O)-R³ où R³ est tel que défini précédemment,
(y) -NH-C(O)-NR⁴R⁵ dans lequel R⁴ et R⁵ sont tels que définis précédemment,
(z) =N-NR⁶R⁷ dans lequel R⁶ et R⁷ sont tels que définis précédemment,
(aa) =N-R³ dans lequel R³ est tel que défini précédemment,
(bb) =N-NH-C(O)-R⁴ dans lequel R⁴ est tel que défini précédemment
et
(cc) =N-NH-C(O)-NR⁴R⁵ dans lequel R⁴ et R⁵ sont tels que définis précédemment,
(4) un groupe alcényle en C₃ substitué par un reste choisi dans le groupe constitué par
(a) un atome d'halogène,
(b) un groupe -CHO,
(c) -CO₂R³ où R³ est tel que défini précédemment,
(d) -C(O)-R⁴ où R⁴ est tel que défini précédemment,
(e) -C(O)-NR⁴R⁵ dans lequel R⁴ et R⁵ sont tels que définis précédemment,
(f) -C≡N,
(g) aryle,
(h) aryle substitué,
(i) hétéroaryle,
(j) hétéroaryle substitué,
(k) cycloalkyle en C₃-C₇,
et
(l) alkyle en C₁-C₁₂ substitué par un groupe hétéroaryle,
(5) un groupe alcényle en C₄-C₁₀,
(6) un groupe alcényle en C₄-C₁₀ substitué par un ou plusieurs substituants choisis dans le groupe constitué par
(a) un atome d'halogène,
(b) un groupe alcoxy en C₁-C₃,
(c) oxo,
(d) -CHO,
(e) -CO₂R³ où R³ est tel que défini précédemment,
(f) -C(O)-NR⁴R⁵ dans lequel R⁴ et R⁵ sont tels que définis précédemment,
(g) -NR⁶R⁷ dans lequel R⁶ et R⁷ sont tels que définis précédemment,
(h) =N-O-R³ dans lequel R³ est tel que défini précédemment,
(i) -C≡N,
(j) -O-S(O)ₙ-R³ dans lequel n est égal à 0, 1 ou 2 et R³ est tel que défini précédemment,
(k) aryle,
(l) aryle substitué,
(m) hétéroaryle,
(n) hétéroaryle substitué,
(o) cycloalkyle en C₃-C₇,
(p) alkyle en C₁-C₁₂ substitué par un groupe hétéroaryle,
(q) -NH-C(O)-R³ où R³ est tel que défini précédemment,
(r) -NH-C(O)-NR⁴R⁵ dans lequel R⁴ et R⁵ sont tels que définis précédemment,
(s) =N-NR⁶R⁷ dans lequel R⁶ et R⁷ sont tels que définis précédemment,
(t) =N-R³ dans lequel R³ est tel que défini précédemment,
(u) =N-NH-C(O)-R³ où R³ est tel que défini précédemment
et
(v) =N-NH-C(O)-NR⁴R⁵ dans lequel R⁴ et R⁵ sont tels que définis précédemment,
(7) un groupe alcynyle en C₃-C₁₀
et
(8) un groupe alcynyle en C₃-C₁₀ substitué par un ou plusieurs substituants choisis dans le groupe constitué par un groupe
(a) trialkylsilyle,
(b) aryle,
(c) aryle substitué,
(d) hétéroaryle,
et
(e) hétéroaryle substitué,
à condition que lorsque R est un groupe allyle et R¹ est un groupe méthyle, R² ne soit pas H;
R^{p} est un atome d'hydrogène ou un groupe protecteur d'un groupe hydroxy ;
R^{w} est choisi dans le groupe constitué par
(1) un atome d'hydrogène,
(2) un groupe alkyle en C₁-C₆, éventuellement substitué par un ou plusieurs substituants choisis dans le groupe constitué par un groupe
(a) aryle,
(b) aryle substitué,
(c) hétéroaryle,
(d) hétéroaryle substitué,
(3) un groupe choisi parmi les groupes de l'option (2) telle que définie précédemment substitués en outre par un groupe -CH₂ M-R⁸, dans lequel M est choisi dans le groupe constitué par un groupe
(i) -O-,
(ii)-NH-,
(iii) -N(CH₃)-,
(iv) -S(O)ₙ-, dans lequel n est tel que décrit précédemment,
(v) -NH-C(O)- et
(vi) -C(O)-NH-
et
R⁸ est choisi dans le groupe constitué par un groupe
(i) -(CH)ₙ-aryle, dans lequel n est tel que décrit précédemment,
(ii) -(CH₂)ₙ-(aryle substitué), dans lequel n est tel que décrit précédemment.
(iii) -(CH₂)ₙ-hétéroaryle, dans lequel n est tel que décrit précédemment,
(iv) -(CH₂)ₙ-(hétéroaryle substitué), dans lequel n est tel que décrit précédemment
et
(v) -(CH)ₙ-hétérocycloalkyle, dans lequel n est tel que décrit précédemment, et
W est absent ou est choisi dans le groupe constitué par un groupe -O-, -NH- et - N(CH₃)-.

2. Composé selon la revendication 1, qui est choisi dans le groupe constitué par
Un composé de formule (I), R est un groupe -CH₂CH=CH-(3-quinolyle), R^{p} est H, R¹ est un groupe méthyle, R² est un atome d'hydrogène ;
Un composé de formule (II), R est un groupe -CH₂CH=CH-(3-quinolyle), R^{p} est un groupe acétyle, R¹ est H, R² est un groupe CH₃, W est absent, R^{w} est H ; Un composé de formule (II); R est un groupe -CH₂CH=CH-(3-quinolyle), R^{p} est H, W est absent, R^{w} est H, R¹ est H, R² est un groupe CH₃ ;
Un composé de formule (II) ; R est un groupe -CH₂CH=CH-(3-quinolyle), R^{p} est H, W est absent, R^{w} est H, R¹ est un groupe acétyle, R² est un groupe CH₃ ;
Un composé de formule (II) ; R est un groupe -CH₂CH=CH-(3-quinolyle), R^{p} est H, W est absent, R^{w} est H, R¹ est un groupe CH₂C(O)-O-CH₂CH₃, R² est un groupe CH₃ ;
Un composé de formule (II) ; R est un groupe -CH₂CH=CH-(3-quinolyle), R^{p} est H, W est absent, R^{w} est H, R¹ est un groupe CH₂CH=H₂, R² est un groupe CH₃ ;
Un composé de formule (II) ; R est un groupe -CH₂CH=CH-(3-quinolyle), R^{p} est H, W est absent, R^{w} est H, R¹ est un groupe CH₂CH₂F, R² est un groupe CH₃ ;
Un composé de formule (II); R est un groupe -CH₂CH=CH-(3-quinolyle), R^{p} est H, W est absent, R^{w} est H, R¹ est un groupe CH₂-phényle, R² est un groupe CH₃ ;
Un composé de formule (II) ; R est un groupe -CH₂CH=CH-(3-quinolyle), R^{p} est H, W est absent, R^{w} est H, R¹ est un groupe CH₂-CN, R² est un groupe CH₃ ;
Un composé de formule (II) ; R est un groupe -CH₂CH=CH-(3-quinolyle), R^{p} est H, W est absent, R^{w} est H, R¹ est un groupe CH₂-C≡CH, R² est un groupe CH₃ ;
Un composé de formule (II) ; R est un groupe -CH₂CH=CH-(3-quinolyle), R^{p} est H, W est absent, R^{w} est H, R¹ est un groupe CH₂CH₂CH₃, R² est un groupe CH₃ ;
Un composé de formule (II) ; R est un groupe -CH₂CH=CH-(3-quinolyle), R^{p} est H, W est absent, R^{w} est H, R¹ est un groupe CH₂-cyclopropyle, R² est un groupe CH₃ ;
Un composé de formule (II) ; R est un groupe -CH₂CH=CH-(3-quinolyle), R^{p} est H, W est absent, R^{w} est H, R¹ est un groupe cyclopropyle, R² est un groupe CH₃ ;
Un composé de formule (II) ; R est un groupe -CH₂CH=CH-(3-quinolyle), R^{p} est H, W est absent, R^{w} est H, R¹ est un groupe CH₂-(3-pyridyle), R² est un groupe CH₃ ;
Un composé de formule (II) ; R est un groupe -CH₂CH=CH-(3-quinolyle), R^{p} est H, W est absent, R^{w} est H, R¹ est un groupe CH₂(cyclo-C₃H₅), R² est un groupe CH₃ ;
Un composé de formule (II) ; R est un groupe -CH₂CH=CH-(3-quinolyle), R^{p} est H, W est absent, R^{w} est H, R¹ est un groupe CH₂CH₂CH₃, R² est un groupe CH₃ ;
Un composé de formule (II) ; R est un groupe -CH₂CH=CH-(3-quinolyle), R^{p} est H, W est absent, R^{w} est H, R¹ est un groupe CH₂CH=CHC₆Hₛ, R² est un groupe CH₃ ;
Un composé de formule (II) ; R est un groupe -CH₂CH=CH-(3-quinolyle), R^{p} est H, W est absent, R^{w} est H, R¹ est un groupe CH₂C(=CH₂)C(O)OCH₃, R² est un groupe CH₃ ;
Un composé de formule (II) ; R est un groupe -CH₂CH=CH-(3-quinolyle), R^{p} est H, W est absent, R^{w} est H, R¹ est un groupe CH₂C(=CH₂)CH₃, R² est un groupe CH₃ ;
Un composé de formule (II) ; R est un groupe -CH₂CH=CH-(3-quinolyle), R^{p} est H, W est absent, R^{w} est H, R¹ est un groupe cyclo-C₃H₅, R² est un groupe CH₃ ;
Un composé de formule (II) ; R est un groupe -CH₂CH=CH-(3-quinolyle), R^{p} est H, W est absent, R^{w} est H, R¹ est un groupe CH₂-(3-pyridyle), R² est un groupe CH₃ ;
Un composé de formule (II) ; R est un groupe -CH₂CH=CH-(3-quinolyle), R^{p} est H, W est absent, R^{w} est H, R¹ est un groupe CH₂-(3-hydroxyphényle), R² est un groupe CH₃ ;
Un composé de formule (II); R est un groupe -CH₂CH=CH-(3-quinolyle), R^{p} est H, W est absent, R^{w} est H, R¹ est un groupe CH₂-(2-hydroxy-3-tert-butyl-5-méthylphényle), R² est un groupe CH₃;
Un composé de formule (II); R est un groupe -CH₂CH=CH-(3-quinolyle), R^{p} est H, W est absent, R^{w} est H, R¹ est un groupe CH₂-(2-hydroxy-3,4-diméthylphényle), R² est un groupe CH₃;
Un composé de formule (II): R est un groupe -CH₂CH=CH-(3-quinolyle), R^{p} est H, W est absent, R^{w} est H, R¹ est un groupe CH₂-(2-hydroxy-3-méthoxy-5-(2-propényl)phényle), R² est un groupe CH₃;
Un composé de formule (II); R est un groupe -CH₂CH=CH-(3-quinolyle), R^{p} est H, W est absent, R^{w} est H, R¹ est un groupe CH₂-(2-hydroxy-3-méthoxy-5-méthylphényle), R² est un groupe CH₃ ;
Un composé de formule (II); R est un groupe -CH₂CH=CH-(3-quinolyle), R^{p} est H, W est absent, R^{w} est H, R¹ est un groupe CH₂-(2-hydroxy-5-cyclopentylphényle), R² est un groupe CH₃;
Un composé de formule (II) ; R est un groupe -CH₂CH=CH-(3-quinolyle), R^{p} est H, W est absent, R^{w} est H, R¹ est un groupe CH₂-(2-hydroxy-5-carboxamidophényle), R² est un groupe CH₃;
Un composé de formule (II); R est un groupe -CH₂CH=CH-(3-quinolyle), R^{p} est H, W est absent, R^{w} est H, R¹ est un groupe CH₂-(2-hydroxy-3-méthoxy-5-(2-méthoxycarbonyléthyl)phényle), R² est un groupe CH₃ ;
Un composé de formule (II); R est un groupe -CH₂CH=CH-(3-quinolyle), R^{p} est H, W est absent, R^{w} est H, R¹ est un groupe CH₂-(2-hydroxy-3-méthyl-5-fluorophényle), R² est un groupe CH₃;
Un composé de formule (II) ; R est un groupe -CH₂CH=CH-(3-quinolyle), R^{p} est H, W est absent, R^{w} est H, R¹ est un groupe CH₂-(2-hydroxy-3-méthoxy-5-acétylphényle), R² est un groupe CH₃:
Un composé de formule (II): R est un groupe -CH₂CH=CH-(3-quinolyle), R^{p} est H, W est absent, R^{w} est H, R¹ est un groupe CH₂-(2-hydroxy-3-bromophényle), R² est un groupe CH₃:
Un composé de formule (II) ; R est un groupe -CH₂CH=CH-(3-quinolyle), R^{p} est H, W est absent, R^{w} est H, R¹ est un groupe CH₂-(2-hydroxy-3-méthoxy-5-alcoxycarbonylphényle), R² est un groupe CH₃;
Un composé de formule (II) ; R est un groupe -CH₂CH=CH-(3-quinolyle), R^{p} est H, W est absent, R^{w} est H, R¹ est un groupe CH₂-(2-hydroxy-3-éthylphényle), R² est un groupe CH₃ ;
Un composé de formule (II); R est un groupe -CH₂CH=CH-(3-quinolyle), R^{p} est H, W est absent, R^{w} est H, R¹ est un groupe CH₂-(2-hydroxy-5-isobutylphényle), R² est un groupe CH₃ ;
Un composé de formule (II); R est un groupe -CH₂CH=CH-(3-quinolyle), R^{p} est H, W est absent, R^{w} est H, R¹ est un groupe CH₂-(2-hydroxy-3-méthyl-5-diéthylamino-6-méthylphényle), R² est un groupe CH₃ ;
Un composé de formule (II) ; R est un groupe -CH₂CH=CH-(3-quinolyle), R^{p} est H, W est absent, R^{w} est H, R¹ est un groupe CH₂-(2-hydroxy-4-méthyl-5-bromo-6-méthylphényle), R² est un groupe CH₃ ; et
Un composé de formule (II) ; R est un groupe -CH₂CH=CH-(3-quinolyle), R^{p} est H, W est absent, R^{w} est H, R¹ est un groupe CH₂-(2-hydroxy-3-hydroxyméthylphényle), R² est un groupe CH₃.

3. Composition pharmaceutique pour traiter les infections bactériennes comprenant une quantité thérapeutiquement efficace d'un composé selon la revendication 1 ou d'un de ses sels ou esters pharmaceutiquement acceptables en association avec un support pharmaceutiquement acceptable.

4. Utilisation d'un composé selon la revendication 1 ou d'un de ses sels ou esters pharmaceutiquement acceptables pour fabriquer un médicament pour traiter les infections bactériennes par administration à un mammifère ayant besoin d'un tel traitement d'une composition pharmaceutique contenant une quantité thérapeutiquement efficace dudit composé ou d'un de ses sels ou esters pharmaceutiquement acceptables.

5. Composé selon la revendication 1 de formule (I)

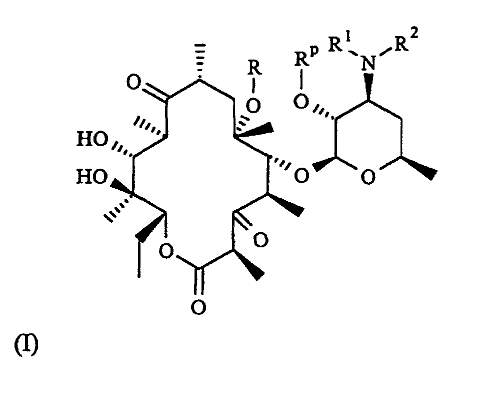

6. Composé selon la revendication 1 de formule (II)

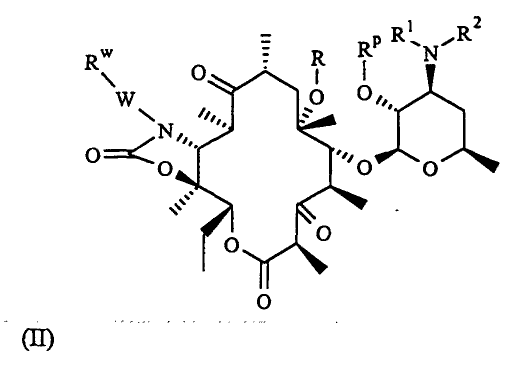

7. Composé selon la revendication 1 de formule (III)

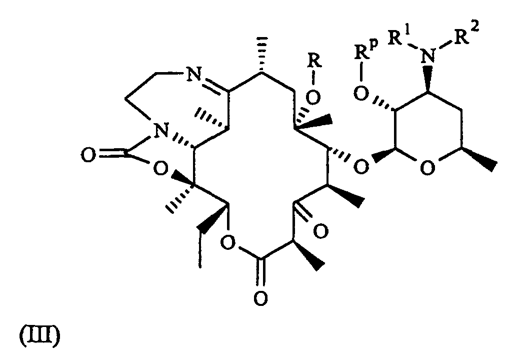

8. Composé selon la revendication 1 de formule (IV)

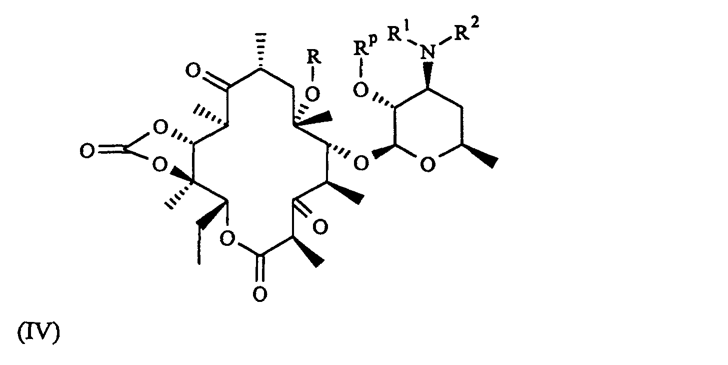

9. Composé selon la revendication 1 de formule (V)

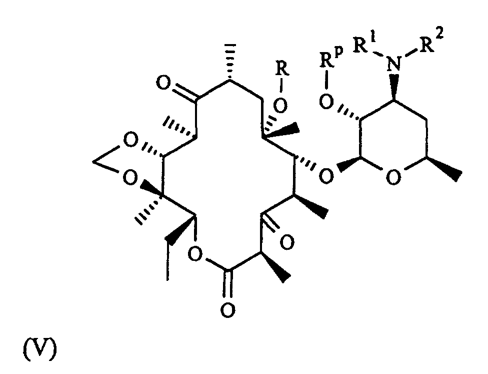

10. Procédé pour préparer un composé choisi dans le groupe constitué par 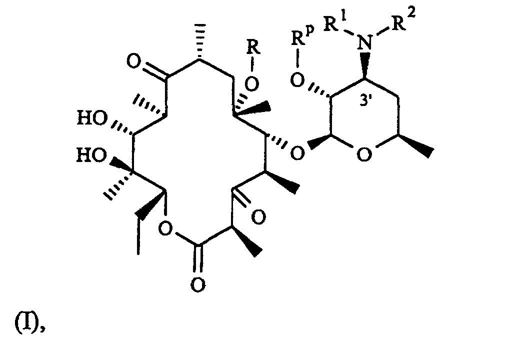 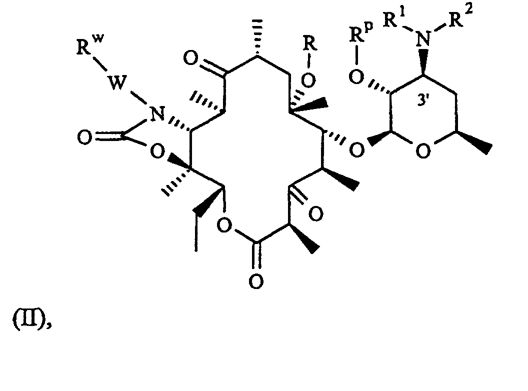 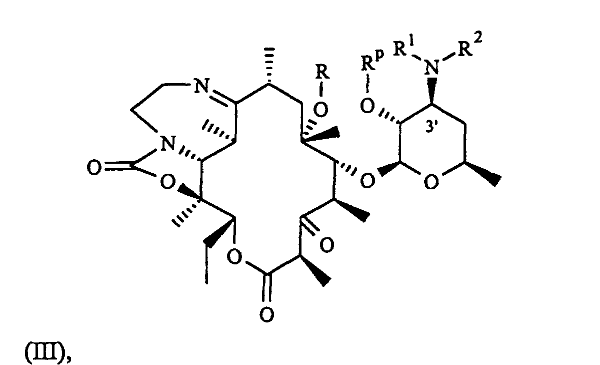 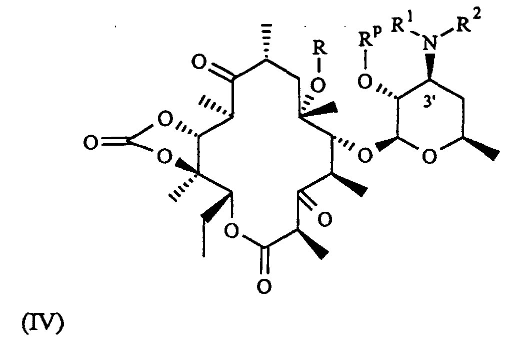 et 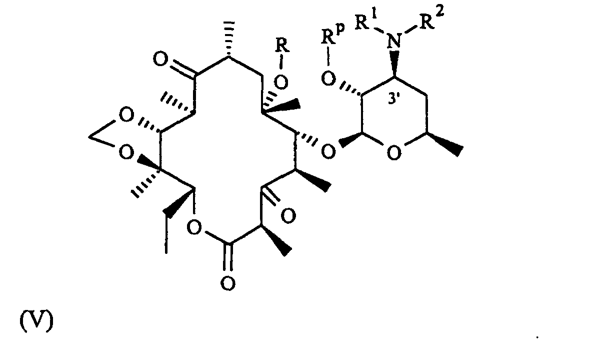 où
R¹ et R², à condition que R¹ et R² ne soient pas tous les deux un groupe méthyle, sont choisis indépendamment dans le groupe constitué par
(1) un atome d'hydrogène,
(2) un groupe alkyle en C₁-C₆ éventuellement substitué par un substituant choisi dans le groupe constitué par
(a) un atome d'halogène,
(b) un groupe cycloalkyle en C₃-C₆,
(c) aryle,
(d) aryle substitué,
(e) hétéroaryle,
(f) hétéroaryle substitué,
(g) -CHO,
(h) -C(O)-(alkyle en C₁-C₆) et
(i) -C(O)-NR'R", dans lequel R' et R" sont choisis indépendamment dans le groupe constitué par un atome d'hydrogène, un groupe alkyle en C₁-C₃, alkyle en C₁-C₃ substitué par un groupe aryle, aryle substitué, hétéroaryle et hétéroaryle substitué,
(3) un groupe alkyle en C₂-C₆ éventuellement substitué par un substituant choisi dans le groupe constitué par un groupe
(a) alcoxy en C₁-C₆,
(b) -NR'R", dans lequel R' et R" sont tels que définis précédemment,
(c) -NH-C(O)-(alkyle en C₁-C₆),
(d) -NH-C(O)-O-(alkyle en C₁-C₆),
(e) -O-C(O)-O-(alkyle en C₁-C₆),
(f) -O-C(O)-(alkyle en C₁-C₆),
(g) -CH(=N-O-(alkyle en C₁-C₆)),
(h) -C(=N-O-(alkyle en C₁-C₆))-(alkyle en C₁-C₆),
(i) -CH(=N-NH-(alkyle en C₁-C₆)) et
(j) -C(=N-NH-(alkyle en C₁-C₆))-(alkyle en C₁-C₆),
(4) un groupe alcényle en C₃-C₆ éventuellement substitué par un substituant choisi dans le groupe constitué par
(a) un atome d'halogène,
(b) un groupe cycloalkyle en C₃-C₆,
(c) aryle,
(d) aryle substitué,
(e) hétéroaryle,
(f) hétéroaryle substitué,
(g) -NH-C(O)-(alkyle en C₁-C₆),
(h) -NH-C(O)-O-(alkyle en C₁-C₆),
(i) -O-C(O)-O-(alkyle en C₁-C₆),
(j) -O-C(O)-(alkyle en C₁-C₆),
(k) -CHO,
(l) -C(O)-(alkyle en C₁-C₆),
(m) -C(O)-NR'R", dans lequel R' et R" sont tels que définis précédemment,
(n) -CH(=N-O-(alkyle en C₁-C₆)),
(o) -C(=N-O-(alkyle en C₁-C₆))-(alkyle en C₁-C₆),
(p) -CH(=N-NH-(alkyle en C₁-C₆)),
(q) -C(=N-NH-(alkyle en C₁-C₆))-(alkyle en C₁-C₆) et
(r) -C(O)-O-(alkyle en C₁-C₆,
(5) un groupe alcynyle en C₁-C₆ éventuellement substitué par un substituant choisi dans le groupe constitué par
(a) un atome d'halogène,
(b) un groupe cycloalkyle en C₃-C₆,
(c) aryle,
(d) aryle substitué,
(e) hétéroaryle et
(f) hétéroaryle substitué,
(6) un groupe cycloalkyle en C₃-C₆,
(7) un groupe -CHO,
(8) un groupe -C(O)-(alkyle en C₁-C₆),
(9) un groupe -C(O)-NR'R", dans lequel R' et R" sont tels que définis précédemment et
(10) un groupe -C(O)-O-(alkyle en C₁-C₆)
ou R' et R² pris ensemble peuvent être un groupe -(CH₂)ₚ-, dans lequel p est 3 à 7, qui pris ensemble avec l'atome d'azote auquel ils sont liés, forme ainsi un noyau hétérocyclique contenant un atome d'azote et 3 à 7 atomes de carbone ;
R est choisi dans le groupe constitué par
(1) un groupe méthyle substitué par un substituant choisi dans le groupe constitué par un groupe
(a) -CN,
(b) -F,
(c) -CO₂R³ dans lequel R³ est un groupe alkyle en C₁-C₃, alkyle en C₁-C₃ substitué par un groupe aryle ou alkyle en C₁-C₃ substitué par un groupe hétéroaryle,
(d) -S(O)ₙ-R³ dans lequel n est égal à 0, 1 ou 2 et R³ est tel que défini précédemment,
(e) -NH-C(O)-R³ où R³ est tel que défini précédemment,
(f) -NH-C(O)-NR⁴R⁵ dans lequel R⁴ et R⁵ sont choisis indépendamment dans le groupe constitué par
(i) un atome d'hydrogène,
(ii) un groupe alkyle en C₁-C₃,
(iii) alkyle en C₁-C₃ substitué par un groupe aryle,
(iv) alkyle en C₁-C₃ substitué par un groupe aryle substitué,
(v) alkyle en C₁-C₃ substitué par un groupe hétéroaryle et
(vi) alkyle en C₁-C₃ substitué par un groupe hétéroaryle substitué,
(g) aryle,
(h) aryle substitué,
(i) hétéroaryle,
et
(j) hétéroaryle substitué, ou
(2) un groupe alkyle en C₂-C₁₀,
(3) un groupe alkyle en C₂-C₁₀ substitué par un ou plusieurs substituants choisis dans le groupe constitué par
(a) un atome d'halogène,
(b) un groupe hydroxy,
(c) alcoxy en C₁-C₃,
(d) (alcoxy en C₁-C₃)-(alcoxy en C₁-C₃),
(e) oxo,
(f) -N₃,
(g) -CHO,
(h) -O-SO₂ (alkyle en C₁-C₆ substitué),
(i) -NR⁶R⁷ dans lequel R⁶ et R⁷ sont choisis dans le groupe constitué par
(i) un atome d'hydrogène,
(ii) un groupe alkyle en C₁-C₁₂,
(iii) alkyle en C₁-C₁₂ substitué,
(iv) alcényle en C₁-C₁₂,
(v) alcényle en C₁-C₁₂ substitué,
(vi) alcynyle en C₁-C₁₂,
(vii) alcynyle en C₁-C₁₂ substitué,
(viii) aryle,
(ix) cycloalkyle en C₃-C₈,
(x) cycloalkyle en C₃-C₈ substitué,
(xi) aryle substitué,
(xii) hétérocycloalkyle,
(xiii) hétérocycloalkyle substitué,
(xiv) alkyle en C₁-C₁₂ substitué par un groupe aryle,
(xv) alkyle en C₁-C₁₂ substitué par un groupe aryle substitué,
(xvi) alkyle en C₁-C₁₂ substitué par un groupe hétérocycloalkyle,
(xvii) alkyle en C₁-C₁₂ substitué par un groupe hétérocycloalkyle substitué,
(xviii) alkyle en C₁-C₁₂ substitué par un groupe cycloalkyle en C₃-C₈,
(xix) alkyle en C₁-C₁₂ substitué par un groupe cycloalkyle en C₃-C₈ substitué,
(xx) hétéroaryle,
(xxi) hétéroaryle substitué,
(xxii) alkyle en C₁-C₁₂ substitué par un groupe hétéroaryle, et
(xxiii) alkyle en C₁-C₁₂ substitué par un groupe hétéroaryle substitué
ou
R⁶ et R⁷ pris ensemble avec l'atome auquel ils sont liés forment un noyau hétérocycloalkyle à 3 à 10 chaînons qui peut être substitué par un ou plusieurs substituants choisis indépendamment dans le groupe constitué par
(i) un atome d'halogène,
(ii) un groupe hydroxy,
(iii) alcoxy en C₁-C₃,
(iv) (alcoxy en C₁-C₃)-(alcoxy en C₁-C₃),
(v) oxo,
(vi) alkyle en C₁-C₃,
(vii) halogénoalkyle en C₁-C₃
et
(vii) (alcoxy en C₁-C₃)-(alkyle en C₁-C₃)
(j) -CO₂R³ dans lequel R³ est tel que défini précédemment,
(k) -C(O)-NR⁴R⁵ dans lequel R⁴ et R⁵ sont tels que définis précédemment,
(l) =N-O-R³ dans lequel R³ est tel que défini précédemment,
(m) -C≡N,
(n) -O-S(O)ₙR³ dans lequel n et R³ sont tels que définis précédemment,
(o) aryle,
(p) aryle substitué,
(q) hétéroaryle,
(r) hétéroaryle substitué,
(s) cycloalkyle en C₃-C₈,
(t) cycloalkyle en C₃-C₈ substitué,
(u) alkyle en C₁-C₁₂ substitué par un groupe hétéroaryle,
(v) hétérocycloalkyle,
(w) hétérocycloalkyle substitué,
(x) -NH-C(O)-R³ où R³ est tel que défini précédemment,
(y) -NH-C(O)-NR⁴R⁵ dans lequel R⁴ et R⁵ sont tels que définis précédemment,
(z) =N-NR⁶R⁷ dans lequel R⁶ et R⁷ sont tels que définis précédemment,
(aa) =N-R³ dans lequel R³ est tel que défini précédemment,
(bb) =N-NH-C(O)-R⁴ dans lequel R⁴ est tel que défini précédemment
et
(cc) =N-NH-C(O)-NR⁴R⁵ dans lequel R⁴ et R⁵ sont tels que définis précédemment,
(4) un groupe alcényle en C₃ substitué par un reste choisi dans le groupe constitué par
(a) un atome d'halogène,
(b) un groupe -CHO,
(c) -CO₂R³ où R³ est tel que défini précédemment,
(d) -C(O)-R⁴ où R⁴ est tel que défini précédemment,
(e) -C(O)-NR⁴R⁵ dans lequel R⁴ et R⁵ sont tels que définis précédemment,
(f) -C≡N,
(g) aryle,
(h) aryle substitué,
(i) hétéroaryle,
(j) hétéroaryle substitué,
(k) cycloalkyle en C₃-C₇,
et
(l) alkyle en C₁-C₁₂ substitué par un groupe hétéroaryle,
(5) un groupe alcényle en C₄-C₁₀,
(6) un groupe alcényle en C₄-C₁₀ substitué par un ou plusieurs substituants choisis dans le groupe constitué par
(a) un atome d'halogène,
(b) un groupe alcoxy en C₁-C₃,
(c) oxo,
(d) -CHO,
(e) -CO₂R³ où R³ est tel que défini précédemment,
(f) -C(O)-NR⁴R⁵ dans lequel R⁴ et R⁵ sont tels que définis précédemment,
(g) -NR⁶R⁷ dans lequel R⁶ et R⁷ sont tels que définis précédemment,
(h) =N-O-R³ dans lequel R³ est tel que défini précédemment,
(i) -C≡N,
(j) -O-S(O)ₙ-R³ dans lequel n est égal à 0, 1 ou 2 et R³ est tel que défini précédemment,
(k) aryle,
(l) aryle substitué,
(m) hétéroaryle,
(n) hétéroaryle substitué,
(o) cycloalkyle en C₃-C₇,
(p) alkyle en C₁-C₁₂ substitué par un groupe hétéroaryle,
(q) -NH-C(O)-R³ où R³ est tel que défini précédemment,
(r) -NH-C(O)-NR⁴R⁵ dans lequel R⁴ et R⁵ sont tels que définis précédemment,
(s) =N-NR⁶R⁷ dans lequel R⁶ et R⁷ sont tels que définis précédemment,
(t) =N-R³ dans lequel R³ est tel que défini précédemment,
(u) =N-NH-C(O)-R³ où R³ est tel que défini précédemment
et
(v) =N-NH-C(O)-NR⁴R⁵ dans lequel R⁴ et R⁵ sont tels que définis précédemment,
(7) un groupe alcynyle en C₃-C₁₀
et
(8) un groupe alcynyle en C₃-C₁₀ substitué par un ou plusieurs substituants choisis dans le groupe constitué par un groupe
(a) trialkylsilyle,
(b) aryle,
(c) aryle substitué,
(d) hétéroaryle,
et
(e) hétéroaryle substitué,
à condition que lorsque R est un groupe allyle et R¹ est un groupe méthyle, R² ne soit pas H ;
R^{p} est un atome d'hydrogène ou un groupe protecteur d'un groupe hydroxy ;
R^{w} est choisi dans le groupe constitué par
(1) un atome d'hydrogène,
(2) un groupe alkyle en C₁-C₆, éventuellement substitué par un ou plusieurs substituants choisis dans le groupe constitué par un groupe
(a) aryle,
(b) aryle substitué,
(c) hétéroaryle,
(d) hétéroaryle substitué,
(3) un groupe choisi parmi les groupes de l'option (2) telle que définie précédemment substitués en outre par un groupe -CH₂-M-R⁸, dans lequel M est choisi dans le groupe constitué par un groupe
(i) -O-,
(ii) -NH-,
(ii) -N(CH₃)-,
(iv) -S(O)ₙ-, dans lequel n est tel que décrit précédemment,
(v) -NH-C(O)- et
(vi) -C(O)-NH-
et
R⁸ est choisi dans le groupe constitué par un groupe
(i) -(CH)ₙ-aryle, dans lequel n est tel que décrit précédemment,
(ii) -(CH₂)ₙ-(aryle substitué), dans lequel n est tel que décrit précédemment.
(iii) -(CH₂)ₙ-hétéroaryle, dans lequel n est tel que décrit précédemment,
(iv) -(CH₂)ₙ-(hétéroaryle substitué), dans lequel n est tel que décrit précédemment
et
(v) -(CH)ₙ-hétérocycloalkyle, dans lequel n est tel que décrit précédemment,
et
W est absent ou est choisi dans le groupe constitué par un groupe -O-, -NH- et - N(CH₃)-
le procédé comprenant :
(a) séquentiellement la déméthylation de l'azote en 3' d'un composé choisi dans le groupe constitué par 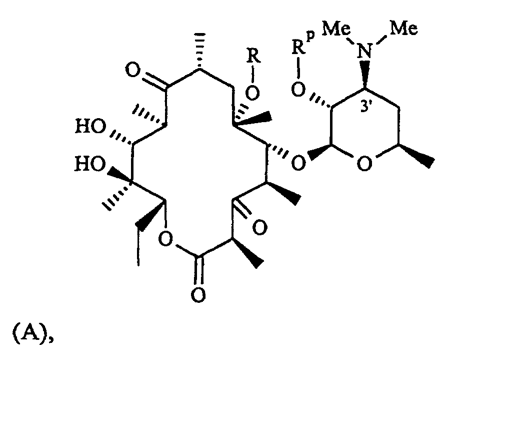 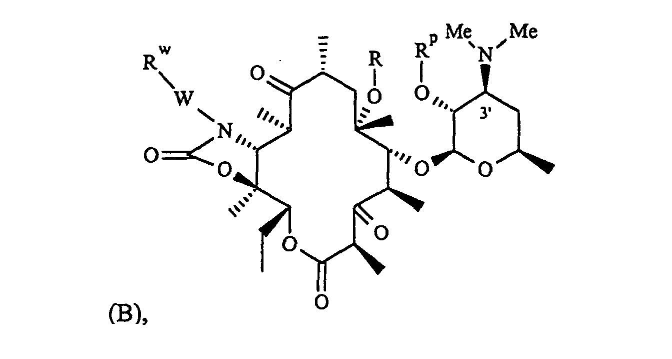 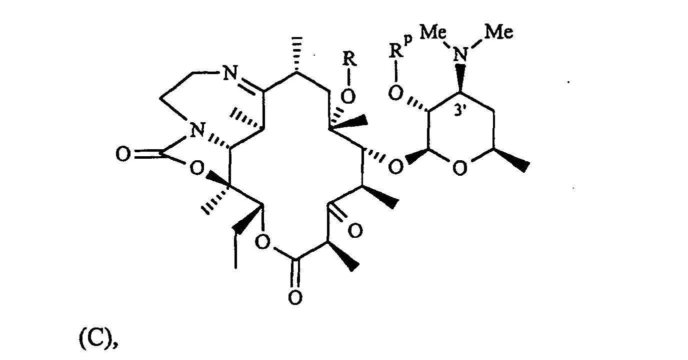 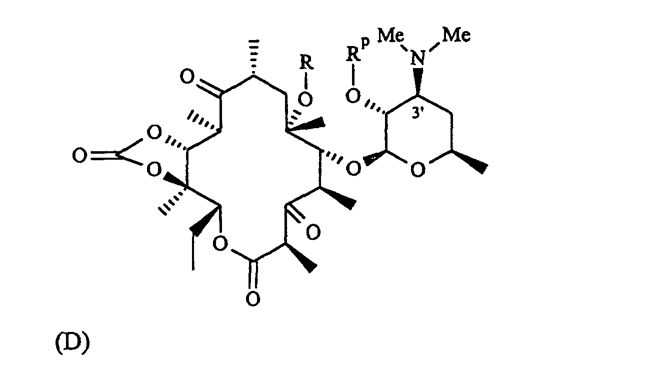 et 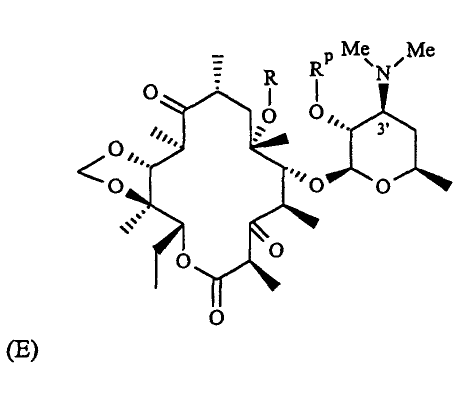 où R et R^{p} sont tels que définis précédemment ; et
(b) séquentiellement la réaction du composé provenant de l'étape (a) avec un composé précurseur du groupe R¹ et un composé précurseur du groupe R².

11. Procédé selon la revendication 10, dans lequel la déméthylation de l'azote en 3' est obtenue par réaction du composé avec le N-iodosuccinimide pour donner un composé correspondant comportant un groupe -NHCH₃ en 3'.

12. Procédé selon la revendication 11, dans lequel dans l'étape (b) on fait réagir le composé avec un précurseur du groupe R¹ choisi dans le groupe constitué par
(i) R¹-X dans lequel R¹ est tel que défini précédemment et X est un groupe partant halogénure ou sulfonate,
(ii) un aldéhyde de formule R*-CHO suivi par une réduction pour donner R*-CH₂, le reste R¹ étant tel que décrit précédemment,
(iii) le carbonyldiimidazole pour donner un composé intermédiaire dans lequel R¹ est un groupe imidazolylcarbonyle et réaction de cet intermédiaire avec une amine ayant la formule HNR'R", dans laquelle R' et R" sont tels que définis précédemment, pour donner un composé dans lequel R¹ est un groupe C(O)-NR'R",
(iv) un alcool de formule HOR', dans laquelle R' est tel que défini précédemment, pour donner un composé dans lequel R¹ est un groupe C(O)-OR',
(v) un agent d'acylation de formule X-C(O)-R', dans laquelle X est un atome d'halogène et R' est tel que défini précédemment, ou de formule O-(C(O)-R')₂ pour donner un composé dans lequel R¹ est un groupe C(O)R' et
(vi) un arylalcool substitué ou non substitué et un agent d'homologation choisi parmi le formaldéhyde ou le paraformaldéhyde pour donner un composé dans lequel R¹ est un groupe méthyle substitué par un groupe aryle substitué.

13. Procédé selon la revendication 11, comprenant en outre le traitement du composé avec de l'iodosuccinimide ou de l'iode en présence de lumière pour donner un composé correspondant comportant un groupe -NH₂ en 3'.

14. Procédé selon la revendication 13, qui comprend en outre le traitement du composé avec un précurseur du groupe R¹ pour donner un composé comportant un groupe -NHR¹CH₃ en 3', dans lequel le précurseur du groupe R¹ est choisi dans le groupe constitué par
(i) R¹-X dans lequel R¹ est tel que défini précédemment et X est un groupe partant halogénure ou sulfonate,
(ii) un aldéhyde de formule R*-CHO suivi par une réduction pour donner R*-CH₂, le reste R¹ étant tel que décrit précédemment,
(iii) le carbonyldiimidazole pour donner un composé intermédiaire dans lequel R¹ est un groupe imidazolylcarbonyle et réaction de cet intermédiaire avec une amine ayant la formule HNR'R", dans laquelle R' et R" sont tels que définis précédemment, pour donner un composé dans lequel R¹ est un groupe C(O)-NR'R",
(iv) un alcool de formule HOR', dans laquelle R' est tel que défini précédemment, pour donner un composé dans lequel R¹ est un groupe C(O)-OR',
(v) un agent d'acylation de formule X-C(O)-R', dans laquelle X est un atome d'halogène et R' est tel que défini précédemment, ou de formule O-(C(O)-R')₂ pour donner un composé dans lequel R¹ est un groupe C(O)R' et
(vi) un arylalcool substitué ou non substitué et un agent d'homologation choisi parmi le formaldéhyde ou le paraformaldéhyde pour donner un composé dans lequel R¹ est un groupe méthyle substitué par un groupe aryle substitué.

15. Procédé selon la revendication 14, qui comprend en outre le traitement du composé avec un composé précurseur du groupe R² pour donner un composé comportant un groupe -NR¹R² en 3', dans lequel le précurseur du groupe R² est choisi dans le groupe constitué par
(i) R²-X dans lequel R² est tel que défini précédemment et X est un groupe partant halogénure ou sulfonate,
(ii) un aldéhyde de formule R*-CHO suivi par une réduction pour donner R*-CH₂, le reste R² étant tel que décrit précédemment,
(iii) le carbonyldiimidazole pour donner un composé intermédiaire dans lequel R² est un groupe imidazolylcarbonyle et réaction de cet intermédiaire avec une amine ayant la formule HNR'R", dans laquelle R' et R" sont tels que définis précédemment, pour donner un composé dans lequel R² est un groupe C(O)-NR'R",
(iv) un alcool de formule HOR' pour donner un composé dans lequel R² est un groupe C(O)-OR',
(v) un agent d'acylation de formule X-C(O)-R', dans laquelle X est un atome d'halogène et R' est tel que défini précédemment, ou de formule O-(C(O)-R')₂ pour donner un composé dans lequel R² est un groupe C(O)R' et
(vi) un arylalcool substitué ou non substitué et un agent d'homologation choisi parmi le formaldéhyde ou le paraformaldéhyde pour donner un composé dans lequel R¹ est un groupe méthyle substitué par un groupe aryle substitué.
